(19) Europäisches Patentamt
European Patent Office
Office européen des brevets

(11) EP 4 317 155 A1

(12) # EUROPEAN PATENT APPLICATION
published in accordance with Art. 153(4) EPC

(43) Date of publication:
**07.02.2024 Bulletin 2024/06**

(21) Application number: **22775326.6**

(22) Date of filing: **16.03.2022**

(51) International Patent Classification (IPC):
***C07D 417/12*** *(2006.01)* ***C07D 417/14*** *(2006.01)*
***C08F 20/38*** *(2006.01)* ***G02B 1/04*** *(2006.01)*

(52) Cooperative Patent Classification (CPC):
**C07D 417/12; C07D 417/14; C08F 20/38; G02B 1/04**

(86) International application number:
**PCT/JP2022/011919**

(87) International publication number:
**WO 2022/202538 (29.09.2022 Gazette 2022/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.03.2021 JP 2021049181**

(71) Applicant: **Mitsubishi Chemical Corporation**
**Chiyoda-ku**
**Tokyo 100-8251 (JP)**

(72) Inventors:
- **YAMASHITA, Shuji**
**Tokyo 100-8251 (JP)**
- **YABE, Akiko**
**Tokyo 100-8251 (JP)**
- **ISHIKAWA, Tatsuya**
**Tokyo 100-8251 (JP)**
- **SATO, Ken**
**Tokyo 100-8251 (JP)**
- **TANAKA, Asato**
**Tokyo 100-8251 (JP)**

(74) Representative: **Hoffmann Eitle**
**Patent- und Rechtsanwälte PartmbB**
**Arabellastraße 30**
**81925 München (DE)**

(54) **COMPOUND, METHOD FOR PRODUCING SAME, POLYMERIZABLE COMPOSITION, POLYMER, HOLOGRAPHIC RECORDING MEDIUM, OPTICAL MATERIAL, AND OPTICAL COMPONENT**

(57) A compound represented by the following formula (1):

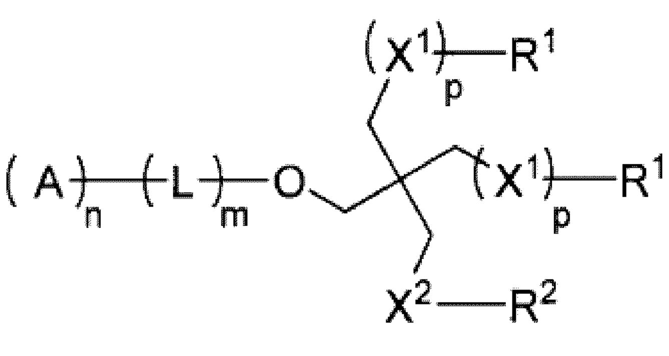

(1)

[wherein A represents a polymerizable group; L represents an optionally branched (n+1)-valent linking group; $R^1$ represents an aromatic ring group optionally having a substituent; $R^2$ represents a monovalent organic group optionally

EP 4 317 155 A1

having a substituent; $X^1$ and $X^2$ each independently represent an oxygen atom, a sulfur atom, or a nitrogen atom optionally having a substituent; m represents an integer of 0 or 1; n represents an integer of 1 to 3; and p represents an integer of 0 or 1, and
wherein two $R^1$s may be bonded together at any positions to form a ring structure,
provided that $R^1 = R^2$, $X^1 = X^2$, and p = 1 do not hold simultaneously].

A compound that has simultaneously a high refractive index, high transparency is provided.

## Description

### Technical Field

The present invention relates to a compound having a high refractive index, high transparency and good polymerizability and a method for producing the same. The present invention also relates to a holographic recording medium, an optical material, and an optical component that use the polymerizable composition or a polymer thereof.

### Background Art

Glass has often been used for optical components. For example, as for optical lenses with the same focal length, a lens produced using a high-refractive index material can have a smaller thickness, and this is advantageous in that the weight of the lens can be reduced and that the design flexibility of an optical path increases. High-refractive index optical lenses are also effective in reducing size of optical imaging devices and increasing their resolution and angle of view.

In recent years, highly transparent plastics are receiving attention as optical materials alternative to glass. Plastic materials have advantages over glass in that they can be easily reduced in weight, that their mechanical strength can be easily improved, and that they can be easily shaped. With the development of peripheral technologies, there is an increasing demand for plastic optical materials with improved performance. For example, materials for optical lens applications are required to be easily polymerized (have easy polymerizability), have good curability, and form polymerized products with a high refractive index (high-refractive index polymerized products).

Various resins have been developed for the purpose of increasing the refractive index.

For example, 9,9-bis[4-(2-acryloyloxyethoxy)phenyl]fluorene is frequently used as a high-refractive index acrylate. However, the viscosity of this acrylate is relatively high, and the refractive index of its monomer is about 1.62, which is not sufficiently high (PTL 1).

One way to increase the refractive index is to introduce an aromatic ring, and introducing a sulfur atom into the molecule is also effective. For example, PTL 2 describes a diacrylate monomer having a pentaerythritol skeleton and including 1 to 2 naphthylthio groups per molecule. In this case, the refractive index is 1.62 to 1.65.

PTL 3 describes an acrylate compound having a glycerin skeleton and including two benzothiazole rings per molecule. In this case also, the refractive index is 1.63.

The above compounds are not sufficient for applications that require an ultrahigh refractive index higher than 1.65.

PTL 4 and PTL 5 describe ultrahigh-refractive index acrylate compounds including dibenzofuran or dibenzocarbazole and having a refractive index higher than 1.7. However, their solubility in various mediums is not sufficient, and usable mediums are limited.

PTL 6 relates to an optical material used for holographic recording mediums. PTL 6 describes, as ultrahigh-refractive index compounds, pentaerythritol-type (meth)acrylate compounds having three aromatic rings. These compounds have a structure including three high-refractive index moieties of the same type that allow the compounds to have a high refractive index. However, sufficient polymerizability is not obtained due to steric hindrance around the polymerizable groups, and holographic recording characteristics may be low.

PTL 1: JPH6-220131A
PTL 2: JP2008-527413A
PTL 3: JP2005-133071A
PTL 4: WO2021/006011A1
PTL 5: WO2021/006012A1
PTL 6: JP2017-14213A


### Summary of Invention

It is an object of the present invention to provide a compound that has simultaneously a high refractive index, high transparency, and high polymerizability and is useful as an optical material or an optical component.

The present inventors have found that a polymerizable pentaerythritol-type compound having different high-refractive index moieties is a compound having simultaneously a high refractive index, high transparency, and high polymerizability and that a polymerizable composition and a polymer using the above compound have a high refractive index and high transparency.

The present invention is summarized as follows.

[1] A compound represented by the following formula (1): [0012]

[Chem. 1]

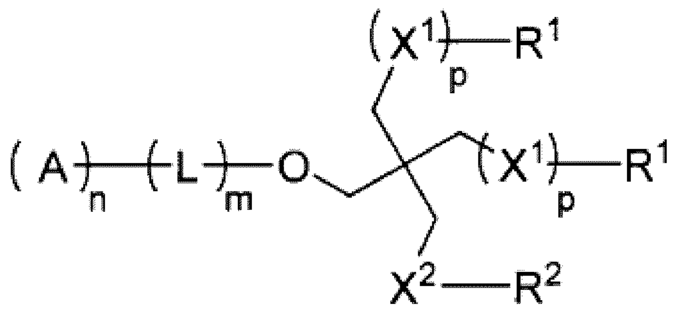

(1)

[wherein A represents a polymerizable group; L represents an optionally branched (n+1)-valent linking group; $R^1$ represents an aromatic ring group optionally having a substituent; $R^2$ represents a monovalent organic group optionally having a substituent; $X^1$ and $X^2$ each independently represent an oxygen atom, a sulfur atom, or a nitrogen atom optionally having a substituent; m represents an integer of 0 or 1; n represents an integer of 1 to 3; and p represents an integer of 0 or 1, and

wherein two $R^1$s may be bonded together at any positions to form a ring structure,
provided that $R^1 = R^2$, $X^1 = X^2$, and p = 1 do not hold simultaneously].

[2] The compound according to [1], wherein the number of polymerizable groups in formula (1) is 1.
[3] The compound according to [1] or [2], wherein A is an oxiranyl group, a vinyl group, an allyl group, or a (meth)acryloyl group.
[4] The compound according to [3], wherein A is a (meth)acryloyl group.
[5] The compound according to any one of [1] to [4], wherein $R^1$ is a fused aromatic ring group optionally having a substituent or a monocyclic aromatic group substituted with an aromatic ring group.
[6] The compound according to any one of [1] to [5], wherein $R^2$ has a partial structure represented by the following formula (2):

[Chem. 2]

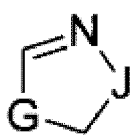

(2)

[wherein J represents a carbon atom optionally having a substituent or a nitrogen atom optionally having a substituent; and G represents a sulfur atom, an oxygen atom, or a nitrogen atom optionally having a substituent].
[7] A compound represented by the following formula (3):

[Chem. 3]

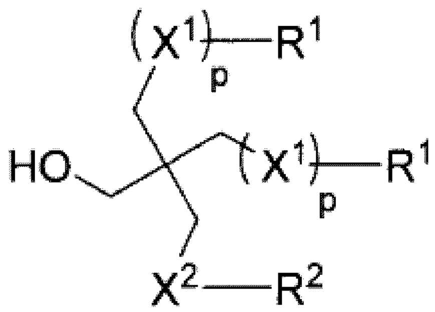

(3)

[wherein $R^1$ represents an aromatic ring group optionally having a substituent; $R^2$ represents a monovalent organic group optionally having a substituent; $X^1$ and $X^2$ each independently represent an oxygen atom, a sulfur atom, or a nitrogen atom optionally having a substituent; and p represents an integer of 0 or 1, and

wherein two $R^1$s may be bonded together at any positions to form a ring structure,
provided that $R^1 = R^2$, $X^1 = X^2$, and p = 1 do not hold simultaneously].

[8] A method for producing the compound according to [7], the method comprising subjecting an aliphatic cyclic compound represented by the following formula (4) to a ring-opening reaction:

[Chem. 4]

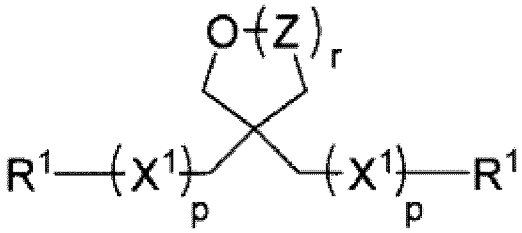

(4)

[wherein $R^1$ represents an aromatic ring group optionally having a substituent; $X^1$ represents an oxygen atom, a sulfur atom, or a nitrogen atom optionally having a substituent; p represents an integer of 0 or 1; Z represents an optionally branched aliphatic linking group optionally having a substituent; and r represents an integer of 0 or 1, and wherein two $R^1$s may be bonded together at any positions to form a ring structure].

[9] The compound production method according to [8], wherein the aliphatic cyclic compound represented by the formula (4) is a compound represented by the following formula (5) or (6): [0020]

[Chem. 5]

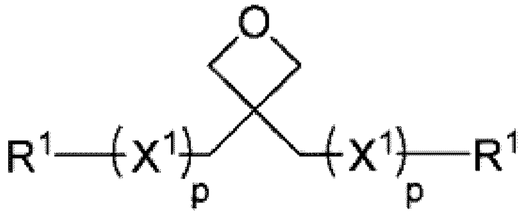

(5)

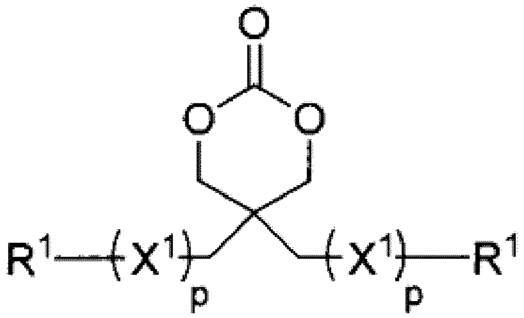

(6)

[wherein $R^1$ represents an aromatic ring group optionally having a substituent; $X^1$ represents an oxygen atom, a sulfur atom, or a nitrogen atom optionally having a substituent; and p represents an integer of 0 or 1, and wherein two $R^1$s may be bonded together at any positions to form a ring structure].

[10] A polymerizable composition comprising: the compound according to any one of [1] to [6]; and a polymerization initiator.

[11] A holographic recording medium comprising the polymerizable composition according to [10].

[12] A polymer obtained by polymerizing the polymerizable composition according to [10].

[13] An optical material comprising the polymer according to [12] .

[14] An optical component comprising the polymer according to [12] .

[15] A large-capacity memory comprising the holographic recording medium according to [11].

[16] An optical element obtained by recording a hologram in the holographic recording medium according to [11].

[17] An AR glass comprising the optical element according to [16] .

Advantageous Effects of Invention

The present invention provides a high-refractive index compound having both high transparency and high polymerizability and useful as an optical material.

The compound of the invention is particularly useful as a reactive compound used for hard coat layers of optical lenses and optical members and for holographic recording mediums.

The use of the compound of the invention makes it possible to obtain an optical material and an optical component having high diffraction efficiency, high light transmittance, and low turbidity.

Brief Description of Drawing

[Fig. 1] Fig. 1 is a schematic illustration showing the outline of the structure of a device used for holographic recording.

Description of Embodiments

Embodiments of the present invention will next be described specifically. The present invention is not limited to the following embodiments and can be modified variously within the scope of the invention.

In the present invention, "(meth)acrylate" is a generic term for acrylate and methacrylate. A "(meth)acryloyl group" is a generic term for an acryloyl group and a meth acryloyl group. In the present invention, the phrase "a group optionally having a substituent" means that the group may have one or more substituents. The "nitrogen atom optionally having substituent(s)" includes an imino group and the like when it does not have a substituent.

1. Compound of invention

The compound of the present invention is a compound represented by the following formula (1).

[Chem. 6]

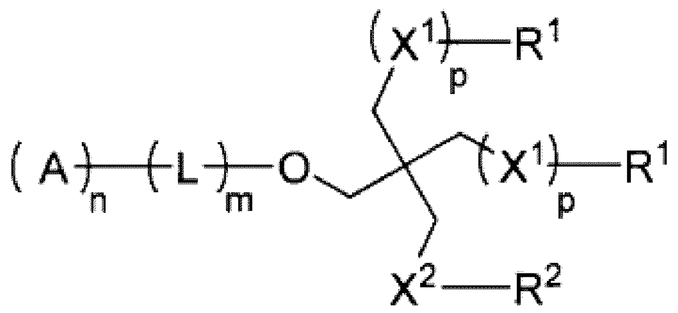

(1)

[wherein A represents a polymerizable group; L represents an optionally branched (n+1)-valent linking group; $R^1$ represents an aromatic ring group optionally having a substituent; $R^2$ represents a monovalent organic group optionally having a substituent; $X^1$ and $X^2$ each independently represent an oxygen atom, a sulfur atom, or a nitrogen atom optionally having a substituent; m represents an integer of 0 or 1; n represents an integer of 1 to 3; and p represents an integer of 0 or 1, and

wherein two $R^1$s may be bonded together at any positions to form a ring structure,
provided that $R^1 = R^2$, $X^1 = X^2$, and p = 1 do not hold simultaneously].

1-1. Structure of compound of formula (1)

The compound of formula (1) has a pentaerythritol skeleton, and one of the molecular chains bonded to the quaternary carbon atom has a polymerizable group. At least one of the remaining three molecular chains has a structure exhibiting a high refractive index. This allows a further increase in the refractive index of the polymer. In the compound represented by formula (1), two of the remaining three molecular chains have the same structure, and the remaining one has a different structure. This allows a further increase in the transparency of the polymer. The different structure is a molecular chain represented by $-X^2-R^2$ in formula (1) and means a structure different from the structure of molecular chains represented by $-(X^1)_p-R^1$. Even when $R^1$ and $R^2$ are identical in their constituent elements, partial structure, and number and $X^1$ and $X^2$ are identical in their constituent elements, partial structure, and number, if their bonding positions are different, the structure of $-X^2-R^2$ is considered to be different from the structure of $-(X^1)_p-R^1$.

1-2. A in formula (1)

A is a polymerizable group, and no particular limitation is imposed on the structure of A. Examples of the polymerizable group include a (meth)acryloyl group, an allyl group, a vinyl group, a vinyl-substituted phenyl group, an isopropenyl-substituted phenyl group, a vinyl-substituted naphthyl group, an isopropenyl-substituted naphthyl group, an oxiranyl group, a 2-methyloxiranyl group, and an oxetanyl group, and a group suitable for the intended polymerization method may be selected. In photopolymerization using a photopolymerization initiator, an oxiranyl group, a vinyl group, an allyl group, and a (meth)acryloyl group are preferable. Of these, a (meth)acryloyl group is particularly preferable because of its high reactivity.

1-3. L in formula (1)

L represents an optionally branched (n+1)-valent linking group. L does not necessarily contain a heteroatom. However, from the viewpoint of ease of synthesis, it is preferable that L contains an oxygen atom, a sulfur atom, or a nitrogen atom optionally having a substituent.

From the viewpoint of imparting high solubility in various mediums to the compound of formula (1) and avoiding coloration, L is preferably an aliphatic hydrocarbon group having an oxygen atom, a sulfur atom, or a nitrogen atom optionally having a substituent. The number of carbon atoms in the aliphatic hydrocarbon group (excluding the number of carbon atoms in a substituent) is preferably 1 to 8. When the number of carbon atoms in the aliphatic hydrocarbon group is 8 or less, the refractive index of the compound of formula (1) is unlikely to decrease. In addition, since the molecular weight is small, the viscosity is small, and the processability tends to be improved. The aliphatic hydrocarbon group forming L may be any of a cyclic aliphatic hydrocarbon group and a chain aliphatic hydrocarbon group, and a combination of these structures may be used. From the viewpoint of reducing steric hindrance around the polymerizable group A, a chain aliphatic hydrocarbon group is preferable.

Examples of the chain aliphatic hydrocarbon group having an oxygen atom, a sulfur atom, or a nitrogen atom optionally having a substituent and forming L when n = 1 include an oxomethylene group, an oxoethylene group, a 1,3-oxopropylene group, a 1,2-oxopropylene group, an oxobutylene group, a 2-hydroxyoxopropylene group, an oxohexylene group, an oxoheptylene group, a 3-oxopentylene group, - $OCH_2CH_2NHC(O)$-, -$OCH_2CH_2OCH_2CH_2NHC(O)$-, -$OCH_2CH_2SCH_2CH_2$-, - $OCH_2CH_2NHC(S)$ -, -$OCH_2CH_2OCH_2CH_2NHC(S)$ -, -$OCH_2CH_2SCH_2CH_2NHC(S)$ -, and -$OCH_2CH_2NHC(S)$-. L may be a combination of two or more of these groups. Examples of L when n = 2 or 3 include -$(OCH_2)_2C(CH_3)NHC(O)$- and a linking group in which a hydrogen atom(s) in the above chain aliphatic hydrocarbon group is(are) replaced with a bond(s) to a polymerizable group(s) A. In this case, L may be bonded to the polymerizable group(s) via a branched structure(s).

From the viewpoint of a high refractive index, L is preferably a cyclic group, and the ring included in the cyclic group forming L may have a monocyclic structure or a fused ring structure. The number of rings included in L is preferably 1 to 4, more preferably 1 to 3, and still more preferably 1 to 2. Each ring included in L does not necessarily have aromaticity. However, to keep the size of L in the molecule small and maintain the high refractive index, L is preferably an aromatic hydrocarbon ring. Examples of the aromatic hydrocarbon ring included in L include a benzene ring, an indene ring, a naphthalene ring, an azulene ring, a fluorene ring, an acenaphthylene ring, an anthracene ring, a phenanthrene ring, and a pyrene ring.

L may have a substituent. Examples of the optional substituent on L include halogen atoms (a chlorine atom, a bromine atom, and an iodine atom), a hydroxy group, a mercapto group, an alkyl group having 1 to 8 carbon atoms, an alkenyl group having 2 to 8 carbon atoms, an alkoxy group having 1 to 8 carbon atoms, a phenyl group, a mesityl group, a tolyl group, a naphthyl group, a cyano group, an acetyloxy group, an alkylcarbonyloxy group having 2 to 9 carbon atoms, an alkoxycarbonyl group having 2 to 9 carbon atoms, a sulfamoyl group, an alkylsulfamoyl group having 2 to 9 carbon atoms, an alkylcarbonyl group having 2 to 9 carbon atoms, a phenethyl group, a hydroxyethyl group, an acetylamido group, a dialkylaminoethyl group to which alkyl groups having 1 to 4 carbon atoms are bonded, a trifluoromethyl group, an alkylthio group having 1 to 8 carbon atoms, an aromatic thio group having 6 to 10 carbon atoms, and a nitro group.

1-4. $X^1$ and $X^2$ in formula (1)

$X^1$ and $X^2$ each independently represent an oxygen atom, a sulfur atom, or a nitrogen atom optionally having a substituent. From the viewpoint of reducing the water absorption rate to a low level, $X^1$ and $X^2$ are each preferably an oxygen atom or a sulfur atom and more preferably a sulfur atom that can impart a high refractive index. No particular limitation is imposed on the group with which the nitrogen atom may be substituted. Preferred examples of such a group include: alkyl groups having 1 to 8 carbon atoms such as a methyl group and an ethyl group; and aromatic hydrocarbon groups such as a phenyl group and a naphthyl group.

1-5. $R^1$ in formula (1)

$R^1$ represents an aromatic ring group optionally having a substituent. The aromatic rings that can be contained in $R^1$ are broadly classified into an aromatic hydrocarbon ring and an aromatic heterocycle.

Examples of the aromatic hydrocarbon ring include groups such as a benzene ring, a naphthalene ring, an anthracene ring, a phenanthrene ring, a perylene ring, a tetracene ring, a pyrene ring, a benzpyrene ring, a chrysene ring, a biphenylene ring, a triphenylene ring, an acenaphthene ring, a fluoranthene ring, and a fluorene ring.

Examples of the aromatic heterocycle include: aromatic heterocycles including one heteroatom such as a furan ring, a benzofuran ring, a dibenzofuran ring, a naphthofuran ring, a benzonaphthofuran ring, a dinaphthofuran ring, a

thiophene ring, a benzothiophene ring, a dibenzothiophene ring, a naphthothiophene ring, a benzonaphthothiophene ring, a dinaphthothiophene ring, a pyrrole ring, an indole ring, a carbazole ring, a benzo carbazole ring, a dibenzo carbazole ring, a pyridine ring, a quinoline ring, and an isoquinoline ring; aromatic heterocycles including two or more heteroatoms such as an imidazole ring, a triazole ring, a tetrazole ring, an oxazole ring, a thiazole ring, a pyridazine ring, a pyrimidine ring, a pyrazine ring, a triazine ring, and a thiadiazole ring; and rings each including two or three fused rings including an aromatic heterocycle having two or more heteroatoms such as a benzoxazole ring, a thienooxazole ring, a thiazolooxazole ring, an oxazolooxazole ring, an oxazoloimidazole ring, an oxazolopyridine ring, an oxazolopyridazine ring, an oxazolopyrimidine ring, an oxazolopyrazine ring, a naphthooxazole ring, a quinolinooxazole ring, a dioxazolopyrazine ring, a phenoxazine ring, a benzothiazole ring, a furothiazole ring, a thienothiazole ring, a thiazolothiazole ring, a thiazoloimidazole ring, a thienothiadiazole ring, a thiazolothiadiazole ring, a thiazolopyridine ring, a thiazolopyridazine ring, a thiazolopyrimidine ring, a thiazolopyrazine ring, a naphthothiazole ring, a quinolinothiazole ring, a thianthrene ring, and a phenothiazine ring.

From the viewpoint of ease of synthesis and availability, the aromatic hydrocarbon ring included in $R^1$ is preferably a benzene ring, a naphthalene ring, an anthracene ring, a phenanthrene ring, a pyrene ring, a biphenylene ring, or a fluorene ring. From the viewpoint of reducing fluorescence from the compound of formula (1), the aromatic hydrocarbon ring included in $R^1$ is more preferably a benzene ring, a naphthalene ring, a biphenylene ring, or a fluorene ring.

The aromatic heterocycle included in $R^1$ is preferably a sulfur-containing aromatic heterocycle because the refractive index of the compound of formula (1) tends to increase. The sulfur-containing aromatic heterocycle has at least a sulfur atom as a heteroatom included in the aromatic heterocycle. The sulfur-containing aromatic heterocycle may have, in addition to the sulfur atom, an oxygen atom or a nitrogen atom as a heteroatom and may have an oxygen atom and a nitrogen atom. From the viewpoint of avoiding coloration and obtaining sufficient solubility, the number of heteroatoms included in the sulfur-containing aromatic heterocycle is preferably 1 to 3 and more preferably 1 to 2.

Examples of the sulfur-containing aromatic heterocycle include: aromatic heterocycles including one sulfur atom such as a thiophene ring, a benzothiophene ring, a dibenzothiophene ring, a benzonaphthothiophene ring, a dinaphthothiophene ring, a thiopyran ring, a naphthothiophene ring, a dinaphthothiophene ring, and a dibenzothiopyran ring; aromatic heterocycles including two or more sulfur atoms such as a thianthrene ring; and aromatic heterocycles including two or more types of heteroatoms such as a thiazole ring, an isothiazole ring, a benzothiazole ring, a naphthothiazole ring, a phenothiazine ring, a thiazoloimidazole ring, a thiazolopyridine ring, a thiazolopyridazine ring, a thiazolopyrimidine ring, a dioxazolopyrazine ring, a thiazolopyrazine ring, a thiazolooxazole ring, a dibenzobenzothiophene ring, a thienooxazole ring, a thienothiadiazole ring, and a thiazolothiadiazole ring. The sulfur-containing aromatic heterocycle may be a monocycle or may be a fused ring. From the viewpoint of increasing the refractive index, the sulfur-containing aromatic heterocycle is preferably a fused ring. The number of rings forming the fused ring is preferably 2 to 8, more preferably 2 to 6, and still more preferably 2 to 5 in terms of availability of raw materials and ease of synthesis.

Among the above rings, a benzothiazole ring, a dibenzothiophene ring, a benzothiophene ring, a benzonaphthothiophene ring, a dinaphthothiophene ring, and a thianthrene ring are preferred in terms of low colorability and obtaining a high refractive index.

From the viewpoint of ease of synthesis, the aromatic heterocycle included in $R^1$ may be a nitrogen-containing aromatic heterocycle. The nitrogen-containing aromatic heterocycle has at least a nitrogen atom as a heteroatom included in the aromatic heterocycle. The nitrogen-containing aromatic heterocycle may have, in addition to the nitrogen atom, an oxygen atom or a sulfur atom as a heteroatom and may have an oxygen atom and a sulfur atom. From the viewpoint of avoiding coloration, the number of heteroatoms included in the nitrogen-containing aromatic heterocycle is preferably 1 to 3 and more preferably 1 to 2.

Examples of the nitrogen-containing aromatic heterocycles include: aromatic heterocycles including one nitrogen atom such as pyrrole ring, indole ring, carbazole ring, benzocarbazole ring, dibenzocarbazole ring, pyridine ring, quinoline ring, isoquinoline ring, oxazole ring, thiazole ring, benzoxazole ring, naphthoxazole ring, benzothiazole ring, naphthothiazole ring, phenoxazine ring, phenothiazine ring, thienooxazole ring, thiazoloxazole ring, oxazoloxazole ring, furothiazole ring, thienothiazole ring, thiazolothiazole ring; aromatic heterocycles including two or more nitrogen atom, such as imidazole ring, triazole ring, tetrazole ring, pyridazine ring, pyrimidine ring, pyrazine ring, triazine ring, thiadiazole ring, benzimidazole ring, oxazoloimidazole ring, oxazolopyridine ring, oxazolopyridazine ring, oxazolopyrimidine ring, oxazolopyrazine ring, quinolinoxazole ring, dioxazolopyrazine ring, thiazoloimidazole ring, thienothiadiazole ring, thiazolothiadiazole ring, thiazolopyridine ring, thiazolopyridazine ring, thiazolopyrimidine ring, thiazolopyrazine ring, quinolinothiazole ring.

The nitrogen-containing aromatic heterocycle may be a monocycle or may be a fused ring. From the viewpoint of increasing the refractive index, the nitrogen-containing aromatic heterocycle is preferably a fused ring. The number of rings forming the fused ring is preferably 2 to 8, more preferably 2 to 6, and still more preferably 2 to 5 in terms of availability of raw materials and ease of synthesis.

In terms of low colorability and increasing the refractive index, the nitrogen-containing aromatic heterocycle is preferably a carbazole ring, a benzocarbazole ring, a dibenzocarbazole ring, a pyridine ring, a quinoline ring, an isoquinoline ring, a benzoxazole ring, a benzothiazole ring, a benzimidazole ring, or a thiadiazole ring and is more preferably a carbazole ring, a benzocarbazole ring, a dibenzocarbazole ring, a benzoxazole ring, a benzothiazole ring, a benzimidazole ring, or a thiadiazole ring.

The aromatic heterocycle included in $R^1$ may be an oxygen-containing aromatic heterocycle. In this case, the heat resistance and weather resistance of the polymer obtained using the compound of formula (1) as a raw material tend to be improved. The oxygen-containing aromatic heterocycle has at least an oxygen atom as a heteroatom included in the aromatic heterocycle. The oxygen-containing aromatic heterocycle may have, in addition to the oxygen atom, a nitrogen atom or a sulfur atom as a heteroatom and may have a nitrogen atom and a sulfur atom. In terms of obtaining sufficient heat resistance, the number of oxygen atoms included in the oxygen-containing aromatic heterocycle is preferably 1 to 3 and more preferably 1 to 2.

Examples of the oxygen-containing aromatic heterocyclic rings include: aromatic heterocycles containing one oxygen atom such as furan ring, benzofuran ring, dibenzofuran ring, naphthofuran ring, benzonaphthofuran ring, dinaphthofuran ring, phenoxazine ring, oxazole ring, isoxazole ring, benzoxazole ring, benzoisoxazole ring, naphthoxazole ring, thienooxazole ring, thiazoloxazole ring, oxazoloimidazole ring and furothiazole ring; aromatic heterocycles containing two or more oxygen atoms such as a dibenzodioxin ring, an oxazoloxazole ring, and a dioxazolopyrazine ring.

The oxygen-containing aromatic heterocycle may be a monocycle or may be a fused ring. From the viewpoint of increasing the refractive index, a fused ring is preferred. The number of rings forming the fused ring is preferably 2 to 8 and more preferably 2 to 6 and is particularly preferably 2 to 5 in terms of availability of raw materials and ease of synthesis.

In particular, in terms of low colorability and increasing the refractive index, the oxygen-containing aromatic heterocycle is preferably a dibenzofuran ring, a benzonaphthofuran ring, a dinaphthofuran ring, an oxazole ring, an isoxazole ring, a benzoxazole ring, a benzoisoxazole ring, or a naphthooxazole ring and more preferably a dibenzofuran ring, a benzonaphthofuran ring, a dinaphthofuran ring, or a benzoxazole ring.

The aromatic ring constituting these $R^1$ may have a substituent. Examples of the substituent that $R^1$ may have include halogen atoms such as chlorine, bromine, and iodine, an alkyl group having 1 to 8 carbon atoms, an alkenyl group having 2 to 8 carbon atoms, an alkoxyl group, a cyano group, an acetyloxy group, an alkylcarbonyloxy group having 2 to 9 carbon atoms, an alkoxycarbonyl group having 2 to 9 carbon atoms, a sulfamoyl group, an alkylsulfamoyl group having 2 to 9 carbon atoms, an alkylcarbonyl group having 2 to 9 carbon atoms, a phenethyl group, a hydroxyethyl group, an acetylamido group, a dialkylaminoethyl group to which an alkyl group having 1 to 4 carbon atoms is bonded, a trifluoromethyl group, and a nitro group. Of these, an alkyl group having 1 to 8 carbon atoms, an alkoxyl group having 1 to 8 carbon atoms, an alkylthio group having 1 to 8 carbon atoms, an arylthio group having 6 to 10 carbon atoms, a cyano group, an acetyloxy group, an alkylcarboxyl group having 2 to 8 carbon atoms, a sulfamoyl group, an alkylsulfamoyl group having 2 to 9 carbon atoms, a nitro group are preferred.

From the viewpoint of increasing the refractive index of the compound of formula (1), it is preferable that the above aromatic ring included in $R^1$ further has, as a substituent, a group having an aromatic ring. The aromatic ring included in the substituent has the same meaning as the aromatic ring included in $R^1$. The aromatic ring included in the substituent may be bonded directly to the aromatic group included in $R^1$ at any position, may be bonded to the aromatic group via an oxygen atom, a sulfur atom, or a nitrogen atom optionally having a substituent, or may be bonded to the aromatic group via any linking group. More preferably, the substituent is bonded directly to the aromatic group.

When the aromatic ring included in the substituent is a sulfur-containing aromatic heterocycle, the refractive index of the compound of formula (1) tends to be higher. The definition of the sulfur-containing aromatic heterocycle is the same as the definition of that in $R^1$. The sulfur-containing aromatic heterocycle is more preferably a fused ring and is particularly preferably a benzothiazole ring, a dibenzothiophene ring, a benzothiophene ring, a benzonaphthothiophene ring, a dinaphthothiophene ring, or a thianthrene ring.

No particular limitation is imposed on the number of aromatic rings included as substituents in $R^1$. From the viewpoint of ease of synthesis and solubility, the number of aromatic rings is preferably 1 to 4 and more preferably 1 to 2.

From the viewpoint of obtaining simultaneously a high refractive index and high solubility in various mediums, the aromatic ring included in $R^1$ is preferably a fused aromatic ring optionally having a substituent or a monocyclic aromatic ring substituted with an aromatic ring group and more preferably a fused aromatic heterocycle optionally having a substituent or an aromatic hydrocarbon ring having an aromatic heterocycle as a substituent.

The aromatic ring included in $R^1$ may include two or more heterocycles selected from the above-described sulfur-containing aromatic heterocycles, the above-described nitrogen-containing aromatic heterocycles, and the above-described oxygen-containing aromatic heterocycles.

For example, the aromatic ring included in $R^1$ may be a carbazole ring having a dibenzothiophene ring as a substituent. The compound of formula (1) having this structure tends to have a high refractive index.

When p = 1, the aromatic ring included in $R^1$ may be bonded to $X^1$ in formula (1) at any position. When p = 0, the aromatic ring included in $R^1$ may be bonded to the pentaerythritol skeleton in formula (1) at any position.

It is preferable that the two $R^1$s in formula (1) are bonded together at any positions to form a ring structure in

order to keep their size in the molecule small and obtain a higher refractive index.

To maintain the overall flexibility of the molecule and obtain higher solubility in various mediums, it is preferable that the two $R^1$s in formula (1) are not bonded together.

1-6. $R^2$ in formula (1)

$R^2$ represents a monovalent organic group. $R^2$ may be linear, branched, or cyclic and may be a combination of these structures according to the required physical properties of the compound of formula (1).

$R^2$ may have a structure selected from the above-described structures for $R^1$ and may have the same structure as $R^1$, so long as $-X^2-R^2$ in formula (1) has the different structure described above.

From the viewpoint of increasing the refractive index of the compound of formula (1), it is preferable that $R^2$ has a ring structure. The ring structure may be a monocyclic structure or may be a fused ring structure. From the viewpoint of allowing the compound of formula (1) to have high solubility in various mediums, the number of rings included in $R^2$ is preferably 1 to 4, more preferably 1 to 3, and still more preferably 1 to 2.

Each ring included in $R^2$ does not necessarily have aromaticity. However, it is preferable that the ring structure includes an aromatic hydrocarbon ring or an aromatic heterocycle because a high refractive index tends to be obtained while the size of $R^2$ in the molecule can be kept low.

Examples of the aromatic hydrocarbon ring constituting $R^2$ include a benzene ring, an indene ring, a naphthalene ring, an azulene ring, a fluorene ring, an acenaphthylene ring, an anthracene ring, a phenanthrene ring, a perylene ring and the like.

Examples of the aromatic heterocycle ring constituting $R^2$ include: aromatic heterocycles including one heteroatom such as a furan ring, a benzofuran ring, a dibenzofuran ring, a naphthofuran ring, a benzonaphthofuran ring, a dinaphthofuran ring, a thiophene ring, a benzothiophene ring, a dibenzothiophene ring, a naphthothiophene ring, a benzonaphthothiophene ring, a dinaphthothiophene ring, a pyrrole ring, an indole ring, a carbazole ring, a pyridine ring, a quinoline ring, and an isoquinoline ring; aromatic heterocycles including two or more heteroatoms such as an imidazole ring, a triazole ring, a tetrazole ring, an oxazole ring, a thiazole ring, a pyridazine ring, a pyrimidine ring, a pyrazine ring, a triazine ring, and a thiadiazole ring; and rings each including two or three fused rings including an aromatic heterocycle having two or more heteroatoms such as a benzoxazole ring, a thienooxazole ring, a thiazolooxazole ring, an oxazolooxazole ring, an oxazoloimidazole ring, an oxazolopyridine ring, an oxazolopyridazine ring, an oxazolopyrimidine ring, an oxazolopyrazine ring, a naphthooxazole ring, a quinolinooxazole ring, a dioxazolopyrazine ring, a phenoxazine ring, a benzothiazole ring, a furothiazole ring, a thienothiazole ring, a thiazolothiazole ring, a thiazoloimidazole ring, a thienothiadiazole ring, a thiazolothiadiazole ring, a thiazolopyridine ring, a thiazolopyridazine ring, a thiazolopyrimidine ring, a thiazolopyrazine ring, a naphthothiazole ring, a quinolinothiazole ring, a thianthrene ring, and a phenothiazine ring.

From the viewpoint of ease of synthesis of the compound of formula (1), $R^2$ has preferably a heterocyclic structure and has more preferably a structure including an azole ring that is a nitrogen-containing 5-membered ring.

Examples of the azole ring include: a pyrrole ring having one nitrogen atom; and a thiazole ring, an oxazole ring, an imidazole ring, a pyrazole ring, a triazole ring, a furazan ring, a thiadiazole ring, and a tetrazole ring that have two or more heteroatoms. It is more preferable that $R^2$ has a partial structure represented by the following formula (2) because the target product is obtained efficiently.

[Chem. 7]

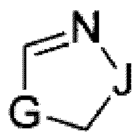

(2)

[wherein J represents a carbon atom optionally having a substituent or a nitrogen atom optionally having a substituent; and G represents a sulfur atom, an oxygen atom, or a nitrogen atom optionally having a substituent].

The partial structure represented by formula (2) may be appropriately selected according to need. From the viewpoint of ease of synthesis of the compound of formula (1) and obtaining high solubility in various mediums, the partial structure is particularly preferably a thiazole ring, an oxazole ring, an imidazole ring, or a thiadiazole ring.

From the viewpoint of increasing the refractive index of the compound of formula (1), $R^2$ has preferably an aromatic heterocycle as described above, and it is more preferable that $R^2$ has a fused aromatic heterocycle.

Examples of the fused aromatic heterocycle included in $R^2$ include an indole ring, a benzothiazole ring, a benzoxazole ring, and a benzimidazole ring. The fused aromatic heterocycle is particularly preferably a benzothiazole ring because the compound of formula (1) tends to have both a high refractive index and high solubility.

The ring structure in $R^2$ described above may be bonded directly to $X^2$ at any position and may be bonded to $X^2$ via an aliphatic linking group optionally having a substituent. From the viewpoint of improving the solubility of the compound of formula (1) in various mediums, the aliphatic linking group is preferably a chain group. When the chain moiety is an aliphatic hydrocarbon group, the solubility tends to be further improved. In this case, the number of carbon atoms in the aliphatic hydrocarbon group (excluding the number of carbon atoms in a substituent) is preferably 1 to 8. When the number of carbon atoms is 8 or less, the refractive index is unlikely to decrease. Moreover, since the molecular weight is small, the viscosity tends to decrease, and the processability tends to be improved. Examples of such an aliphatic linking group include a methylene group, an ethylene group, a propylene group, a carbonyl group, a thiocarbonyl group, -OC(O)-, -NHC(O)-, -OC(S)-, -NHC(S)-, $-CH_2OC(O)-$, $-CH_2CH_2OC(O)-$, $-CH_2CH_2OCH_2CH_2OC(O)-$, $-CH_2CH_2SCH_2CH_2OC(O)-$, $-CH_2OC(S)-$, $-CH_2CH_2OC(S)-$, $-CH_2CH_2OCH_2CH_2OC(S)-$, $-CH_2CH_2SCH_2CH_2OC(S)-$, $-CH_2SC(O)-$, $-CH_2CH_2SC(O)-$, $-CH_2CH_2OCH_2CH_2SC(O)-$, $-CH_2CH_2SCH_2CH_2SC(O)-$, $-CH_2NHC(O)-$, $-CH_2NHC(S)-$, $-CH_2CH_2NHC(O)-$, $-CH_2CH_2NHC(S)-$, $-CH_2CH_2OCH_2CH_2NHC(O)-$, $-CH_2CH_2OCH_2CH_2NHC(S)-$, $-CH_2CH_2SCH_2CH_2NHC(O)-$, and $-CH_2CH_2SCH_2CH_2NHC(S)-$.

The ring structure in $R^2$ may have a substituent. Examples of the optional substituent in $R^2$ include halogen atoms such as chlorine, bromine, and iodine, an alkyl group having 1 to 8 carbon atoms, an alkenyl group having 2 to 8 carbon atoms, an aromatic hydrocarbon ring having 1 to 14 carbon atoms, an aromatic heterocycle, an alkoxyl group, a cyano group, an acetyloxy group, an alkylcarbonyloxy group having 2 to 9 carbon atoms, an alkoxycarbonyl group having 2 to 9 carbon atoms, a sulfamoyl group, an alkylsulfamoyl group having 2 to 9 carbon atoms, an alkylcarbonyl group having 2 to 9 carbon atoms, a phenethyl group, a hydroxyethyl group, an acetylamido group, a dialkylaminoethyl group to which alkyl groups having 1 to 4 carbon atoms are bonded, a trifluoromethyl group, an alkylthio group having 1 to 8 carbon atoms, an aromatic thio group having 6 to 10 carbon atoms, and a nitro group. In particular, from the viewpoint of availability, the optional substituent in $R^2$ is preferably an alkyl group having 1 to 8 carbon atoms, an aromatic hydrocarbon ring having 1 to 14 carbon atoms, an aromatic heterocycle, an alkoxy group having 1 to 8 carbon atoms, a cyano group, an acetyloxy group, an alkylcarboxyl group having 2 to 8 carbon atoms, a sulfamoyl group, an alkylsulfamoyl group having 2 to 9 carbon atoms, or a nitro group.

When $R^2$ has a ring structure, a group further having an aromatic ring as a substituent may be appropriately selected and used, from the viewpoint of increasing the refractive index of the compound of formula (1). The aromatic ring included in the substituent has the same meaning as the aromatic ring included in $R^1$. The aromatic ring included in the substituent may be bonded directly to the ring structure of $R^2$ at any position, may be bonded to the ring structure via an oxygen atom, a sulfur atom, or a nitrogen atom optionally having a substituent, or may be bonded to the ring structure via any linking group. More preferably, the substituent is bonded directly to the ring structure of $R^2$.

1-7. m and n in formula (1)

m represents an integer of 0 or 1. m can be appropriately selected. However, from the viewpoint of reducing steric hindrance around the polymerizable group A and increasing the reactivity of the compound of formula (1), m is preferably 1.

n represents an integer of 1 to 3. n can also be appropriately selected. For example, from the viewpoint of increasing the ease of polymerization of the compound of formula (1), n may be 2 or 3.

In the compound of formula (1), the smaller the number of polymerizable groups, the better, because the refractive index tends to increase. More preferably, the compound of formula (1) is a monofunctional group having one polymerizable group. Specifically, from the viewpoint of increasing the refractive index, n is preferably 1 or 2 and more preferably 1.

1-8. Molecular weight

From the viewpoint of reducing the viscosity to a low level and maintaining good processability, the molecular weight of the compound of formula (1) is preferably 2000 or less and more preferably 1500 or less. From the viewpoint of reducing the shrinkage ratio during polymerization, the molecular weight of the compound of formula (1) is preferably 400 or more, more preferably 500 or more, and still more preferably 550 or more.

1-9. Relation between molecular structure and physical properties

In the compound of formula (1), one of the four molecular chains in the pentaerythritol skeleton has a polymerizable group, and at least one of the remaining three molecular chains has a structure exhibiting a high refractive

index. Therefore, the compound of formula (1) can be used as a polymerizable compound (monomer) having a high refractive index. In particular, when the high-refractive index moiety having the above-described different structure is appropriately introduced, the solubility of the monomer in various mediums can be kept high, and at the same time, the compatibility between the polymer after the polymerization reaction and the mediums can be controlled. For example, the molecular chain $-(X^1)_p-R^1$ provides a high refractive index. The molecular chain $-X^2-R^2$ ensures high compatibility with a target medium. In this manner, a high-refractive index transparent polymer with less turbidity can be obtained.

1-10. Exemplary compounds

Specific examples of the compound represented by formula (1) are exemplified below. The compound of the present invention is not limited to these compounds, and other compounds may be used so long as they do not depart from the scope of the invention.

[chem. 8]

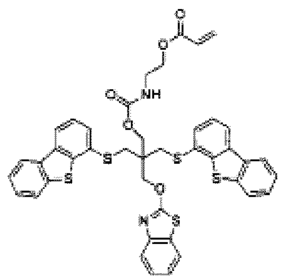
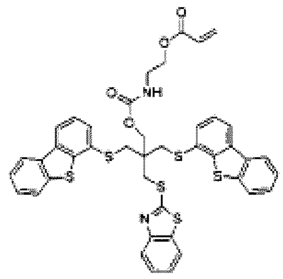
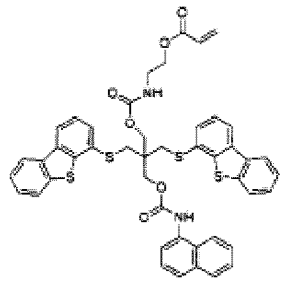
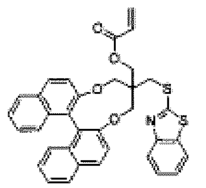
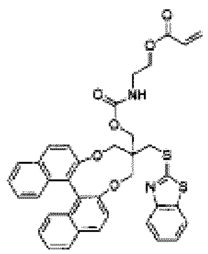
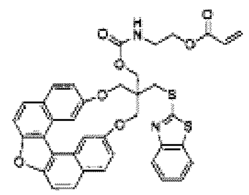
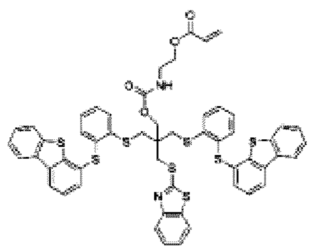
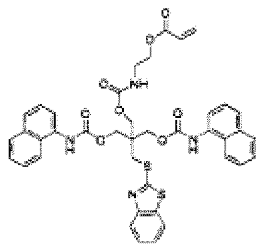
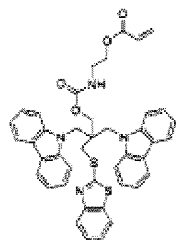
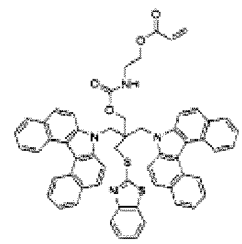

[chem. 9]

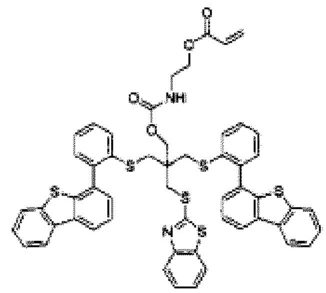
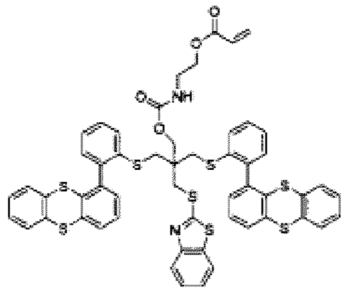
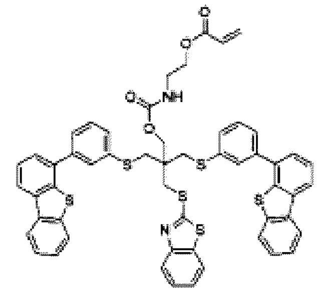
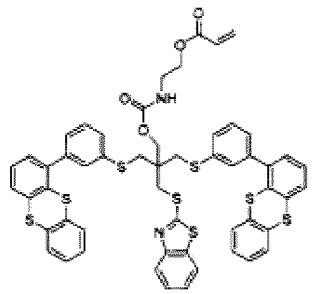
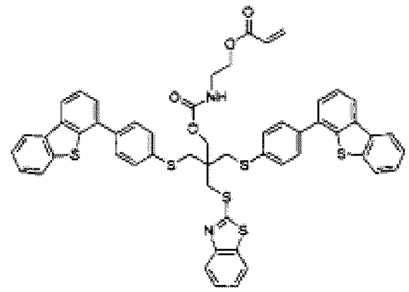
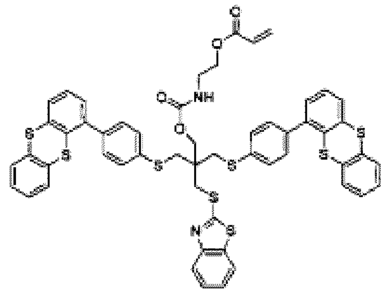
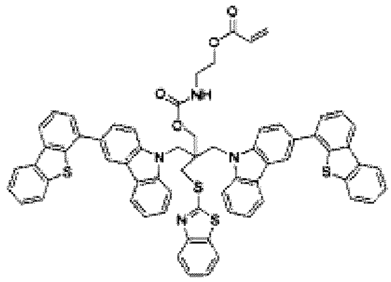
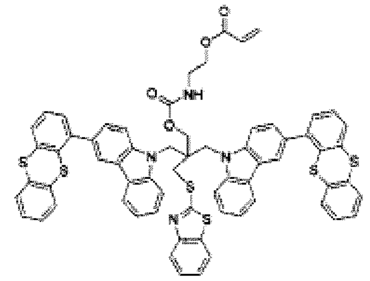
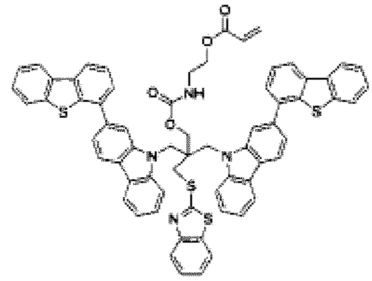
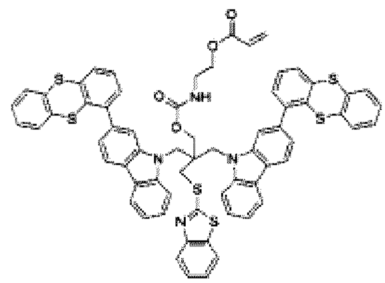
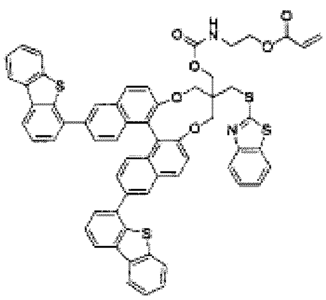
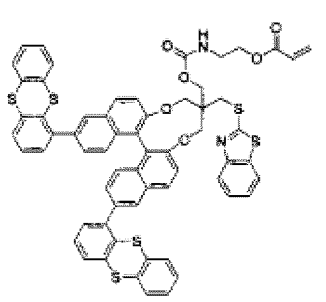

[chem. 10]

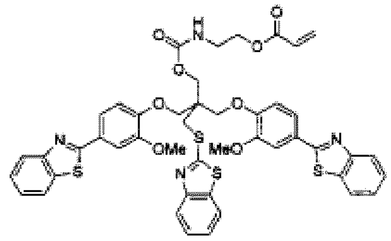
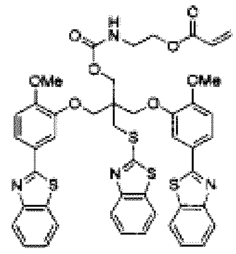
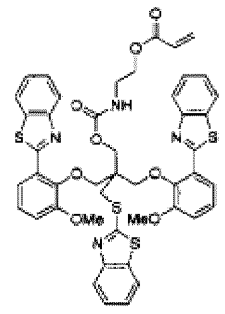
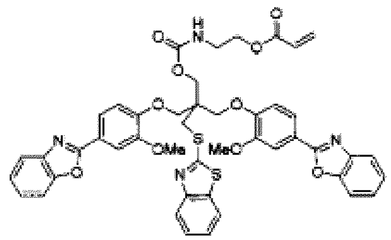
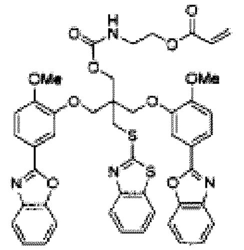
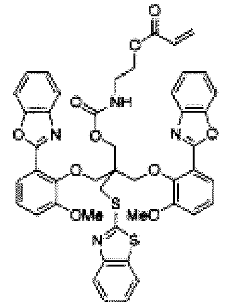
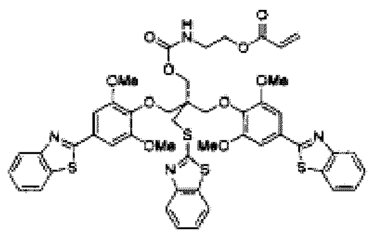
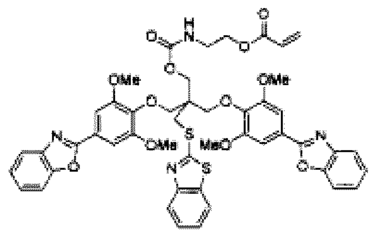
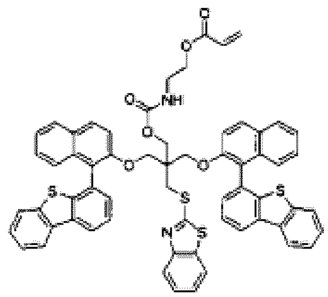
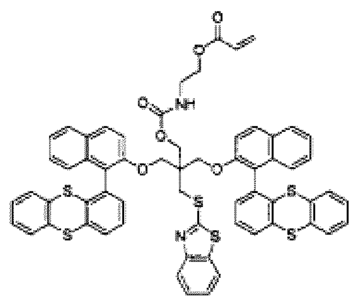
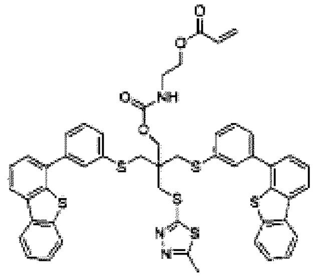
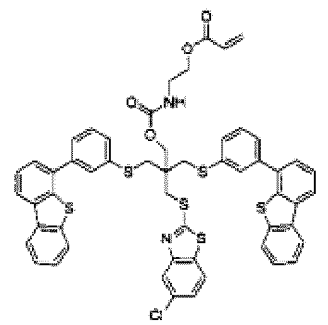

[chem. 11]

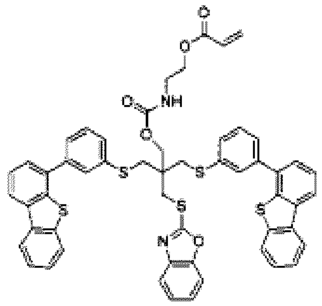
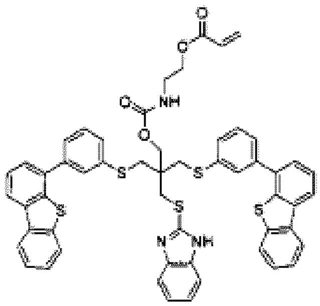
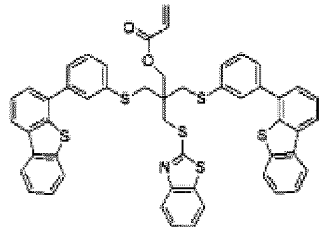

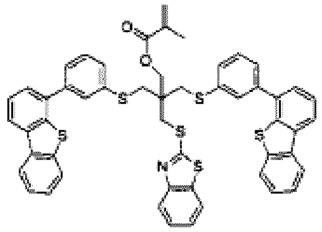
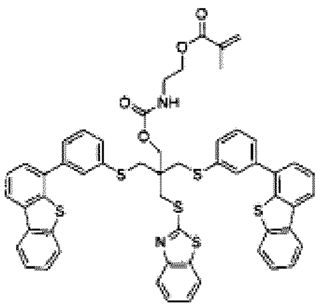
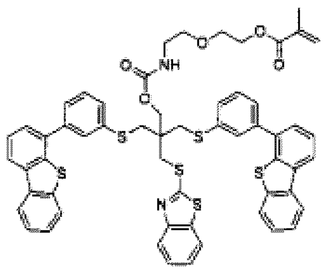

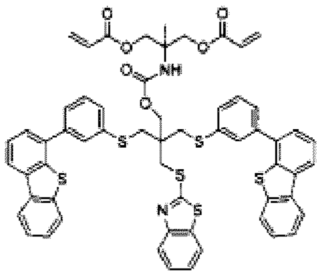
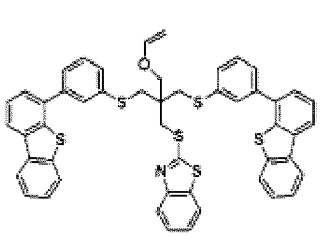
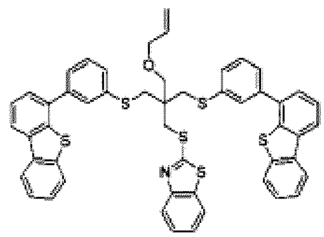

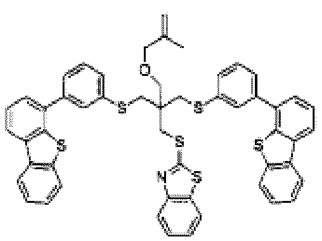
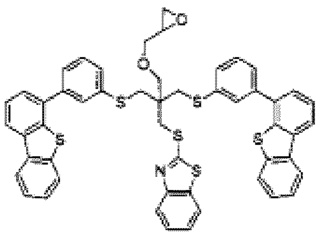
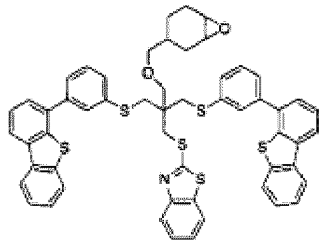

[chem. 12]

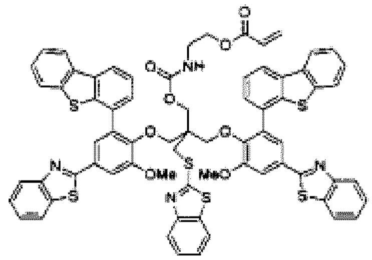
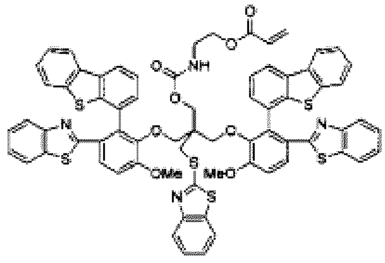
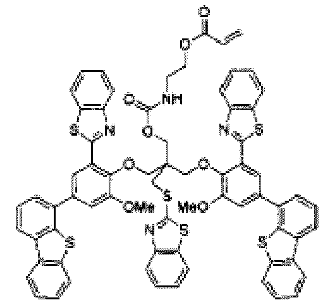

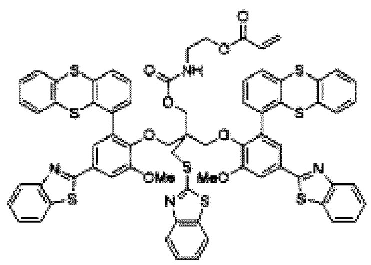
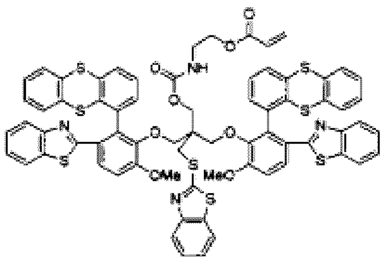
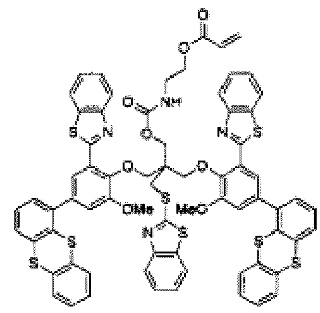

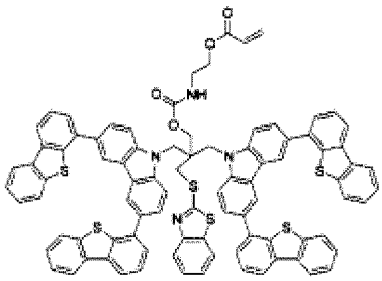
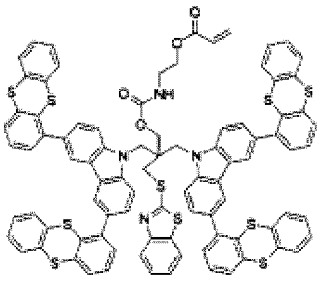
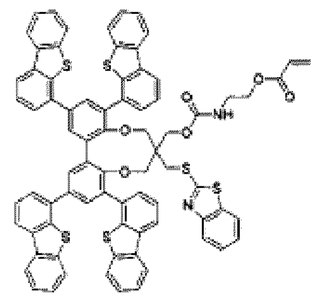

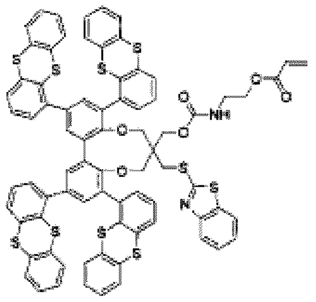
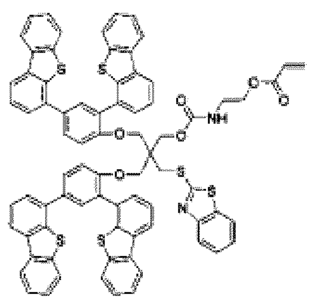
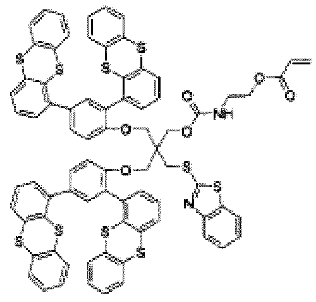

[chem. 13]

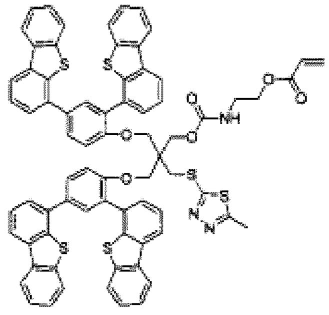

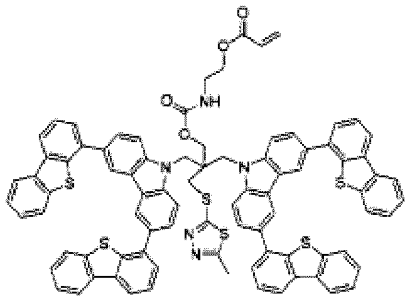

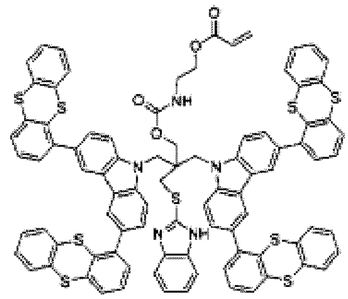

1-11. Synthesis method

The compound of formula (1) can be synthesized using a combination of various known methods. For example, the compound of formula (1) can be synthesized through the reaction of a compound represented by the following formula (3) and a compound having a group that can react with a hydroxy group.

[Chem. 14]

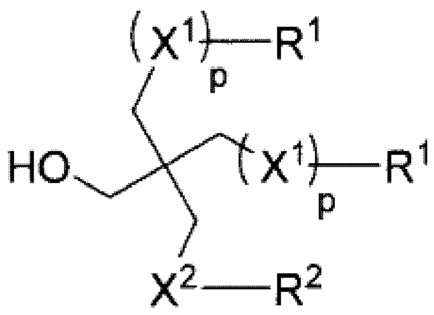

(3)

[wherein $R^1$ represents an aromatic ring group optionally having a substituent; $R^2$ represents a monovalent organic group optionally having a substituent; $X^1$ and $X^2$ each independently represent an oxygen atom, a sulfur atom, or a nitrogen atom optionally having a substituent; and p represents an integer of 0 or 1, and

wherein two $R^1$s may be bonded together at any positions to form a ring structure,
provided that $R^1 = R^2$, $X^1 = X^2$, and p = 1 do not hold simultaneously].

One synthesis example of the compound of formula (1) will be described below.

[Chem. 15]

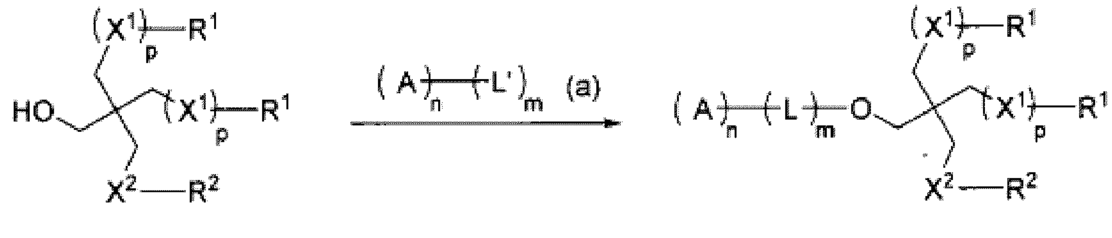

(3)

(1)

For example, the compound of formula (1) is a compound (1A) in which the polymerizable group A in formula (1) is a (meth)acryloyl group. The compound (1A) can be produced by a method in which the hydroxy group in the compound of formula (3) is reacted with a (meth)acrylating agent corresponding to the compound (a).

The (meth)acrylating agent is any compound that has a (meth)acryloyl group or a group convertible to a (meth)acryloyl group and that can be reacted with the active hydrogen in the hydroxy group in formula (3).

Examples of the (meth)acrylating agent include (meth)acrylic chloride, (meth)acrylic anhydride, (meth)acrylates, 3-chloropropionic acid chloride, 2-acryloyloxyethyl isocyanate, 2-methacryloyloxyethyl isocyanate, 2-(2-methacryloyloxyethyloxy)ethyl isocyanate, and 1,1-(bisacryloyloxymethyl)ethyl isocyanate.

A known method can be used for the reaction of the active hydrogen in the hydroxy group in formula (3) with the (meth)acrylating agent. For example, the compound of formula (3) and the (meth)acrylating agent are reacted in the presence of a basic compound, and the compound (1A) is thereby obtained.

The basic compound may be at least one of organic basic compounds (such as triethylamine, pyridine, and imidazole), at least one of inorganic basic compounds (such as sodium carbonate and potassium carbonate), or a combination of at least one of the organic basic compounds and at least one of the inorganic basic compounds.

Preferably, an organic solvent is used for the reaction of the compound of formula (3) and the (meth)acrylating agent. Examples of the organic solvent include dimethoxyethane, dichloromethane, tetrahydrofuran (THF), toluene, and N,N-dimethylformamide (DMF). One organic solvent may be used, or a combination of two or more organic solvents may be used.

In the production of the compound (1A), it is preferable to purify a reaction product (crude product) obtained by the synthetic reaction. By removing impurities by purification, low colorability can be achieved. A well-known purification method can be used. For example, an extraction method, column chromatography, a recrystallization method, a distillation method, etc. may be used for the purification. One of these purification methods may be performed, or any combination of these methods may be performed.

When the compound (1A) is solid at room temperature, it is preferable to use a recrystallization method because coloring materials can be efficiently removed with ease.

Examples of the recrystallization solvent include: aliphatic hydrocarbon-based solvents such as n-pentane, n-hexane, and n-heptane; alicyclic hydrocarbons such as cyclopentane and cyclohexane; aromatic hydrocarbon-based solvents such as toluene, ethylbenzene, xylene, and mesitylene; halogenated hydrocarbon-based solvents such as methylene chloride, chloroform, and 1,2-dichloroethane; ether-based solvents such as diethyl ether, diisopropyl ether, tetrahydrofuran, t-butyl methyl ether, and 1,4-dioxane; ketone-based solvents such as acetone, methyl ethyl ketone, and methyl isobutyl ketone; ester-based solvents such as ethyl acetate, n-butyl acetate, and propylene glycol monomethyl ether acetate; nitrile-based solvents such as acetonitrile and propionitrile; alcohol-based solvents such as methanol, ethanol, n-propanol, isopropanol, n-butanol, 2-butanol, t-butanol, 2-methoxyethanol, 2-butoxyethanol, and propylene glycol monomethyl ether; glycol-based solvents such as ethylene glycol and diethylene glycol; and water. One of these solvents may be used alone, or a combination of two or more of them may be used.

The compound of formula (3) can be produced using a combination of various known methods.

For example, the compound of formula (3) can be synthesized by performing a nucleophilic substitution reaction to pentaerythritol trihalide (b) to link the following compounds (c-1) and (c-2) each having a high-refractive index moiety simultaneously or sequentially, although the yield is low and the load on isolation and purification is large in some cases.

[Chem. 16]

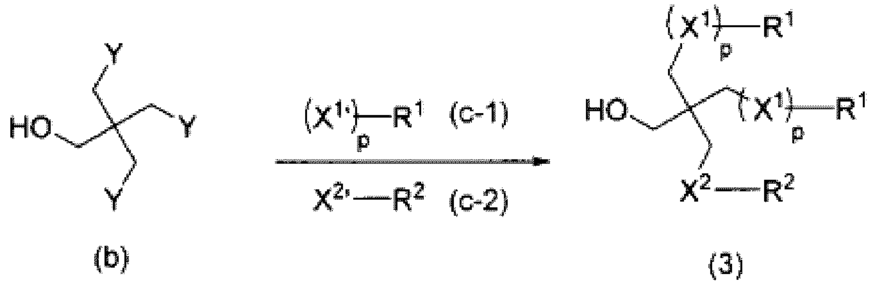

The compound of formula (3) can be synthesized at a high yield using a ring-opening reaction of an aliphatic cyclic compound represented by the following formula (4).

[Chem. 17]

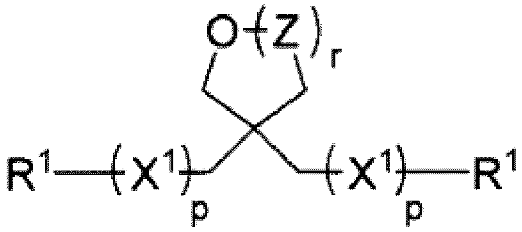

(4)

[wherein $R^1$ represents an aromatic ring group optionally having a substituent; $X^1$ represents an oxygen atom, a sulfur atom, or a nitrogen atom optionally having a substituent; p represents an integer of 0 or 1; Z represents an optionally branched aliphatic linking group optionally having a substituent; and r represents an integer of 0 or 1, and wherein two $R^1$s may be bonded together at any positions to form a ring structure].

When r = 0, formula (4) represents an oxetane compound.

When r = 1, Z represents an optionally branched aliphatic linking group optionally having a substituent.

Examples of the aliphatic cyclic compound of formula (4) include cyclic carbonate compounds, cyclic carbamate compounds, cyclic thiocarbonate compounds, cyclic thiocarbamate compounds, cyclic sulfate compounds, cyclic sulfite compounds, and cyclic phosphate compounds.

In the production of the compound of formula (3), an appropriate compound may be selected and produced as the aliphatic cyclic compound of formula (4) in accordance with need. Preferably, the aliphatic cyclic compound of formula (4) used is an oxetane compound represented by the following formula (5) or a cyclic carbonate compound represented by the following formula (6) because the compound obtained has both a high refractive index and high solubility.

[Chem. 18]

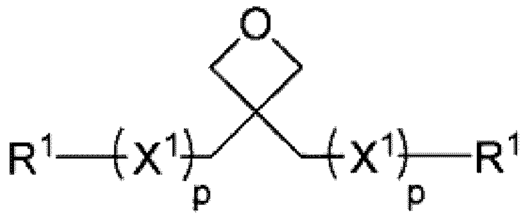

(5)

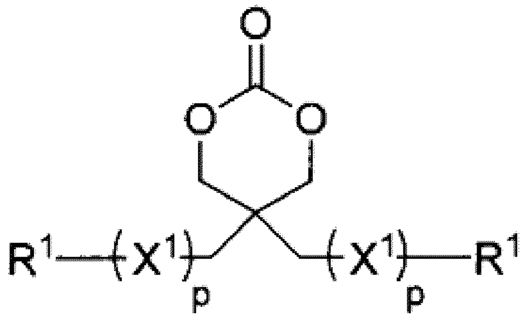

(6)

[wherein $R^1$ represents an aromatic ring group optionally having a substituent; $X^1$ represents an oxygen atom, a sulfur atom, or a nitrogen atom optionally having a substituent; and p represents an integer of 0 or 1, and wherein two $R^1$s may be bonded together at any positions to form a ring structure].

<Synthesis example of aliphatic cyclic compound of formula (4) with r = 1>

As shown in reaction formulas shown below, a reagent (d) selected from a cyclic carbonating reagent, a cyclic carbamating reagent, a cyclic thiocarbonating reagent, a cyclic thiocarbamating reagent, a cyclic sulfating reagent, a cyclic sulfiting reagent, and a cyclic phosphating reagent is reacted with pentaerythritol trihalide (b) to thereby obtain an intermediate (e). Then the compound (c-1) having a high-refractive index moiety is linked to the intermediate (e), and the aliphatic cyclic compound of formula (4) can thereby be synthesized.

The aliphatic cyclic compound of formula (4) can also be synthesized by reacting a compound (f) with the intermediate (e) to pre-introduce partial structures of the high-refractive index moieties and then bonding a compound (h) to the obtained intermediate (g).

The aliphatic cyclic compound of formula (4) can also be synthesized by linking the compound (c-1) having a high-refractive index moiety to pentaerythritol dihalide (i) instead of pentaerythritol trihalide (b) to obtain an intermediate (j) and reacting a reagent (d) with the intermediate (j).

The compound (c-2) having a high-refractive index moiety is reacted with the aliphatic cyclic compound of formula (4). In this case, the high-refractive index moiety can be introduced while the aliphatic ring structure is subjected to ring opening, and the compound of formula (3) can thereby be synthesized.

The compound of formula (3) can also be synthesized by bonding the high-refractive index moiety (c-2) directly to the intermediate (j).

[Chem. 19]

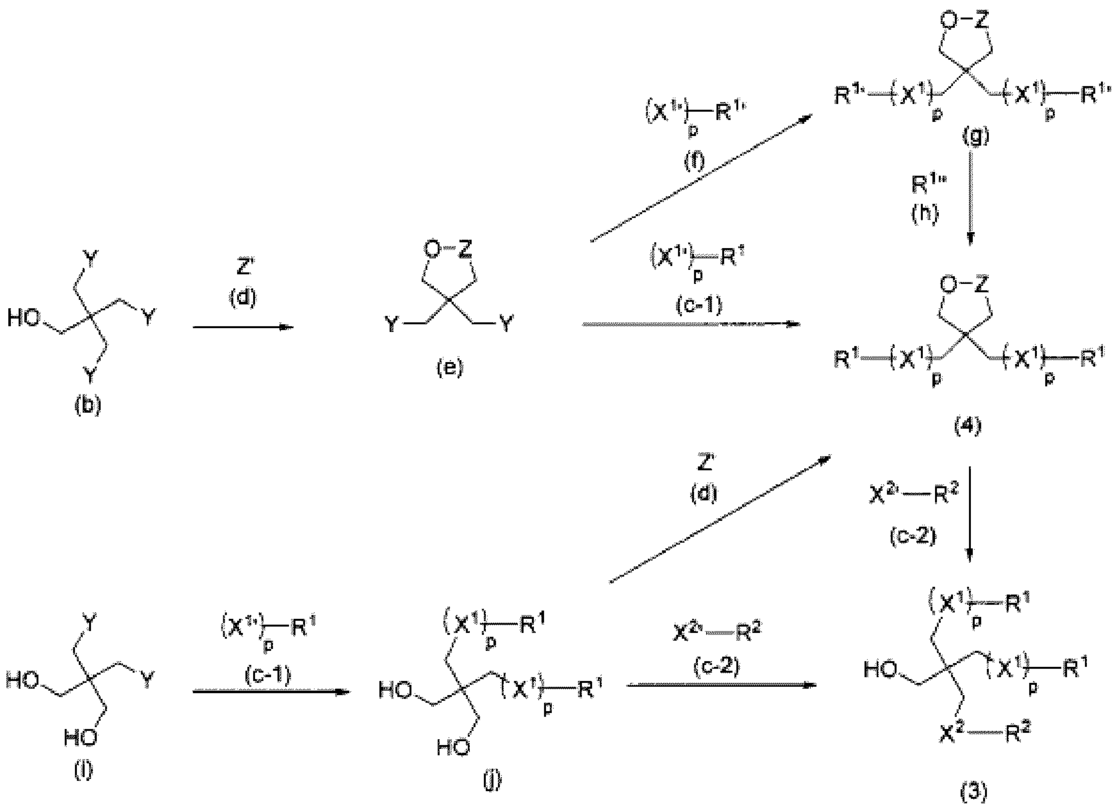


<Synthesis example of aliphatic cyclic compound of formula (4) with r = 0>

In this case, the aliphatic cyclic compound of formula (4) is an oxetane compound represented by formula (5).

As shown in reaction formulas below, the aliphatic cyclic compound of formula (4) can be synthesized, for example, by forming an oxetane structure using pentaerythritol trihalide (b) under acidic or basis conditions to obtain an intermediate (k) and then linking the compound (c-1) having a high-refractive index moiety to the intermediate (k).

The compound of formula (5) can also be synthesized by reacting the compound (f) with the intermediate (k) to pre-introduce partial structures of high-refractive index moieties to thereby obtain an intermediate (1) and then bonding the compound (h) to the obtained intermediate (1).

The compound (c-2) having a high-refractive index moiety is reacted with the compound of formula (5). In this manner, the high-refractive index moiety can be introduced while the oxetane structure is subjected to ring opening, and the compound of formula (3) can thereby be synthesized.

[Chem. 20]

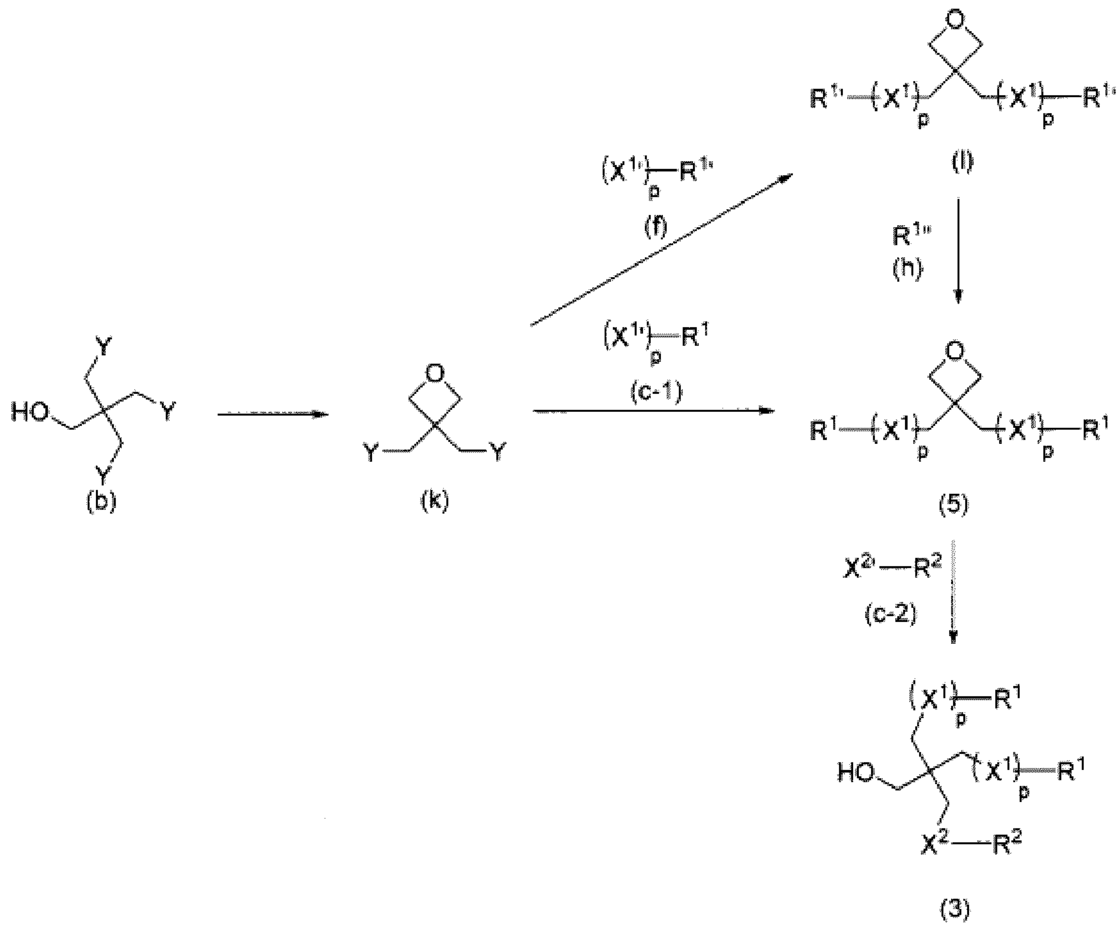

2. Polymerizable composition of invention

The polymerizable composition of the invention contains the compound represented by formula (1) and a polymerization initiator.

The polymerization initiator initiates a polymerization reaction of the polymerizable functional group A, in the compound represented by formula (1), and the polymer of the invention can thereby be obtained.

2-1. Polymerization initiator

No particular limitation is imposed on the type of polymerization initiator, and a suitable polymerization initiator may be selected from known polymerization initiators according to the polymerization method. No limitation is imposed on the polymerization method, and the polymerization may be performed by any known method such as a bulk polymerization method, a solution polymerization method, a suspension polymerization method, an emulsion polymerization method, or a partial polymerization method.

Examples of the polymerization initiator contained in the polymerizable composition of the invention include radical polymerization initiators, redox-based polymerization initiators, anionic polymerization initiators, and cationic polymerization initiators. Moreover, a cationic photopolymerization initiator that generates cations that are active species under irradiation with light can also be used.

Examples of the polymerization initiator described later include initiators referred generally to as polymerization catalysts.

2-1-1. Radical polymerization initiator

<Photopolymerization initiator>

Any known photo radical polymerization initiator can be used as the photopolymerization initiator that assists the polymerization of the polymerizable composition of the invention. Examples of the photopolymerization initiator used

include azo-based compounds, azide-based compounds, organic peroxides, organic borates, onium salts, bisimidazole derivatives, titanocene compounds, iodonium salts, organic thiol compounds, halogenated hydrocarbon derivatives, acetophenones, benzophenones, hydroxybenzenes, thioxanthones, anthraquinones, ketals, acylphosphine oxides, sulfone compounds, carbamic acid derivatives, sulfonamides, triarylmethanols, and oxime esters. In particular, the photopolymerization initiator is preferably benzophenones, acylphosphine oxide compounds, oxime ester compounds, etc. in terms of compatibility, availability, etc.

Specific examples of the photopolymerization initiator include benzophenone, 2,4,6-trimethylbenzophenone, methylorthobenzoylbenzoate, 4-phenylbenzophenone, t-butylanthraquinone, 2-ethylanthraquinone, diethoxyacetophenone, 2-hydroxy-2-methyl-1-phenylpropan-1-one, oligo{2-hydroxy-2-methyl-1-[4-(1-methylvinyl)phenyl]propanone}, benzil dimethyl ketal, 1-hydroxycyclohexyl phenyl ketone, benzoin methyl ether, benzoin ethyl ether, benzoin isopropyl ether, benzoin isobutyl ether, 2-methyl-[4-(methylthio)phenyl]-2-morpholino-1-propanone, 2-benzyl-2-dimethylamino-1-(4-morpholinophenyl)-butanone-1, diethylthioxanthone, isopropylthioxanthone, 2,4,6-trimethylbenzoyldiphenylphosphine oxide, bis(2,6-dimethoxybenzoyl)-2,4,4-trimethylpentylphosphine oxide, bis(2,4,6-trimethylbenzoyl)-phenylphosphine oxide, 2-hydroxy-1-{4-[4-(2-hydroxy-2-methylpropionyl)benzyl]phenyl}-2-methylpropan-1-one, methylbenzoylformate, 1-[4-(phenylthio)-2-(O-benzoyloxime)]-1,2-octanedione, and 1-[9-ethyl-6-(2-methylbenzoyl)-9H-carbazol-3-yl]-1-(O-acetyloxime)ethenone.

Any one of these photopolymerization initiators may be used alone, or any combination of two or more of them may be used at any ratio.

The content of the photopolymerization initiator in the polymerizable composition of the invention is generally 0.01 parts by mass or more, preferably 0.02 parts by mass or more, and still more preferably 0.05 parts by mass or more based on 100 parts by mass of the total of all radical polymerizable compounds in the polymerizable composition. The upper limit of the content is generally 10 parts by mass or less, preferably 5 parts by mass or less, and still more preferably 3 parts by mass or less. If the amount of the photopolymerization initiator added is excessively large, the polymerization proceeds abruptly. In this case, not only does the birefringence of the cured product increase, but also its hue may deteriorate. If the content is excessively small, the polymerizable composition may not be polymerized sufficiently.

<Thermal polymerization initiator>

Any known thermal radical polymerization initiator can be used as the thermal polymerization initiator that assists the polymerization of the polymerizable composition of the invention. Examples of such a thermal radical polymerization initiator include organic peroxides and azo compounds. Of these, organic peroxides are preferred because air bubbles are unlikely to be formed in the polymer obtained through the polymerization reaction.

Specific examples of the organic peroxides include: ketone peroxides such as methyl ethyl ketone peroxide; peroxy ketals such as 1,1-di(t-hexylperoxy)-3,3,5-trimethylcyclohexane, 1,1-di(t-hexylperoxy)cyclohexane, and 1,1-di(t-butylperoxy)cyclohexane; hydroperoxides such as 1,1,3,3-tetramethylbutyl hydroperoxide, cumene hydroperoxide, and p-menthane hydroperoxide; dialkyl peroxides such as dicumyl peroxide and di-t-butyl peroxide; diacyl peroxides such as dilauroyl peroxide and dibenzoyl peroxide; peroxydicarbonates such as di(4-t-butylcyclohexyl)peroxydicarbonate and di(2-ethylhexyl)peroxydicarbonate; and peroxyesters such as t-butylperoxy-2-ethylhexanoate, t-hexylperoxyisopropyl monocarbonate, t-butyl peroxybenzoate, and 1,1,3,3-tetramethylbutyl-2-ethylhexanoate.

Specific examples of the azo compounds include 2,2'-azobisisobutyronitrile, 2,2'-azobis(2-methylbutyronitrile), 2,2'-azobis(2,4-dimethylvaleronitrile), 1,1'-azobis-1-cyclohexanecarbonitrile, dimethyl-2,2'-azobisisobutyrate, 4,4'-azobis-4-cyanovaleric acid, and 2,2'-azobis-(2-amidinopropane)dihydrochloride.

Any one of these thermal polymerization initiators may be used alone, or any combination of two or more may be used at any ratio.

The content of the thermal polymerization initiator in the polymerizable composition of the invention is generally 0.1 parts by mass or more, preferably 0.5 parts by mass or more, and more preferably 0.8 parts by mass or more based on 100 parts by mass of the total of all the radical polymerizable compounds in the polymerizable composition. The upper limit of the content is generally 10 parts by mass or less, preferably 5 parts by mass or less, and more preferably 2 parts by mass or less. If the amount of the thermal polymerization initiator is excessively large, the polymerization proceeds abruptly. In this case, not only is the optical uniformity of the polymer to be obtained impaired, but also its hue may deteriorate. If the content is excessively small, the thermal polymerization may not proceed sufficiently.

When the photopolymerization initiator is used in combination with the thermal polymerization initiator, the mass ratio is generally "100:1" to "1:100" ("the photopolymerization initiator:the thermal polymerization initiator", the same applies to this paragraph) and preferably "10:1" to "1:10." If the amount of the thermal polymerization initiator is excessively small, the polymerization may be insufficient. If the amount is excessively large, coloration may occur.

2-1-2. Redox-based polymerization initiator

The redox-based polymerization initiator is a radical initiator that utilizes a redox reaction of a peroxide and a reducing agent used in combination, can generate radicals even at low temperature, and is generally used for emulsion polymerization etc.

Specific examples of the redox-based polymerization initiator include: combinations of dibenzoyl peroxide serving as a peroxide and an aromatic tertiary amine serving as a reducing agent such as N,N-dimethylaniline, N,N-dimethyl-p-toluidine, and N,N-bis(2-hydroxypropyl)-p-toluidine; combinations of a hydroperoxide serving as a peroxide and a metallic soap serving as a reducing agent; and combinations of a hydroperoxide serving as a peroxide and thioureas serving as a reducing agent.

As for a water-soluble redox-based polymerization initiator, a peroxide such as a persulfate, hydrogen peroxide, or a hydroperoxide is used in combination with a water-soluble inorganic reducing agent (such as $Fe^{2+}$ or NaHSOs) or a water-soluble organic reducing agent (such as an alcohol or a polyamine).

A preferred range of the content of the redox-based polymerization initiator in the polymerizable composition of the invention is the same as that for the thermal polymerization initiator.

2-1-3. Anionic polymerization initiator

Examples of the anionic polymerization initiator used for the polymerizable composition of the invention include alkali metals, n-butyllithium, sodium amide, sodium naphthalenide, a Grignard reagent, lithium alkoxides, and alkali metal benzophenone ketyls. Any of these may be used alone, or any combination of two or more of them may be used at any ratio.

2-1-4. Cationic polymerization initiator

Examples of the cationic polymerization initiator used for the polymerizable composition of the invention include: Bronsted acids such as perchloric acid, sulfuric acid, and trichloroacetic acid; Lewis acids such as boron trifluoride, aluminum trichloride, aluminum tribromide, and tin tetrachloride; iodine; and chlorotriphenylmethane. Any one of these may be used alone, or any combination of two or more of them may be used at any ratio.

The amount of the anionic polymerization initiator or the cationic polymerizing agent in the polymerizable composition of the invention is generally 0.001 parts by mass or more, preferably 0.005 parts by mass or more, and still more preferably 0.01 parts by mass or more based on 100 parts by mass of the total of all anionically or cationically polymerizable compounds in the polymerizable composition. The upper limit of the amount is generally 5 parts by mass or less, preferably 1 part by mass or less, and more preferably 0.5 parts by mass or less. If the amount of the anionic or cationic polymerization initiator is less than 0.001 parts by mass, the reaction is not sufficient. If the amount added exceeds 5 parts by mass, it is difficult to achieve a sufficient pot life and a sufficient polymerization rate simultaneously.

2-1-5. Cationic photopolymerization initiator

The cationic photopolymerization initiator in the present invention is an initiator that generates cationic species under light. No particular limitation is imposed on the cationic photopolymerization initiator so long as it is a compound that generates cationic species upon irradiation with light, and onium salts are generally well known. Examples of the onium salts include diazonium salts of Lewis acids, iodonium salts of Lewis acids, and sulfonium salts of Lewis acids. Specific examples include a phenyldiazonium salt of boron tetrafluoride, a diphenyl iodonium salt of phosphorus hexafluoride, a diphenyl iodonium salt of antimony hexafluoride, a tri-4-methylphenylsulfonium salt of arsenic hexafluoride, and a tri-4-methylphenylsulfonium salt of antimony tetrafluoride. Preferably, aromatic sulfonium salts are used.

Specific examples of the cationic photopolymerization initiator include S,S,S',S'-tetraphenyl-S,S'-(4,4'-thiodiphenyl)disulfonium bishexafluorophosphate, diphenyl-4-phenylthiophenyl sulfonium hexafluorophosphate, and diphenyl-4-phenylthiophenyl sulfonium hexafluoroantimonate. Other examples include product name: UVI-6992 manufactured by The Dow Chemical Company, product name: CPI-100P manufactured by San-Apro Ltd., product name: CPI-101A manufactured by San-Apro Ltd., product name: CPI-200K manufactured by San-Apro Ltd., and product name: Omnicat 270 manufactured by IGM Resins Ltd.

Any one of these cationic photopolymerization initiators may be used alone, or any combination of two or more of them may be used at any ratio.

The amount of the cationic photopolymerization initiator in the polymerizable composition of the invention is preferably from 0.02 parts by mass to 20 parts by mass inclusive and more preferably from 0.1 parts by mass to 10 parts by mass inclusive based on 100 parts by mass of the total of all cationically photopolymerizable compounds in the polymerizable composition. If the amount of the cationic photopolymerization initiator is lower than 0.02 parts by mass, the reaction is insufficient. If the amount added exceeds 20 parts by mass, it is difficult to achieve a sufficient pot life and a sufficient polymerization rate simultaneously.

The cationic photopolymerization initiator may be used in combination with the cationic polymerization initiator described above. In this case, the cationic polymerization initiator is used in an amount of generally 0.1 to 10 parts by mass and preferably 1 to 5 parts by mass based on 100 parts by mass of the cationically polymerizable compounds in the polymerizable composition. If the amount of the cationic polymerization initiator used is excessively small, a reduction in the polymerization rate occurs. If the amount is excessively large, the physical properties of the polymer to be obtained may deteriorate.

The cationic photopolymerization initiator may be used in combination with a cationic photopolymerization sensitizer. The cationic photopolymerization sensitizer is a formulation used when light emitted from a light source used for cationic photopolymerization does not match well the absorption wavelength of the cationic photopolymerization initiator. The cationic photopolymerization sensitizer is used in combination with the cationic photopolymerization initiator to transmit the energy of the irradiation light efficiently to the cationic photopolymerization initiator. Known examples of the cationic photopolymerization sensitizer include phenol-based compounds such as methoxyphenol (JP5-230189A), thioxanthone compounds (JP2000-204284A), and dialkoxyanthracene compounds (JP2000-119306A).

The cationic photopolymerization sensitizer is used in an amount of generally 0.2 to 5 parts by mass and preferably 0.5 to 1 part by mass based on 1 part by mass of the cationic photopolymerization initiator. If the amount of the cationic photopolymerization sensitizer is excessively small, the sensitizing effect may not be easily obtained. If the amount is excessively large, the physical properties of the polymer may deteriorate.

2-2. Polymerizable compounds

The polymerizable composition of the invention may contain only one compound of the invention represented by formula (1) as a polymerizable compound or any combination of two or more compounds represented by formula (1) as polymerizable compounds at any ratio.

The polymerizable composition of the invention may contain an additional polymerizable compound other than the compound of the invention.

The content of the compound of the invention in the polymerizable composition of the invention is from 1% mass to 99% by mass inclusive and preferably from 5% mass to 95% by mass inclusive based on the total amount of solids in the polymerizable composition of the invention. If the content of the compound of the invention is less than 1% by mass, the effect obtained by using the compound of the invention is insufficient. If the content exceeds 99% by mass, curability tends to decrease.

Examples of the additional polymerizable compound include cationically polymerizable monomers, anionically polymerizable monomers, and radically polymerizable monomers. Any one of these polymerizable compounds may be used alone, or any combination of two or more of them may be used at any ratio. A polymerizable compound having two or more polymerizable functional groups per molecule (which may be referred to as a polyfunctional monomer) may also be used. When the polyfunctional monomer is used, a crosslinked structure is formed in the polymer, so that thermal stability, weather resistance, solvent resistance, etc. can be improved.

When the polymerizable composition of the invention contains the additional polymerizable compound other than the compound of the invention, the content of the additional polymerizable compound is from 0.1% mass to 10% by mass inclusive and preferably from 0.3% mass to 5% by mass inclusive based on the total amount of solids in the polymerizable composition of the invention. If the content of the additional polymerizable compound is less than 0.1% by mass, the effect of imparting characteristics by the addition of the additional polymerizable compound is not sufficient. If the content exceeds 5% by mass, problems such as a reduction in optical characteristics and a reduction in strength tend to occur.

<Cationically polymerizable monomer>

Examples of the cationically polymerizable monomer include compounds having an oxirane ring, styrene and derivatives thereof, vinylnaphthalene and derivatives thereof, vinyl ethers, N-vinyl compounds, and compounds having an oxetane ring.

In particular, a compound having at least an oxetane ring is preferably used, and a combination of a compound having an oxetane ring and a compound having an oxirane ring is used more preferably.

Examples of the compound having an oxirane ring include prepolymers having two or more oxirane rings per molecule.

Examples of such prepolymers include alicyclic polyepoxies, polyglycidyl esters of polybasic acids, polyglycidyl ethers of polyhydric alcohols, polyglycidyl ethers of polyoxyalkylene glycols, polyglycidyl ethers of aromatic polyols, hydrogenated compounds of polyglycidyl ethers of aromatic polyols, urethane polyepoxy compounds, and epoxidized polybutadienes.

Examples of the styrene and derivatives thereof include styrene, p-methylstyrene, p-methoxystyrene, β-methylstyrene, p-methyl-β-methylstyrene, α-methylstyrene, p-methoxy-β-methylstyrene, and divinylbenzene.
Examples of the vinylnaphthalene and derivatives thereof include 1-vinylnaphthalene, α-methyl-1-vinylnaphthalene, β-methyl-1-vinylnaphthalene, 4-methyl-1-vinylnaphthalene, and 4-methoxy-1-vinylnaphthalene.
Examples of the vinyl ethers include isobutyl ether, ethyl vinyl ether, phenyl vinyl ether, p-methylphenylvinyl ether, and p-methoxyphenylvinyl ether.
Examples of the N-vinyl compounds include N-vinylcarbazole, N-vinylpyrrolidone, N-vinylindole, N-vinylpyrrole, and N-vinylphenothiazine.
Examples of the compounds having an oxetane ring include various known oxetane compounds described in JP2001-220526A, JP2001-310937A, etc.
Any one of these cationically polymerizable monomers may be used alone, or any combination of two or more of them may be used at any ratio.

<Anionically polymerizable monomer>

Examples of the anionically polymerizable monomer include hydrocarbon monomers and polar monomers.
Examples of the hydrocarbon monomers include styrene, α-methylstyrene, butadiene, isoprene, vinylpyridine, vinylanthracene, and derivatives thereof.
Examples of the polar monomers include: methacrylates (such as methyl methacrylate, ethyl methacrylate, and isopropyl methacrylate); acrylates (such as methyl acrylate and ethyl acrylate); vinyl ketones (such as methyl vinyl ketone, isopropyl vinyl ketone, cyclohexyl vinyl ketone, and phenyl vinyl ketone); isopropenyl ketones (such as methyl isopropenyl ketone and phenyl isopropenyl ketone); and other polar monomers (such as acrylonitrile, acrylamide, nitroethylene, methylene malonate, cyanoacrylates, and vinylidene cyanide).
Any one of these anionically polymerizable monomers may be used alone, or any combination of two or more of them may be used at any ratio.

<Radically polymerizable monomer>

The radically polymerizable monomer is a compound having at least one ethylenically unsaturated double bond per molecule, and examples thereof include (meth)acrylates, (meth)acrylamides, vinyl esters, and styrenes.
Examples of the (meth)acrylates include methyl (meth)acrylate, ethyl (meth)acrylate, (n- or i-)propyl (meth)acrylate, (n-, i-, sec-, or t-)butyl (meth)acrylate, amyl (meth)acrylate, adamantyl (meth)acrylate, chloroethyl (meth)acrylate, 2-hydroxyethyl (meth)acrylate, 2-hydroxypropyl (meth)acrylate, 2-hydroxypentyl (meth)acrylate, cyclohexyl (meth)acrylate, allyl (meth)acrylate, trimethylolpropane mono(meth)acrylate, pentaerythritol mono(meth)acrylate, benzyl (meth)acrylate, methoxybenzyl (meth)acrylate, chlorobenzyl (meth)acrylate, hydroxybenzyl (meth)acrylate, hydroxyphenethyl (meth)acrylate, dihydroxyphenethyl (meth)acrylate, furfuryl (meth)acrylate, tetrahydrofurfuryl (meth)acrylate, phenyl (meth)acrylate, hydroxyphenyl (meth)acrylate, chlorophenyl (meth)acrylate, sulfamoylphenyl (meth)acrylate, 2-phenoxyethyl (meth)acrylate, 2-(hydroxyphenylcarbonyloxy)ethyl (meth)acrylate, phenol EO-modified (meth)acrylate, phenylphenol EO-modified (meth)acrylate, paracumylphenol EO-modified (meth)acrylate, nonylphenol EO-modified (meth)acrylate, N-acryloyloxyethylhexahydrophthalimide, bisphenol F EO-modified diacrylate, bisphenol A EO-modified diacrylate, dibromophenyl (meth)acrylate, tribromophenyl (meth)acrylate, dicyclopentenyloxyethyl (meth)acrylate, dicyclopentanyl acrylate, tricyclodecanedimethylol di(meth)acrylate, bisphenoxyethanolfluorene di(meth)acrylate, trimethylolpropane tri(meth)acrylate, pentaerythritol tri(meth)acrylate, pentaerythritol tetra(meth)acrylate, dipentaerythritol penta(meth)acrylate, and dipentaerythritol hexa(meth)acrylate. Here, "EO" means "ethylene oxide."
Examples of the (meth)acrylamides include (meth)acrylamide, N-methyl(meth)acrylamide, N-ethyl(meth)acrylamide, N-propyl(meth)acrylamide, N-butyl(meth)acrylamide, N-benzyl(meth)acrylamide, N-hydroxyethyl(meth)acrylamide, N-phenyl(meth)acrylamide, N-tolyl(meth)acrylamide, N-(hydroxyphenyl)(meth)acrylamide, N-(sulfamoylphenyl)(meth)acrylamide, N-(phenylsulfonyl)(meth)acrylamide, N-(tolylsulfonyl)(meth)acrylamide, N,N-dimethyl(meth)acrylamide, N-methyl-N-phenyl(meth)acrylamide, and N-hydroxyethyl-N-methyl(meth)acrylamide.
Examples of the vinyl esters include vinyl acetate, vinyl butyrate, vinyl benzoate, benzoic acid vinyl ester, vinyl t-butyl benzoate, vinyl chlorobenzoate, vinyl 4-ethoxybenzoate, vinyl 4-ethylbenzoate, vinyl 4-methylbenzoate, vinyl 3-methylbenzoate, vinyl 2-methylbenzoate, vinyl 4-phenylbenzoate, and vinyl pivalate.
Examples of the styrenes include styrene, p-acetylstyrene, p-benzoylstyrene, 2-butoxymethylstyrene, 4-butylstyrene, 4-sec-butylstyrene, 4-tert-butylstyrene, 2-chlorostyrene, 3-chlorostyrene, 4-chlorostyrene, dichlorostyrene, 2,4-diisopropylstyrene, dimethylstyrene, p-ethoxystyrene, 2-ethylstyrene, 2-methoxystyrene, 4-methoxystyrene, 2-methylstyrene, 3-methylstyrene, 4-methylstyrene, p-methylstyrene, p-phenoxystyrene, p-phenylstyrene, and divinylbenzene.
Any one of these radically polymerizable monomers may be used alone, or any combination of two or more of them may be used at any ratio.

Any of the above-exemplified cationically polymerizable monomers, anionically polymerizable monomers, and radically polymerizable monomers may be used, or two or more of them may be used in combination.

For a holographic recording medium, it is preferable to use a radically polymerizable monomer as the additional polymerizable compound used in combination with the compound of the invention represented by formula (1) because a reaction for forming a resin matrix is unlikely to be inhibited.

2-3 Additional additive component

An additional component may be added to the polymerizable composition of the invention so long as the effects of the invention are not impaired.

Examples of the additional component include various additives such as a solvent, an antioxidant, a plasticizer, an ultraviolet absorber, a sensitizer, a chain transfer agent, an antifoaming agent, a polymerization inhibitor, any filler formed of an organic or inorganic material, a dispersing agent, a pigment, and a wavelength conversion material such as a phosphor.

The polymerizable composition of the invention may contain a solvent for the purpose of controlling the viscosity.

The solvent is selected according to the physical properties of the polymerizable composition, and specific examples of the solvent include organic solvents such as: alcohols such as ethanol, propanol, isopropanol, ethylene glycol, and propylene glycol; aliphatic hydrocarbons such as hexane, pentane, and heptane; alicyclic hydrocarbon such as cyclopentane and cyclohexane; aromatic hydrocarbons such as toluene and xylene; halogenated hydrocarbons such as methylene chloride and chloroform; chain ethers such as dimethyl ether and diethyl ether; cyclic ethers such as dioxane and tetrahydrofuran; esters such as methyl acetate, ethyl acetate, butyl acetate, ethyl lactate, and ethyl butyrate; ketones such as acetone, ethyl methyl ketone, methyl isobutyl ketone, and cyclohexanone; cellosolves such as methyl cellosolve, ethyl cellosolve, and butyl cellosolve; carbitols such as methyl carbitol, ethyl carbitol, and butyl carbitol; propylene glycol monoalkyl ethers such as propylene glycol monomethyl ether, propylene glycol monoethyl ether, and propylene glycol mono-n-butyl ether; glycol ether esters such as ethylene glycol monomethyl ether acetate and propylene glycol monomethyl ether acetate; N,N-dimethylformamide; N,N-dimethylacetamide); sulfoxides (amides such as dimethyl sulfoxide; nitriles such as acetonitrile and benzonitrile; and N-methylpyrrolidone.

Any of these solvents may be used alone, or a solvent mixture thereof may be used. Water may be used for some polymerization methods (such as emulsion polymerization and suspension polymerization).

No particular limitation is imposed on the amount of the solvent (or a dispersion medium) used. The solvent may be used such that the polymerizable composition has a viscosity suitable for the polymerization method, a processing method, or its intended application.

In the present invention, it is preferable that an antioxidant or light stabilizer used as an additive is added to the polymerizable composition in order for the polymer to be obtained to have good resistance to thermal yellowing or good weatherability.

Specific examples of the antioxidant include: phenol-based antioxidants such as 2,6-di-t-butylphenol, 2,6-di-t-butyl-p-cresol, n-octadecyl-3-(3',5'-di-t-butyl-4'-hydroxyphenyl)propionate, tetrakis-[methylene-3-(3',5'-di-t-butyl-4'-hydroxyphenyl)propionate]methane, triethylene glycol bis[3-(3-t-butyl-5-methyl-4-hydroxyphenyl)propionate], and 1,6-hexanediol bis[3-(3,5-dit-butyl-4-hydroxyphenyl)propionate]; and phosphorus-based antioxidants such as triphenyl phosphite, trisisodecyl phosphite, isodecyldiphenyl phosphite, 2-ethylhexyldiphenyl phosphite, tetra(C12-C15alkyl)-4,4'-isopropylidenediphenyl diphosphite, tris(nonylphenyl)phosphite, tristridecyl phosphite, 2,4,8,10-tetra-tert-butyl-6-[(2-ethylhexan-1-yl)oxy]-12H-dibenzo[d,g][ 1,3,2]dioxaphosphosine, 3,9-bis(2,6-di-tert-butyl-4-methylphenoxy)-2,4,8,10-tetraoxa-3,9-diphosphaspiro[5.5]undecane, 3,9-dioctadecan-1-yl-2,4,8,10-tetraoxa-3,9-diphosphaspiro[5.5]undecane, and tris(2,4-di-t-butylphenyl)phosphite. One of these may be used alone, or a combination of two or more may be used.

Preferably, the antioxidant used is a combination of a phenol-based antioxidant and a phosphorus-based antioxidant. Preferred examples of the combination of the phenol-based antioxidant and the phosphorus-based antioxidant include a combination of tris(2,4-di-t-butylphenyl) phosphite used as the phosphorus-based antioxidant and at least one phenol-based antioxidant selected from tetrakis-[methylene-3-(3',5'-di-t-butyl-4'-hydroxyphenyl)propionate]methane and n-octadecyl-3-(3',5'-di-t-butyl-4'-hydroxyphenyl)propionate.

From the viewpoint of allowing the polymer to be obtained to have good resistance to thermal yellowing, the amount of the antioxidant added to the polymerizable composition of the invention is preferably 0.01 to 5 parts by mass, more preferably 0.05 to 3 parts by mass, and still more preferably 0.1 to 2 parts by mass based on 100 parts by mass of the total amount of the polymerizable composition.

The light stabilizer used is preferably a hindered amine-based light stabilizer (HALS). Specific examples of the HALS include 2,2,6,6-tetramethyl-4-piperidinyl stearate, 2,2,6,6-tetramethyl-4-piperidyl methacrylate, 1,2,2,6,6-pentamethyl-4-piperidyl methacrylate, bis(2,2,6,6-tetramethyl-1-undecyloxypiperidin-4-yl)carbonate, sebacic acid bis(2,2,6,6-tetramethyl-4-piperidyl) ester, sebacic acid bis(1,2,2,6,6-pentamethyl-4-piperidyl) ester, ADEKA STAB LA-68 (manufactured by ADEKA Corporation), ADEKA STAB LA-63P (manufactured by ADEKA Corporation), butane-1,2,3,4-tetracarboxylic acid tetrakis(1,2,2,6,6-pentamethyl-4-piperidinyl) ester, 1,2,3,4-butanetetracarboxylic acid tetrakis(2,2,6,6-tetramethyl-4-piperidinyl) ester, and TINUVIN 111FDL, TINUVIN 123, TINUVIN 144, TINUVIN 152, TINUVIN 249, TINUVIN 292, and TINUVIN 5100 (which are manufactured by BASF). One of them may be used alone, or two or more of them may be used in combination.

The mixing amount of the light stabilizer in the polymerizable composition of the invention is preferably 0.01 to 5 parts by mass, more preferably 0.05 to 3 parts by mass, and still more preferably 0.1 to 2 parts by mass based on 100 parts by mass of the total mass of the polymerizable composition in order for a polymer to be obtained to have good resistance to thermal yellowing and good weather resistance.

One of the above antioxidants and one of the above light stabilizers may be used. A combination of two or more of the antioxidants may be used, and a combination of two or more of the light stabilizers may be used.

2-4 Method for producing polymerizable composition

The polymerizable composition of the invention may be produced by mixing the components or may be produced by mixing components other than the polymerization initiator in advance and adding the polymerization initiator immediately before the polymerization reaction.

3. Method for polymerizing polymerizable composition of invention

No particular limitation is imposed on the method for polymerizing the polymerizable composition of the invention. Examples of the method include a polymerization method using active energy ray irradiation and a thermal polymerization method.

3-1. Polymerization initiation method (active energy rays)

When the polymerizable composition of the invention is subjected to photo radical polymerization, the polymerizable composition is irradiated with active energy rays.

Preferably, the active energy rays used are an electron beam or light in the ultraviolet to infrared wavelength range. An extra-high pressure mercury light source or a metal halide light source can be used when the active energy rays are ultraviolet rays, and a metal halide light source or a halogen light source can be used when the active energy rays are visible light. A halogen light source can be used when the active energy rays are infrared light. In addition, light sources such as lasers and LEDs can also be used.

The dose of the active energy rays is appropriately set according to the type of light source, the thickness of a coating, etc. and is appropriately set such that the total reaction rate of the polymerizable groups in the compound of the invention represented by formula (1) and other polymerizable compounds is preferably 80% or more and more preferably 90% or more. The reaction rate is computed from changes in the intensities of the absorption peaks attributed to the polymerizable groups in an infrared absorption spectrum before and after the reaction.

After the polymerization under irradiation with the active energy rays, heat treatment or annealing treatment may be optionally performed to allow the polymerization to further proceed. The heating temperature in this case is preferably in the range of 80 to 200°C. The heating time is preferably in the range of 10 to 60 minutes.

3-2. Polymerization initiation method (heating)

When the polymerizable composition of the invention is subjected to heat treatment for polymerization, the heating temperature is preferably in the range of 80 to 200°C and more preferably in the range of 100 to 150°C. If the heating temperature is lower than 80°C, it is necessary to increase the heating time, and cost efficiency tends to decrease. If the heating temperature is higher than 200°C, the cost of energy is high, and the heating time and cooling time are long, so that cost efficiency tends to decrease.

4. Polymer

The polymer of the invention produced by polymerizing the polymerizable composition of the invention will be described.

4-1. Refractive index

Generally, the polymerization reaction causes the overall density to increase. Therefore, the refractive index of the polymer tends to be higher than the refractive index of the unpolymerized compound that is a precursor of the

polymer (which is referred to as a monomer). When a monomer having a high refractive index is used and its polymerization reaction is allowed to proceed sufficiently, the polymer obtained can have a high refractive index. It is therefore thought to be important to increase the refractive index of the polymer by appropriately designing the molecular structure of the monomer.

The value of the refractive index is high when it is evaluated using irradiation light with a short wavelength. A sample that exhibits a relatively high refractive index at a short wavelength also exhibits a relatively large refractive index at a high wavelength, and this relation is not reversed. Therefore, by evaluating the refractive indexes of materials at a certain wavelength for comparison, the magnitudes of the intrinsic refractive indexes of the materials can be compared. In the present invention, the value at an irradiation wavelength of 587 nm is used as a reference.

The refractive index of the polymer of the invention is preferably 1.55 or more, more preferably 1.60 or more, particularly preferably 1.63 or more, and most preferably 1.65 or more. No particular limitation is imposed on the upper limit of the refractive index of the polymer of the invention, but the refractive index is generally 2.0 or less.

The polymer of the invention can be used as an optical material for lenses etc. In this case, if the refractive index of the polymer is lower than 1.55, central portions of the lenses etc. are thick. This is not preferred because the lightweight of the plastic, which is one of its features, is not utilized. To develop precision optical members such as lenses, it is also important to use a combination of optical materials having a plurality of refractive indexes to thereby obtain optical characteristics suitable for the members. From this point of view, it can be said that a polymer having a refractive index of 1.63 or higher is a material particularly useful for optical components.

When the polymer of the invention is used as a recording layer material of a holographic recording medium, the refractive of the polymer of the invention is generally in the range of from 1.65 to 1.78 inclusive and preferably 1.77 or less. If the refractive index is less than 1.65, the diffraction efficiency is low, and multiplicity is insufficient. If the refractive index is larger than 1.78, the difference in the refractive index between the polymer and the matrix resin is excessively large. In this case, strong scattering occurs, and therefore transmittance decreases, so that larger energy is required for recording and reproduction.

4-2. Glass transition temperature

The glass transition temperature of the polymer of the invention is preferably 90°C or higher, more preferably 100°C or higher, still more preferably 110°C or higher, and particularly preferably 120°C or higher and is preferably 250°C or lower, more preferably 220°C or lower, and still more preferably 200°C or lower. If the glass transition temperature is below the above range, the optical properties may deviate from the design values in the use environment, and the heat resistance may not satisfy that required for practical use. If the glass transition temperature is above the above range, the processability of the polymer is low. In this case, a molded product having good appearance and high dimensional accuracy may not be obtained, and the polymer becomes brittle. Therefore, the mechanical strength decreases, and the handleability of the molded product may deteriorate.

5. Optical material and optical component

The compound of the invention, the polymerizable composition of the invention, and the polymer of the invention have properties such as a high refractive index, easy processability, and a low shrinkage factor and can therefore be applied to various optical materials and optical components.

Examples of the optical material include overcoats for optical use, hard coat agents, adhesives for optical members, resins for optical fibers, and acrylic-based resin reformers.

Examples of the optical component include lenses, filters, diffraction gratings, prisms, optical guides, glass covers for display devices, photosensors, photoswitches, LEDs, light-emitting elements, optical waveguides, optical splitters, optical fiber adhesives, substrates for display elements, substrates for color filters, substrates for touch panels, polarizing plates, display backlights, light guide plates, antireflective films, viewing angle widening films, optical recording, optical fabrication, and optical relief printing.

Moreover, the polymer of the invention can be used for a layer in any of the above components. Examples of such a layer include a display protective films.

In particular, the polymer of the invention is preferably applicable to plastic lenses because of the high-refractive index characteristics of the polymer. Examples of the lenses include imaging lenses of cameras (vehicle-mounted cameras, digital cameras, PC cameras, mobile phone camaras, monitoring cameras, etc.), eyeglass lenses, light beam condensing lenses, and beam diverging lenses.

A lens formed using the polymer of the invention may be optionally subjected to physical or chemical treatment such as surface polishing, antistatic treatment, hard coating treatment, antireflective coating treatment, or staining treatment for the purpose of preventing reflection, imparting high hardness, improving wear resistance, imparting chemical resistance, imparting anti-fogging properties, imparting fashionability, etc.

6. Holographic recording medium

The polymerizable composition of the invention can be preferably used for a recording layer of a holographic recording medium. In this case, the polymerizable composition of the invention is preferably a photoreactive composition containing, in addition to the compound of the invention, a matrix resin, a photopolymerization initiator, a radical scavenger and other additives. The details of these materials when they are used as materials for a holographic recording medium will be described.

6-1. Matrix resin

Preferably, the polymerizable composition of the invention contains a matrix resin. In particular, the matrix resin forming a recording layer of a holographic recording medium is an organic material that is not largely modified chemically and physically under irradiation with light and is formed mainly of a polymer of an organic compound.

The matrix resin, together with the above-described polymerizable compound, a photopolymerization initiator described later, etc., forms the polymerizable composition of the invention and is therefore strongly required to have good compatibility with the polymerizable compound, the photopolymerization initiator, etc. If the compatibility between the matrix resin and the other components is low, interfaces are formed between the materials, and reflection and refraction of light occurs at the interfaces. This causes leakage of light to unintended portions. Therefore, the interference fringes are distorted or broken, and recording may be performed in unwanted portions, so that deterioration in information may occur. The compatibility between the matrix resin and the other components can be evaluated based on, for example, light scattering intensity obtained by a detector disposed in a direction different from the direction of light passing through a sample, as described in, for example, Japanese Patent No. 3737306.

The matrix resin in the polymerizable composition of the invention may be a resin that includes a plurality of materials soluble in a solvent in the polymerizable composition and is to be three-dimensionally crosslinked after shaped into a usable form. Examples of such a resin include thermoplastic resins, thermosetting resins, and photocurable resins described below.

A three-dimensionally crosslinked resin is insoluble in a solvent and is a reaction cured product of a polymerizable compound that is liquid at room temperature and a compound having reaction activity with the polymerizable compound. The three-dimensionally crosslinked resin serves as physical obstacles and therefore reduces a volume change during recording. Specifically, in the recording layer after recording, bright portions are expanded, and dark portions are shrunk, so that irregularities tend to be formed on the surface of the holographic recording medium. To reduce the volume change, it is more preferable that a polymerizable composition containing a three-dimensionally crosslinked resin matrix is used for the recording layer.

In particular, from the viewpoint of adhesion to a support, the matrix resin is preferably a thermosetting resin. Resin materials that can be used as the matrix resin will be described in detail.

6-1-1. Thermoplastic resin

Specific examples of the thermoplastic resin material include chlorinated polyethylene, polymethyl methacrylate resins (PMMA), copolymers of methyl methacrylate with other alkyl acrylates, copolymers of vinyl chloride with acrylonitrile, polyvinyl acetate resins (PVAC), polyvinyl alcohol, polyvinyl formal, polyvinylpyrrolidone, cellulose resins such as ethylcellulose and nitrocellulose, polystyrene resins, and polycarbonate resins. One of these resins may be used alone, or a mixture of two or more may be used.

No particular limitation is imposed on the solvent for these thermoplastic resins so long as it can dissolve these resins. Examples of such a solvent include: ketones such as acetone and methyl ethyl ketone; esters such as butyl acetate and propylene glycol methyl ether acetate; aromatic hydrocarbons such as toluene and xylene; ethers such as tetrahydrofuran and 1,2-dimethoxyethane; and amides such as N,N-dimethylacetamide and N-methylpyrrolidone. Only one of these solvents may be used alone, or a combination of two or more may be used.

6-1-2. Thermosetting resin

When the matrix resin used is a thermosetting resin, its curing temperature varies largely depending on the type of crosslinking agent and the type of catalyst.

Representative examples of the combination of functional groups that allows the matrix resin to cure at room temperature include a combination of an epoxy and an amine, a combination of an epoxy and a thiol, and a combination of an isocyanate and an amine. Representative example of the combination when a catalyst is used include a combination of an epoxy and a phenol, a combination of an epoxy and an acid anhydride, and a combination of an isocyanurate and a polyol.

The former is simple because the reaction starts immediately after mixing. However, when the matrix resin is formed into, for example, a holographic recording medium, the matrix resin is cured while shaped into the holographic recording medium, and it is difficult to control the shape because there is only a limited time available for the formation of the holographic recording medium. In the latter case, the curing temperature and the curing time can be freely selected by appropriately selecting the type of catalyst and the amount of the catalyst used, and this is suitable for the case in which the thermosetting resin is cured while shaped into, for example, a holographic recording medium. Various resin raw materials including low molecular weight to large molecular weight materials are commercially available. Therefore, a suitable raw material can be selected such that the compatibility with a polymerizable reactive compound and a photo initiator and the adhesion to a substrate are maintained.

Each of the raw materials will next be described. For each raw material, one type may be used alone, or two or more types may be used in combination.

<Epoxy>

Examples of the epoxy include: polyglycidyl ether compounds of polyols such as (poly)ethylene glycol, (poly)propylene glycol, (poly)tetramethylene glycol, trimethylolpropane, and glycerin; alicyclic epoxy compounds having a 4 to 7-membered cyclic aliphatic group such as 3,4-epoxycyclohexylmethyl-3,4-epoxycyclohexanecarboxylate and 3,4-epoxy-1-methylcyclohexyl-3,4-epoxy-1-methylhexanecarboxylate; bisphenol A-type epoxy compounds; hydrogenated bisphenol A-type epoxy compounds; bisphenol F-type epoxy compounds; and phenol and cresol novolac-type epoxy compounds.

Preferably, the epoxy has two or more epoxy groups per molecule, but no particular limitation is imposed on the type of epoxy. If the number of epoxy groups is small, hardness necessary for the matrix may not be obtained. No particular limitation is imposed on the upper limit of the number of epoxy groups per molecule. The number of epoxy groups is generally 8 or less and preferably 4 or less. If the number of epoxy groups is excessively large, it takes a long time to consume the epoxy groups, and an excessively long time is necessary to form the matrix resin.

<Amine>

The amine used may contain a primary amino group or a secondary amino group. Examples of such an amine include: aliphatic polyamines such as ethylenediamine and diethylenetriamine and derivatives thereof; alicyclic polyamines such as isophoronediamine, menthanediamine, and N-aminoethylpiperazine and derivatives thereof; aromatic polyamines such as m-xylylenediamine and diaminodiphenylmethane and derivatives thereof; polyamides such as a condensation product of a dicarboxylic acid such as dimer acid and any of the above polyamines; imidazole compounds such as 2-methylimidazole and derivatives thereof; dicyandiamide; and adipic acid dihydrazide.

<Thiol>

Examples of the thiol include: dithiols such as 1,3-butanedithiol, 1,4-butanedithiol, 2,3-butanedithiol, 1,2-benzenedithiol, 1,3-benzenedithiol, 1,4-benzenedithiol, 1,10-decanedithiol, 1,2-ethanedithiol, 1,6-hexanedithiol, and 1,9-nonanedithiol; and thiol compounds including polythiols etc. such as THIOKOL (manufactured by Toray Fine Chemicals Co., Ltd.) and jERCURE QX40 (manufactured by Mitsubishi Chemical Corporation). Of these, commercial fast curable polythiols such as jERCURE QX40 are used preferably.

<Phenol>

Examples of the phenol include phenolic resins such as bisphenol A and novolac type phenolic resins and resol type phenolic resins.

<Acid anhydride>

Examples of the acid anhydride include: monofunctional acid anhydrides such as phthalic anhydride and tetrahydrophthalic anhydride and derivatives thereof; and bifunctional acid anhydrides such as pyromellitic anhydride and benzophenonetetracarboxylic anhydride and derivatives thereof.

<Amounts of amine, thiol, phenol, and acid anhydride used>

The ratios of the amounts of the amine, thiol, phenol, and acid anhydride used to the number of moles of the epoxy groups are generally 0.1 equivalents or more and preferably 0.7 equivalents or more and is generally 2.0 equivalents

or less and preferably 1.5 equivalents or less. If the amounts of the amine, thiol, phenol, and acid anhydride used are excessively small or are excessively large, the number of unreacted functional groups is large, and the storage stability may be impaired.

<Polymerization initiator for thermosetting resin>

An anionic polymerization initiator or a cationic polymerization initiator selected according to the curing temperature and the curing time may be used as a catalyst for curing the thermosetting resin.
The anionic polymerization initiator generates anions under the application of heat or irradiation with light, and examples thereof include amines. Examples of the amines include: amino group-containing compounds such as dimethylbenzylamine, dimethylaminomethylphenol, and 1,8-diazabicyclo[5.4.0]undecene-7 and derivatives thereof; and imidazole compounds such as imidazole, 2-methylimidazole, and 2-ethyl-4-methylimidazole and derivatives thereof. One or a plurality of them may be used according to the curing temperature and the curing time.
The cationic polymerization initiator generates cations under the application of heat or irradiation with light, and examples thereof include aromatic onium salts. Specific examples include compounds containing an anionic component such as $SbF_6$-, $BF_4$-, $AsF_6$-, $PF_6$-, $CF_3SO_3$-, or $B(C_6F_5)_4$- and an aromatic cationic component containing an iodine atom, a sulfur atom, a nitrogen atom, a phosphorus atom, etc. In particular, diaryliodonium salts, triarylsulfonium salts, etc. are preferred. One or a plurality of them may be used according to the curing temperature and the curing time.
The amount used of the polymerization initiator for the thermosetting resin relative to the amount of the matrix resin is generally 0.001% mass or more and preferably 0.01% mass or more and is generally 50% by mass or less and preferably 10% by mass or less. If the amount used of the polymerization initiator for the thermosetting resin is excessively small, the concentration of the polymerization initiator for the thermosetting resin is excessively small, so that the polymerization may take an excessively long time. If the amount used of the polymerization initiator for the thermosetting resin is excessively large, a continuous ring-opening reaction, which is a polymerization reaction, may not occur.

<Isocyanate>

Preferably, the isocyanate includes two or more isocyanate groups per molecule, and no particular limitation is imposed on the type of isocyanate. If the number of isocyanate groups per molecule is small, hardness necessary for the matrix resin may not be obtained. No particular limitation is imposed on the upper limit of the number of isocyanate groups per molecule, but the number of isocyanate groups is generally 8 or less and preferably 4 or less. If the number of isocyanate groups per molecule is excessively large, it takes a long time to consume the isocyanate groups, and an excessively long time may be necessary to form the matrix resin. No particular limitation is imposed on the upper limit of the number of isocyanate groups per molecule, but the number of isocyanate groups is generally about 20 or less.
Examples of the isocyanate include: aliphatic isocyanates such as hexamethylene diisocyanate, lysine methyl ester diisocyanate, and 2,4,4-trimethylhexamethylene diisocyanate; alicyclic isocyanates such as isophorone diisocyanate and 4,4'-methylenebis(cyclohexyl isocyanate); aromatic isocyanates such as tolylene diisocyanate, 4,4'-diphenylmethane diisocyanate, xylylene diisocyanate, and naphthalene-1,5'-diisocyanate; and multimers thereof. In particular, trimers to heptamers thereof are preferred.
Other examples include: reaction products of any of the above isocyanates with water and polyhydric alcohols such as trimethylolethane and trimethylolpropane; and multimers of hexamethylene diisocyanate and derivatives thereof.
As for the molecular weight of the isocyanate, its number average molecular weight is preferably from 100 to 50000 inclusive, more preferably from 150 to 10000 inclusive, and still more preferably from 150 to 5000 inclusive. If the number average molecular weight is excessively small, the crosslinking density increases. In this case, the hardness of the matrix resin is excessively high, and the recording speed may decrease. If the number average molecular weight is excessively large, the compatibility with other components decreases, and the crosslinking density decreases. In this case, the hardness of the matrix resin is excessively low, and recorded contents may be lost.

<Polyol>

Examples of the polyol include polypropylene polyols, polycaprolactone polyols, polyester polyols, and polycarbonate polyols.

(Polypropylene polyol)

The polypropylene polyol is obtained by a reaction of propylene oxide with a diol or a polyhydric alcohol. Examples of the diol and the polyhydric alcohol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol. Commercial examples of the polypropylene polyol include: SANNIX GP-400 and GP-1000 (product names, products of Sanyo Chemical Industries, Ltd.); and ADEKA POLYETHER G400, G700, and G1500 (product names, products of ADEKA CORPORATION).

(Polycaprolactone polyol)

The polycaprolactone polyol is obtained by a reaction of a lactone with a diol or a polyhydric alcohol. Examples of the lactone include α-caprolactone, β-caprolactone, γ-caprolactone, ε-caprolactone, α-methyl-ε-caprolactone, and β-methyl-ε-caprolactone.

Examples of the diol and the polyhydric alcohol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol.

Commercial examples of the polycaprolactone polyol obtained by a reaction of ε-caprolactone include PLACCEL 205, PLACCEL 205H, PLACCEL 205U, PLACCEL 205UT, PLACCEL 210, PLACCEL 210N, PLACCEL 210CP, PLACCEL 220, PLACCEL 230, PLACCEL 230N, PLACCEL 240, PLACCEL 220EB, PLACCEL 220EC, PLACCEL 303, PLACCEL 305, PLACCEL 308, PLACCEL 309, PLACCEL 312, PLACCEL 320, PLACCEL 401, PLACCEL L205AL, PLACCEL L212AL, PLACCEL L220AL, PLACCEL L320AL, PLACCEL T2103, PLACCEL T2205, and PLACCEL P3403 (product names, products of Daicel Corporation).

(Polyester polyol)

The polyester polyol is obtained, for example, by polycondensation of a dicarboxylic acid or an anhydride thereof with a polyol.

Examples of the dicarboxylic acid include succinic acid, adipic acid, sebacic acid, azelaic acid, dimer acid, maleic anhydride, isophthalic acid, terephthalic acid, and trimellitic acid.

Examples of the polyol include ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, polyethylene glycol, and polytetramethylene glycol.

Examples of the polyester polyol include polyethylene adipate, polybutylene adipate, and polyhexamethylene adipate. Commercial examples of the polyester polyol include: ADEKA NEWACE F series, ADEKA NEWACE Y series, and ADEKA NEWACE NS series (product names, products of ADEKA CORPORATION); and Kuraray Polyol N-2010, P-4011, and P-1020 (produce names, products of KURARAY Co., Ltd.).

(Polycarbonate polyol)

Examples of the polycarbonate polyol include; polyols obtained by a dealcoholization condensation reaction of glycols with dialkyl carbonates (such as dimethyl carbonate and diethyl carbonate); polyols obtained by a dephenolization condensation reaction of glycols with diphenyl carbonates; and polyols obtained by a deglycolization condensation reaction of glycols with carbonates (such as ethylene carbonate and diethyl carbonate).

Examples of the glycols include: aliphatic diols such as 1,6-hexanediol, diethylene glycol, propylene glycol, 1,4-butanediol, 3-methyl-1,5 pentanediol, and neopentyl glycol; and alicyclic diols such as 1,4-cyclohexanediol and 1,4-cyclohexanedimethanol.

Examples of the polycarbonate polyol include: poly(hexamethylene carbonate)polyol obtained by a condensation reaction of 1,6-hexanediol with diethyl carbonate; poly(pentylene carbonate) obtained by a condensation reaction of pentanediol with diethyl carbonate; and poly(butylene carbonate) obtained by a condensation reaction of 1,4-butanediol with diethyl carbonate.

Commercial examples of the polycarbonate polyol include: PLACCEL CD CD205, PLACCEL CD CD210, and PLACCEL CD CD220 (product names, products of Daicel Corporation); and DURANOL T5651, DURANOL T5652, and DURANOL T5650J (product names, products of Asahi Kasei Corporation).

(Molecular weight of polyol)

As for the molecular weight of the polyol described above, its number average molecular weight is preferably from 100 to 50000 inclusive, more preferably from 150 to 10000 inclusive, and still more preferably from 150 to 5000 inclusive. If the number average molecular weight is excessively small, the crosslinking density increases. In this case, the hardness of the matrix resin is excessively high, and the recording speed may decrease. If the number average molecular weight is excessively large, the compatibility with other components decreases, and the crosslinking density decreases. In this case, the hardness of the matrix resin is excessively low, and recorded contents may be lost.

<Additional components>

The matrix resin in the present embodiment may contain, in addition to the components described above, additional components so long as the gist of the invention is retained.

Examples of the additional components include compounds having a hydroxyl group such as ethylene glycol, propylene glycol, 1,4-butanediol, 1,5-pentanediol, 3-methyl-1,5-pentanediol, 1,6-hexanediol, neopentyl glycol, diethylene glycol, 1,4-cyclohexanediol, 1,4-cyclohexanedimethanol, decamethylene glycol, trimethylolpropane, polyethylene glycol, and polytetramethylene glycol. These compounds are used for the purpose of changing the physical properties of the matrix resin.

<Urethane polymerization catalyst>

To facilitate the reaction of the isocyanate with the polyol, an appropriate urethane polymerization catalyst may be contained.

Examples of the urethane polymerization catalyst include: onium salts such as bis(4-t-butylphenyl)iodonium perfluoro-1-butanesulfonate, bis(4-t-butylphenyl)iodonium p-toluenesulfonate, bis(4-t-butylphenyl)iodonium trifluoromethanesulfonate, (4-bromophenyl)diphenylsulfonium triflate, (4-t-butylphenyl)diphenylsulfonium trifluoromethanesulfonate, diphenyliodonium perfluoro-1-butanesulfonate, (4-fluorophenyl)diphenylsulfonium trifluoromethanesulfonate, diphenyl-4-methylphenylsulfonium trifluoromethanesulfonate, triphenylsulfonium trifluoromethanesulfonate, and bis(alkylphenyl)iodonium hexafluorophosphonate; catalysts prepared using Lewis acids as main components such as zinc chloride, tin chloride, iron chloride, aluminum chloride, and $BF_3$; proton acids such as hydrochloric acid and phosphoric acid; amines such as trimethylamine, triethylamine, triethylenediamine, dimethylbenzylamine, and diazabicycloundecene; imidazoles such as 2-methylimidazole, 2-ethyl-4-methylimidazole, and 1-cyanoethyl-2-undecylimidazolinium trimellitate; bases such as sodium hydroxide, potassium hydroxide, and potassium carbonate; tin catalysts such as dibutyltin laurate, dioctyltin laurate, and dibutyltin octoate; bismuth catalysts such as bismuth tris(2-ethylhexanoate) and tribenzoyloxy bismuth; and zirconium catalysts such as zirconium tetrakis(ethylacetoacetate), 1,1'-isopropylidenezirconocene dichloride, and zirconium tetrakis(2,4-pentanedionato) .

Of these, bismuth catalysts and zirconium catalysts are preferred in order to improve storage stability.

No particular limitation is imposed on the bismuth-based catalyst so long as it is a catalyst containing elemental bismuth and is a compound that facilitates the reaction of the isocyanate with the polyol.

Examples of the bismuth-based catalyst include bismuth tris(2-ethylhexanoate), tribenzoyloxy bismuth, bismuth triacetate, bismuth tris(dimethyldithiocarbamate), bismuth hydroxide, triphenylbismuth(V) bis(trichloroacetate), tris(4-methylphenyl)oxobismuth(V), and triphenylbis(3-chlorobenzoyloxy)bismuth(V).

In particular, a trivalent bismuth compound is preferred in terms of catalytic activity, and bismuth carboxylate, i.e., a compound represented by general formula $Bi(OCOR)_3$ ((R is a linear or branched alkyl group, a cycloalkyl group, or a substituted or unsubstituted aromatic group), is more preferred. Any one of these bismuth-based catalysts may be used alone, or any combination of two or more of them may be used at any ratio.

No particular limitation is imposed on the zirconium-based catalyst so long as it is a catalyst containing elemental zirconium and is a compound that facilitates the reaction of the isocyanate with the polyol.

Examples of the zirconium-based catalyst include cyclopentadienylzirconium trichloride, decamethylzirconocene dichloride, 1,1'-dibutylzirconocene dichloride, 1,1'-isopropylidenezirconocene dichloride, tetrakis(2,4-pentanedionato)zirconium, tetrakis(trifluoro-2,4-pentanedionato)zirconium, tetrakis(hexafluoro-2,4-pentanedionato)zirconium, zirconium butoxide, zirconium-t-butoxide, zirconium propoxide, zirconium isopropoxide, zirconium ethoxide, bis(ethylacetoacetate)dibutoxy zirconium, tetrakis(ethylacetoacetate)zirconium, zirconium oxide, barium zirconium oxide, calcium zirconium oxide, zirconium bromide, zirconium chloride, zirconium fluoride, (indenyl)zirconium dichloride, and zirconium carbonate.

In particular, compounds having an organic ligand are preferred in terms of compatibility with other components, and compounds having an alkoxide structure or an acetylacetonate (2,4-pentanedionato) structure are more preferred. Any one of the above zirconium compounds may be used alone, or any combination of two or more of them may be used at any ratio.

One of the bismuth-based catalysts and the zirconium-based catalysts may be used alone, or any mixture of them may be used.

The ratio of the amount of the urethane polymerization catalyst used to the amount of the matrix resin is generally 0.0001% mass or more and preferably 0.001% mass or more and is generally 10% by mass or less and preferably 5% by mass or less. If the amount of the urethane polymerization catalyst used is excessively small, an excessively long time may be necessary for curing. If the amount used is excessively large, it may be difficult to control the curing reaction.

The use of the urethane polymerization catalyst allows curing at room temperature. However, the curing may be performed at increased temperature. The temperature in this case is preferably between 40°C to 90°C.

6-1-3. Photocurable resin

When the matrix resin used is a photocurable resin, it is necessary to cure the matrix resin using a photo-initiator for the matrix resin suitable for the wavelength used. During curing of the matrix resin under irradiation with light, defective forming or poor bonding may occur. It is therefore desirable that the curing reaction is stable at around room temperature, which is main working temperature. In consideration of this, catalytic curing using the photo-initiator for the matrix resin is a desirable choice.

An active species, i.e., cations or anions, is generally generated from the photo-initiator for the matrix resin under irradiation with light. It is therefore preferable that a photocurable resin that is cured by such an active species is selected as the matrix resin.

Examples of the functional group reactive with cations such as protons include an epoxy group and an oxetanyl group. Specific examples of a compound having an epoxy group include: polyglycidyl ether compounds of polyols such as (poly)ethylene glycol, (poly)propylene glycol, (poly)tetramethylene glycol, trimethylolpropane, and glycerin; alicyclic epoxy compounds having a 4- to 7-membered cyclic aliphatic group such as 3,4-epoxycyclohexylmethyl-3,4-epoxycyclohexanecarboxylate and 3,4-epoxy-1-methylcyclohexyl-3,4-epoxy-1-methylhexanecarboxylate; bisphenol A-type epoxy compounds; hydrogenated bisphenol A-type epoxy compounds; bisphenol F-type epoxy compounds; and phenol and cresol novolac-type epoxy compounds. Examples of a compound having an oxetanyl group include; 2-ethyl-2-oxetanyl ether of bisphenol A; and 1,6-bis(2-ethyl-2-oxetanyloxy)hexane. (Note that the term "(poly)ethylene glycol," for example, means both "ethylene glycol" and "polyethylene glycol" which is a polymer of ethylene glycol.)

Examples of the functional group reactive with anions include an epoxy group and an episulfide group. Specific examples of a compound having an episulfide group include phenyl episulfide and diepisulfide methyl ether of bisphenol A.

The ratio of the amount of the photo-initiator for the matrix resin that is used to photo-cure the matrix resin to the amount of the polymerizable compound is generally 0.01% mass or more and preferably 0.1% mass or more and is generally 1% by mass or less and preferably 0.5% by mass or less. If the amount used of the photo-initiator for the matrix resin is excessively small, an excessively long time may be necessary for curing. If the amount used is excessively large, it may be difficult to control the curing reaction.

In particular, when the polymerizable composition is used as a holographic recording material, the polymerizable composition is irradiated with light also during recording. It is therefore important that the wavelength for curing be different from the wavelength for recording, and the difference in wavelength is at least 10 nm and preferably 30 nm. The selection of the photo-initiator for the matrix resin can be roughly estimated from the absorption wavelength of the initiator.

6-2. Photopolymerization initiator

Any known photo radical polymerization initiator can be used as the photopolymerization initiator that assists the polymerization of the compound of the invention. Examples include azo-based compounds, azide-based compounds, organic peroxides, organic borates, onium salts, bisimidazole derivatives, titanocene compounds, iodonium salts, organic thiol compounds, halogenated hydrocarbon derivatives, acetophenones, benzophenones, hydroxybenzenes, thioxanthones, anthraquinones, ketals, acylphosphine oxides, sulfone compounds, carbamic acid derivatives, sulfonamides, triarylmethanols, and oxime esters. In particular, the photopolymerization initiator is preferably a titanocene compound, an acylphosphine oxide compound, an oxime ester compound, etc. because the polymerization reaction proceeds under light in the visible range.

6-2-1. Titanocene compound

When a titanocene compound is used as the photopolymerization initiator, no particular limitation is imposed on the type of titanocene compound. For example, one selected from various titanocene compounds described in JP59-152396A, JP61-151197A, etc. can be appropriately used.

Specific examples of the titanocene compound include di-cyclopentadienyl-Ti-di-chloride, di-cyclopentadienyl-Ti-bis-phenyl, di-cyclopentadienyl-Ti-bis-2,3,4,5,6-pentafluorophen-1-yl, dicyclopentadienyl-Ti-bis-2,3,5,6-tetrafluorophen-1-yl, di-cyclopentadienyl-Ti-bis-2,4,6-trifluorophen-1-yl, di-cyclopentadienyl-Ti-bis-2,6-di-fluorophen-1-yl, di-cyclopentadienyl-Ti-bis-2,4-di-fluorophen-1-yl, di-methylcyclopentadienyl-Ti-bis-2,3,4,5,6-pentafluorophen-1-yl, di-methylcyclopentadienyl-Ti-bis-2,3,5,6-tetrafluorophen-1-yl, di-methylcyclopentadienyl-Ti-bis-2,6-difluorophen-1-yl, and di-cyclopentadienyl-Ti-bis-2,6-difluoro-3-(pyrr-1-yl)-phen-1-yl.

6-2-2. Acylphosphine oxide compound

Specific examples of the acylphosphine oxide compound include monofunctional initiators having only one

photo-cleavage point per molecule and bifunctional initiators having two photo-cleavage points per molecule.

Examples of the monofunctional initiator include triphenylphosphine oxide, diphenyl(2,4,6-trimethylbenzoyl)phosphine oxide, and 2,6-dichlorobenzoyldiphenylphosphine oxide.

Examples of the bifunctional initiator include bis(2,4,6-trimethylbenzoyl)-phenylphosphine oxide, bis(2,6-dimethoxybenzoyl)-2,4,4-trimethylpentylphosphine oxide, bis(2,6dichlorobenzoyl)-4-propylphenylphosphine oxide, and bis(2,6dichlorobenzoyl)-2,5dimethylphenylphosphine oxide.

6-2-3. Oxime ester-based compound

Specific examples of the oxime ester-based compound include 1-[4-(phenylthio)-2-(O-benzoyloxime)]-1,2-octanedione, 1-[9-ethyl-6-(2-methylbenzoyl)-9H-carbazol-3-yl]-1-(O-acetyloxime)ethanone, 4-(acetoxyimino)-5-[9-ethyl-6-(2-methylbenzoyl)-9H-carbazol-3-yl]-5-oxopentanoic acid methyl ester, 1-(9-ethyl-6-cyclohexanoyl-9H-carbazol-3-yl)-1-(O-acetyloxime)glutaric acid methyl ester, 1-(9-ethyl-9H-carbazol-3-yl)-1-(O-acetyloxime)glutaric acid methyl ester, and 1-(9-ethyl-9H-carbazol-3-yl)-1-(O-acetyloxime)-3-methylbutanoic acid.

6-2-4. Amount of photopolymerization initiator used

Any one of the above photopolymerization initiators may be used alone, or any combination of two or more of them may be used at any ratio.

As for the content of the photopolymerization initiator in the polymerizable composition of the invention, the molar amount of the photopolymerization initiator per unit weight of the polymerizable composition is preferably 0.5 μmol/g or more. The content is more preferably 1 μmol/g or more. As for the content of the photopolymerization initiator in the polymerizable composition of the invention, the molar amount per unit weight of the polymerizable composition is preferably 100 μmol/g or less. The content is more preferably 50 μmol/g or less.

If the content of the photopolymerization initiator is excessively small, the amount of radicals generated is small. In this case, the rate of photopolymerization decreases, and the recording sensitivity of a holographic recording medium may decrease. If the content of the photopolymerization initiator is excessively large, radicals generated by irradiation with light recombine with each other or disproportionate. In this case, the contribution of the radicals to photopolymerization is reduced, and again the recording sensitivity of a holographic recording medium may decrease. When two or more photopolymerization initiators are used in combination, it is preferable that the total amount of the photopolymerization initiators is set so as to fall within the above range.

6-3. Radical scavenger

In holographic recording, to fix the intensity pattern of interference light accurately as a polymer distribution in a holographic recording medium, a radical scavenger may be added. Preferably, the radical scavenger has both a functional group that scavenges radicals and a reactive group to be fixed to the matrix resin through a covalent bond. Examples of the functional group that scavenges radicals include a stable nitroxyl radical group.

6-3-1. Type of radical scavenger

Examples of the reactive group to be fixed to the matrix resin through a covalent bond include a hydroxy group, an amino group, an isocyanate group, and a thiol group. Examples of such a radical scavenger include 4-hydroxy-2,2,6,6-tetramethylpiperidine-1-oxyl free radicals (TEMPOL), 3-hydroxy-9-azabicyclo[3.3.1]nonane N-oxyl, and 5-HO-AZADO: 5-hydroxy-2-azatricyclo[3.3.1.1$^{3.7}$]decane N-oxyl.

6-3-2. Content of radical scavenger

Any one of the above various radical scavengers may be used alone, or any combination of two or more of them may be used at any ratio.

As for the content of the radical scavenger in the polymerizable composition of the invention, the molar amount of the radical scavenger per unit weight of the polymerizable composition is preferably 0.5 μmol/g or more and more preferably 1 μmol/g or more. The content of the radical scavenger in the polymerizable composition of the invention is preferably 100 μmol/g or less and more preferably 50 μmol/g or less.

If the content of the radical scavenger is excessively small, the efficiency of scavenging radicals is low, and a polymer with a low degree of polymerization diffuses, so that the amount of components not contributing to signals tends to increase. If the content of the radical scavenger is excessively large, the efficiency of the polymerization of the polymer decreases, and signals tend not to be recorded. When two or more radical scavengers are used in combination, it is

preferable that the total amount of the radical scavengers is set so as to fall within the above range.

6-4. Additional components

The polymerizable composition of the invention may contain additional components in addition to the above components unless the invention departs from the scope thereof.

Examples of the additional components include components for preparing the polymerizable composition such as a solvent, a plasticizer, a dispersant, a leveling agent, an antifoaming agent, and an adhesion promoter. Examples of the additional components when the polymerizable composition is used for a holographic recording medium include components for controlling a recording reaction such as a chain transfer agent, a polymerization terminator, a compatibilizer, a reaction aid, and a sensitizer. Examples of other additives necessary for improving properties include a preservative, a stabilizer, an antioxidant, an ultraviolet absorber and a light stabilizer. Any one of these components may be used alone, or any combination of two or more of them may be used at any ratio.

<Sensitizer>

A compound that controls excitation of the photopolymerization initiator may be added to the polymerizable composition of the invention. Examples of such a compound include a sensitizer and a sensitization aid.

The sensitizer used may be selected from various known sensitizers. Generally, a colored compound such as a coloring agent is often used as the sensitizer in order to absorb visible and ultraviolet laser beams. When the polymerizable composition is used for a holographic recording medium, a suitable sensitizer is selected according to the wavelength of a laser beam used for recording and the type of initiator used. Specific preferred examples of the sensitizer used for a system using a green laser include compounds described in JPH5-241338A, JPH2-69A, JPH2-55446B, etc. Examples of the sensitizer used for a system using a blue laser include compounds described in JP2000-10277A, JP2004-198446A, etc. Any one of these sensitizers may be used alone, or any combination of two or more of them may be used at any ratio.

When a holographic recording medium to be obtained is required to be colorless and transparent, it is preferable to use a cyanine-based coloring agent as the sensitizer. Generally, a cyanine-based coloring agent is easily decomposed by light. Therefore, when the holographic recording medium is subjected to postexposure, i.e., left to stand under exposure to indoor light or sunlight for several hours to several days, the cyanine-based coloring agent in the holographic recording medium is decomposed, and the holographic recording medium does not exhibit absorption in the visible range. The thus-obtained holographic recording medium is colorless and transparent.

It is necessary to increase or decrease the amount of the sensitizer according to the thickness of a recording layer to be formed, and the ratio of the amount of the sensitizer to the amount of the photopolymerization initiator described above in 6-2 is generally 0.01% mass or more and preferably 0.1% mass or more and is generally 10% by mass or less and preferably 5% by mass or less. If the amount of the sensitizer used is excessively small, the initiation efficiency is low, and recording may take a very large amount of time. If the amount of the sensitizer used is excessively large, absorption of light used for recording and reproduction increases, and the light may not easily penetrate deep into the recording layer. When two or more sensitizers are used in combination, the total amount of the sensitizers is set so as to fall within the above range.

<Plasticizer>

To improve the reaction efficiency and adjust the physical properties of the recording layer of a holographic recording medium, the polymerizable composition of the invention may contain a plasticizer.

Examples of the plasticizer include: phthalates such as dioctyl phthalate, diisononyl phthalate, diisodecyl phthalate, and diundecyl phthalate; adipates such as bis(2-ethylhexyl) adipate, diisononyl adipate, and di-n-butyl adipate; sebacates such as dioctyl sebacate and dibutyl sebacate; phosphates such as tricresyl phosphate; citrates such as acetyl tributyl citrate; trimellitates such as trioctyl trimellitate; epoxidized soybean oil; chlorinated paraffin; alkoxylated (poly)alkylene glycol esters such as acetoxymethoxypropane; and alkoxy-terminated polyalkylene glycols such as dimethoxypolyethylene glycol.

A plasticizer containing elemental fluorine and exemplified in Japanese Patent No. 6069294 may also be used. Examples of the plasticizer containing elemental fluorine include 2,2,2-trifluoroethyl butylcarbamate, bis(2,2,2-trifluoroethyl)-(2,2,4-trimethylhexane-1,6-diyl)biscarbamate, bis(2,2,2-trifluoroethyl)-[4-({[(2,2,2-trifluoroethoxy)carbonyl]amino}-methyl)octane-1,8-diyl]biscarbamate, 2,2,3,3,4,4,5,5,6,6,7,7,8,8,9,9-hexadecafluorononyl butylcarbamate, and 2,2,2-trifluoroethyl phenylcarbamate.

The ratio of the amount of the plasticizer to the total solid content of the polymerizable composition is generally from 0.01% mass to 50% by mass inclusive and preferably from 0.05% mass or to 20% by mass inclusive. If the content

of the plasticizer is below the above range, the effects of improving the reaction efficiency and adjusting the physical properties are not obtained. If the content is above the above range, the transparency of the recording layer deteriorates, and bleeding of the plasticizer becomes significant.

<Leveling agent>

A leveling agent may be used for the polymerizable composition of the invention. Examples of the leveling agent include sodium polycarboxylates, ammonium polycarboxylates, amine polycarboxylates, silicon-based leveling agents, acrylic-based leveling agents, ester compounds, ketone compounds, and fluorine compounds. Any one of them may be used alone, or any combination of two or more of them may be used at any ratio.

<Chain transfer agent>

A chain transfer agent may be used for the polymerizable composition of the invention. Examples of the chain transfer agent include: phosphinates such as sodium phosphite and sodium hypophosphite; mercaptans such as mercaptoacetic acid, mercaptopropionic acid, 2-propanethiol, 2-mercaptoethanol, and thiophenol; aldehydes such as acetaldehyde and propionaldehyde; ketones such as acetone and methyl ethyl ketone; halogenated hydrocarbons such as trichloroethylene and perchloroethylene; terpenes such as terpinolene, α-terpinene, β-terpinene, and γ-terpinene; nonconjugated dienes such as 1,4-cyclohexadiene, 1,4-cycloheptadiene, 1,4-cyclooctadiene, 1, 4-heptadiene, 1,4-hexadiene, 2-methyl-1,4-pentadiene, 3,6-nonanedien-1-ol, and 9,12-octadecadienol; linolenic acids such as linolenic acid, γ-linolenic acid, methyl linoleate, ethyl linoleate, isopropyl linoleate, and linolenic anhydride; linoleic acids such as linoleic acid, methyl linoleate, ethyl linoleate, isopropyl linoleate, and linoleic anhydride; eicosapentaenoic acids such as eicosapentaenoic acid and ethyl eicosapentaenoate; and docosahexaenoic acids such as docosahexaenoic acid and ethyl docosahexaenoate.

The ratio of the amount of the additives used to the total amount of solids in the polymerizable composition in the present embodiment is generally 0.001% mass or more preferably 0.01% mass or more and is generally 30% by mass or less and preferably 10% by mass or less. When two or more additive are used in combination, the total amount of the additives is set so as to fall within the above range.

6-5. Compositional ratio of components in polymerizable composition

The contents of the components in the polymerizable composition of the invention can be freely set so long as they do not deviate from the scope of the invention. Preferably, the ratios of the components shown below in terms of their molar amounts per unit mass of the polymerizable composition fall within the following ranges.

The content of the polymerizable compounds including the compound of the invention is preferably 5 μmol/g or more, more preferably 10 μmol/g or more, and still more preferably 100 μmol/g or more. The content of the polymerizable compounds is preferably 1000 μmol/g or less, more preferably 500 μmol/g or less, and still more preferably 300 μmol/g or less.

When the content of the polymerizable compounds is equal to or lower than the above lower limit, a holographic recording medium with sufficient diffraction efficiency is obtained. When the content is equal to or lower than the above upper limit, the compatibility with the resin matrix in the recording layer is maintained, and the degree of shrinkage of the recording layer due to recording tends to be small.

When the isocyanate and the polyol are used for the matrix resin in the polymerizable composition of the invention, the total content of the isocyanate and the polyol is generally 0.1% mass or more, preferably 10% mass or more, and still more preferably 35% mass or more and is generally 99.9% by mass or less and preferably 99% by mass or less. When the total content is equal to or more than the lower limit, the recording layer can be formed easily.

In this case, the ratio of the number of functional groups in the polyol that are reactive with the isocyanate to the number of isocyanate groups in the isocyanate is preferably 0.1 or more and more preferably 0.5 or more and is generally 10.0 or less and preferably 2.0 or less. When this ratio is within the above range, the number of unreacted functional groups is small, and the storage stability is improved.

In the polymerizable composition, it is preferable to determine the content of the urethane polymerization catalyst in consideration of the reaction rate of the isocyanate and the polyol, and the content is preferably 5% by mass or less, more preferably 4% by mass or less, and still more preferably 1% by mass or less. The amount of the urethane polymerization catalyst used is preferably 0.003% mass or more.

The total amount of additional components other than the above components may be 30% by mass or less and is preferably 15% by mass or less and more preferably 5% by mass.

6-6. Method for producing polymerizable composition

In the present invention, no particular limitation is imposed on the method for producing the polymerizable

composition containing the polymerizable compound, the matrix resin, and the photopolymerization initiator, and the order of mixing etc. can be appropriately adjusted. When the polymerizable composition contains a component other than the above components, any combination of these components may be mixed in any order.

The polymerizable composition when the isocyanate and the polyol are used for the matrix resin can be obtained, for example, by the following method, but the present invention is not limited thereto.

The polymerizable compound, the photopolymerization initiator, and components other than the isocyanate and the urethane polymerization catalyst are mixed together to prepare a photoreactive composition (solution A). A mixture of the isocyanate and the urethane polymerization catalyst is prepared as solution B.

Alternatively, the polymerizable compound, the photopolymerization initiator, and components other than the isocyanate may be mixed to prepare a photoreactive composition (solution A).

Preferably, each solution is subjected to dewatering and degassing. If the dewatering and degassing are insufficient, air bubbles are generated during the production of a holographic recording medium, and therefore a uniform recording layer may not be obtained. The dewatering and degassing may be performed by heating under reduced pressure so long as the components are not damaged.

Preferably, the polymerizable composition including a mixture of the solution A and solution B is produced immediately before molding of the holographic recording medium. In this case, a mixing technique using a conventional method may be used. When the solution A and the solution B are mixed, degassing may be optionally performed in order to remove residual gas. Preferably, the solution A and the solution B are subjected to a filtration process separately or simultaneously after mixing in order to remove foreign substances and impurities. It is more preferable to filter these solutions separately.

The isocyanate used for the matrix resin may be an isocyanate-functional prepolymer prepared by a reaction of an isocyanate having an excessive amount of isocyanate groups with the polyol. The polyol used for the matrix resin may be an isocyanate reactive prepolymer prepared by a reaction of a polyol containing an excessive amount of isocyanate reactive functional groups with the isocyanate.

6-7. Holographic recording medium of invention

The holographic recording medium of the present invention that uses the polymerizable composition of the invention includes the recording layer and optionally includes a support and additional layers. Generally, the holographic recording medium includes the support, and the recording layer and the additional layers are stacked on the support to form the holographic recording medium. However, when the recording layer and the additional layers have the strength and durability required for the medium, the holographic recording medium may include no support. Examples of the additional layers include a protective layer, a reflecting layer, and an anti-reflection layer (anti-reflection film).

6-7-1. Recording layer

The recording layer of the holographic recording medium of the invention is a layer formed from the polymerizable composition of the invention, and information is recorded in the recording layer. The information is generally recorded as a hologram. As described later in detail in a recording method section, the polymerizable compound (hereinafter referred to as a polymerizable monomer) contained in the recording layer partially undergoes a chemical change such as polymerization during holographic recording etc. Therefore, in the holographic recording medium after recording, part of the polymerizable monomer is consumed and present as a reacted compound such as a polymer.

No particular limitation is imposed on the thickness of the recording layer, and the thickness may be appropriately set in consideration of the recording method etc. The thickness is preferably 1 μm or more and more preferably 10 μm or more and is preferably 1 cm or less and more preferably 3 mm or less. When the thickness of the recording layer is equal to or more than the above lower limit, selectivity for holograms when multiple recording is performed on the holographic recording medium is high, and therefore the degree of multiple recording can tend to be increased. When the thickness of the recording layer is equal to or less than the above upper limit, the recording layer as a whole can be formed uniformly. Therefore, the holograms can have uniform diffraction efficiency, and multiple recording can tend to be performed with a high S/N ratio.

Preferably, the rate of shrinkage of the recording layer due to exposure to light during information recording or reproduction is 0.25% or less, from the viewpoint of recording reproducibility.

6-7-2. Support

No particular limitation is imposed on the details of the support so long as it has the strength and durability required for the holographic recording medium, and any support can be used.

No limitation is imposed on the shape of the support, and the support is generally formed into a flat plate or a film.

No limitation is imposed on the material forming the support, and the material may be transparent or may be opaque.

Examples of the transparent material for the support include: organic materials such as acrylic, polyethylene terephthalate, polyethylene naphthoate, polycarbonate, polyethylene, polypropylene, amorphous polyolefin, polystyrene, polycycloolefin, and cellulose acetate; and inorganic materials such as glass, silicon, and quartz. Of these, polycarbonate, acrylic, polyester, amorphous polyolefin, glass, etc. are preferred, and polycarbonate, acrylic, amorphous polyolefin, polycycloolefin, and glass are more preferred.
Examples of the opaque material for the support include: metals such as aluminum; and a coating prepared by coating the transparent support with a metal such as gold, silver, or aluminum or a dielectric such as magnesium fluoride or zirconium oxide.
No particular limitation is imposed on the thickness of the support. Preferably, the thickness is in the range of 0.05 mm or more and 1 mm or less. When the thickness of the support is equal to or more than the above lower limit, the mechanical strength of the holographic recording medium can be ensured, and warpage of the substrate can be prevented. When the thickness of the support is equal to or less than the above upper limit, advantages such as an increase in the transmission amount of light, a reduction in the weight of the holographic recording medium, and a rection in cost can be obtained.
The surface of the support may be subjected to surface treatment. The surface treatment is generally performed in order to improve the adhesion between the support and the recording layer. Examples of the surface treatment include corona discharge treatment performed on the support and the formation of an undercoat layer on the support in advance. Examples of the composition for the undercoat layer include halogenated phenols, partially hydrolyzed vinyl chloride-vinyl acetate copolymers, and polyurethane resins.
The surface treatment on the support may be performed for a purpose other than the improvement in adhesion. Examples of such surface treatment include: reflecting coating treatment in which a reflecting coating layer is formed using a metal material such as gold, silver, or aluminum; and dielectric coating treatment in which a dielectric layer formed of magnesium fluoride, zirconium oxide, etc. is formed. Such a layer may be formed as a single layer, or two or more layers may be formed.
The surface treatment may be performed for the purpose of controlling the gas and water permeability of the substrate. For example, when the support supporting the recording layer has the function of preventing permeation of gas and water, the reliability of the holographic recording medium can be improved.
The support may be disposed only on one of the upper and lower sides of the recording layer of the holographic recording medium of the present invention or may be disposed on both sides. When supports are disposed on both the upper and lower sides of the recording layer, at least one of the supports is made transparent so that it can transmit active energy rays (such as excitation light, reference light, and reproduction light).
When the holographic recording medium has the support on one side or both sides of the recording layer, a transmission hologram or a reflection hologram can be recorded. When a support having anti-reflection characteristics is used on one side of the recording layer, a reflection hologram can be recorded.
A pattern for data addressing may be provided on the support. In this case, no limitation is imposed on the patterning method. For example, irregularities may be formed on the support itself, or the pattern may be formed on the reflecting layer described later. The pattern may be formed using a combination of these methods.

6-7-3. Protective layer

The protective layer is a layer for preventing deterioration of the recording-reproduction propertied of the recording layer. No limitation is imposed on the specific structure of the protective layer, and any known protective layer can be used. For example, a layer formed of a water-soluble polymer, an organic or inorganic material, etc. can be formed as the protective layer.
No particular limitation is imposed on the formation position of the protective layer. The protective layer may be formed, for example, on the surface of the recording layer or between the recording layer and the support or may be formed on the outer surface side of the support. The protective layer may be formed between the support and another layer.

6-7-4. Reflecting layer

The reflecting layer is formed when the holographic recording medium formed is of the reflection type. In the reflection holographic recording medium, the reflecting layer may be formed between the support and the recording layer or may be formed on the outer side of the support. Generally, it is preferable that the reflecting layer is present between the support and the recording layer.
Any known reflecting layer may be used, and a thin metal film, for example, may be used.

6-7-5. Anti-reflection film

In each of the transmission and reflection holographic recording mediums, an anti-reflection film may be disposed on the side on/from which information light, reference light, and reproduction light are incident/emitted or between the recording layer and the support. The anti-reflection film improves the efficiency of utilization of light and prevents the occurrence of noise.
Any known anti-reflection film may be used.

6-7-6. Method for producing holographic recording medium

No limitation is imposed on the method for producing the holographic recording medium of the present invention. For example, the holographic recording medium can be produced by coating the support with the polymerizable composition of the invention without using a solvent to form the recording layer. Any known coating method can be used. Specific examples of the coating method include a spray method, a spin coating method, a wire bar method, a dipping method, an air knife coating method, a roll coating method, a blade coating method, and a doctor roll coating method.
When a recording layer with a large thickness is formed, a method in which the polymerizable composition is molded using a die, a method in which the polymerizable composition is applied to a release film and punched with a die, etc. may be used to form the recording layer. The holographic recording medium of the invention may be produced by mixing the polymerizable composition of the present invention with a solvent or an additive to prepare a coating solution, coating the support with the coating solution, and then drying the coating solution to form the recording layer. In this case also, any coating method can be used. For example, any of the above described methods can be used.
No limitation is imposed on the solvent used for the coating solution. It is generally preferable to use a solvent that can dissolve the component used sufficiently, provides good coating properties, and does not damage the support such as a resin substrate. One solvent may be used alone, or any combination of two or more solvents may be used at any ratio. No limitation is imposed on the amount of the solvent used. However, from the viewpoint of coating efficiency and handleability, it is preferable to prepare a coating solution having a solid concentration of about 1 to about 100% by mass.
Examples of the solvent include: ketone-based solvents such as acetone, methyl ethyl ketone, methyl isobutyl ketone, cyclohexanone, and methyl amyl ketone; aromatic-based solvents such as toluene and xylene; alcohol-based solvents such as methanol, ethanol, propanol, n-butanol, heptanol, hexanol, diacetone alcohol, and furfuryl alcohol; ketone alcohol-based solvents such as diacetone alcohol and 3-hydroxy-3-methyl-2-butanone; ether-based solvents such as tetrahydrofuran and dioxane; halogen-based solvents such as dichloromethane, dichloroethane, and chloroform; cellosolve-based solvents such as methyl cellosolve, ethyl cellosolve, butyl cellosolve, methyl cellosolve acetate, and ethyl cellosolve acetate; propylene glycol-based solvents such as propylene glycol monomethyl ether, propylene glycol monoethyl ether, propylene glycol monobutyl ether, propylene glycol monomethyl ether acetate, propylene glycol monoethyl ether acetate, propylene glycol monobutyl ether acetate, and dipropylene glycol dimethyl ether; ester-based solvents such as ethyl acetate, butyl acetate, amyl acetate, butyl acetate, ethylene glycol diacetate, diethyl oxalate, ethyl pyruvate, ethyl-2-hydroxybutyrate ethylacetoacetate, methyl lactate, ethyl lactate, methyl 2-hydroxyisobutyrate, and methyl 3-methoxypropionate; perfluoroalkyl alcohol-based solvents such as tetrafluoropropanol, octafluoropentanol, and hexafluorobutanol; highly polar solvents such as dimethylformamide, dimethylacetamide, N-methylpyrrolidone, and dimethyl sulfoxide; chain hydrocarbon-based solvents such as n-hexane and n-octane; cyclic hydrocarbon-based solvents such as cyclohexane, methylcyclohexane, ethylcyclohexane, dimethylcyclohexane, n-butylcyclohexane, tert-butylcyclohexane, and cyclooctane; and mixtures of these solvents.
Examples of the holographic recording medium production method include: a production method in which the recording layer is formed by coating the support with the polymerizable composition fused by heat and cooling the polymerizable composition to solidify the composition; a production method in which the recording layer is formed by coating the support with the polymerizable composition in liquid form and subjecting the polymerizable composition to thermal polymerization to cure the composition; and a production method in which the recording layer is formed by coating the support with the polymerizable composition in liquid form and subjecting the polymerizable composition to photopolymerization to cure the composition.
The thus-produced holographic recording medium can be in the form of a self-supporting slab or disk and can be used for three-dimensional image display devices, diffraction optical elements, large-capacity memories, etc.
In particular, the holographic recording medium of the present invention that uses the polymerizable composition of the invention has a high refractive index modulation and is useful also for light guide plates for AR glasses.

6-7-7. Applications of holographic recording medium

<Large-capacity memory applications>

Information is written (recorded)/read (reproduced) in/from the holographic recording medium of the present invention by irradiating the medium with light.

To record information, light capable of causing a chemical change of the polymerizable monomer, i.e., its polymerization and a change in concentration, is used as the object light (referred to also as recording light).

For example, when information is recorded as a volume hologram, the object light together with the reference light is applied to the recording layer to allow the object light to interfere with the reference light in the recording layer. In this case, the interfering light causes polymerization of the polymerizable monomer and a change in its concentration within the recording layer. Therefore, the interference fringes cause refractive index differences within the recording layer, and the information is recorded as a hologram through the interference fringes recorded in the recording layer.

To reproduce the volume hologram recorded in the recording layer, prescribed reproduction light (generally the reference light) is applied to the recording layer. The applied reproduction light is diffracted by the interference fringes. Since the diffracted light contains the same information as that in the recording layer, the information recorded in the recording layer can be reproduced by reading the diffracted light using appropriate detection means.

The wavelength ranges of the object light, the reproduction light, and the reference light can be freely set according to their applications and may be the visible range or the ultraviolet range. Preferred examples of such light include light from lasers with good monochromaticity and directivity such as: solid lasers such as ruby, glass, Nd-YAG, and Nd-$YVO_4$ lasers; diode lasers such as GaAs, InGaAs, and GaN lasers; gas lasers such as helium-neon, argon, krypton, excimer, and $CO_2$ lasers; and dye lasers including dyes.

No limitation is imposed on the amounts of irradiation with the object light, the reproduction light, and the reference light, and these amounts can be freely set so long as recording and reproduction are possible. If the amounts of irradiation are extremely small, the chemical change of the polymerizable monomer is too incomplete, and the heat resistance and mechanical properties of the recording layer may not be fully obtained. If the amounts of irradiation are extremely large, the components of the recording layer (the components of the polymerizable composition of the invention) may deteriorate. Therefore, the object light, the reproduction light, and the reference light are applied generally at 0.1 $J/cm^2$ or more and 20 $J/cm^2$ or less according to the chemical composition of the polymerizable composition of the invention used to form the recording layer, the type of photopolymerization initiator, the amount of the photopolymerization initiator mixed, etc.

Examples of the holographic recording method include a polarized collinear holographic recording method and a reference light incidence angle multiplexing holographic recording method. When the holographic recording medium of the present invention is used as a recording medium, good recording quality can be provided using any of the recording methods.

<AR glass light guide plate applications>

Volume holograms are recorded in the holographic recording medium of the present invention in the same manner as in the large-capacity memory applications.

The volume holograms recorded in the recording layer are irradiated with prescribed reproduction light through the recording layer. The applied reproduction light is diffracted by the interference fringes. In this case, even when the wavelength of the reproduction light does not coincide with the wavelength of the recording light, diffraction occurs when the Bragg condition for the interference fringes is satisfied. Therefore, by recording interference fringes corresponding to the wavelengths and incident angles of reproduction light beams to be diffracted, the reproduction light beams in a wide wavelength range can be diffracted, and the color display range of AR glasses can be increased.

By recording interference fringes corresponding to the wavelength and diffraction angle of reproduction light, the reproduction light entering from the outside of the holographic recording medium can be guided to the inside of the holographic recording medium, and the reproduction light guided inside the holographic recording medium can be reflected, split, and expanded or reduced in size. Moreover, the reproduction light guided through the inside of the holographic recording medium can be emitted to the outside of the holographic recording medium. This allows the viewing angle of AR glasses to be increased.

The wavelength ranges of the object light and the reproduction light can be freely set according to their applications, and the object light and the reproduction light may be in the visible range or in the ultraviolet range. Preferred examples of the object light and the reproduction light include light from the above-described lasers. The reproduction light is not limited to light from a laser etc., and display devices such as liquid crystal displays (LCDs), organic electroluminescent displays (OLEDs), etc. can also be used preferably.

No limitation is imposed on the amounts of irradiation with the object light, the reproduction light, and the

reference light, and these amounts can be freely set so long as recording and reproduction are possible. If the amounts of irradiation are extremely small, the chemical change of the polymerizable monomer is too incomplete, and the heat resistance and mechanical properties of the recording layer may not be fully obtained. If the amounts of irradiation are extremely large, the components of the recording layer (the components of the polymerizable composition of the invention) may deteriorate. Therefore, the object light, the reproduction light, and the reference light are applied generally at 0.1 $J/cm^2$ or more and 20 $J/cm^2$ or less according to the chemical composition of the polymerizable composition of the invention used to form the recording layer, the type of photopolymerization initiator, the amount of the photopolymerization initiator mixed, etc.

6-8. Performance indicator of holographic recording medium

The total $\Delta n$ calculated using the sum of diffraction efficiency over the entire multiple recording is used as the indicator of the performance of the holographic recording medium. For a transmission hologram, the diffraction efficiency of the hologram is given as the ratio of the intensity of diffracted light to the sum of the intensity of transmitted light and the intensity of the diffracted light. $\Delta n$ is computed from the following formula in Coupled Wave Theory (H. Kogelnik, The Bell System Technical Journal (1969), 48, 2909-2947) using the obtained diffraction efficiency, and the total over the entire multiple recording is used as the total $\Delta n$.

[Math. 1]

$$\eta = \sin^2\left(\frac{\pi \cdot T \cdot \Delta n}{\lambda \cdot \cos\theta}\right)$$

$$total\Delta n = \sum \Delta n$$

Here, $\eta$ is the diffraction efficiency, and T is the thickness of the medium. $\lambda$ is the wavelength of the reference light, and $\theta$ is the incident angle of the reference light.

In large-capacity memories, a large total $\Delta n$ is preferred because this means that a larger amount of information can be recorded per unit volume. In AR glass applications, a large total $\Delta n$ is preferred because this means that an image projected from a projector can be delivered to the eyes without loss of brightness, because power consumption can be reduced, and because the viewing angle can be increased.

[EXAMPLES]

The present invention will be described in more detail by way of Examples. However, the present invention is not limited to the Examples so long as they do not depart from the scope of the invention.

Methods for synthesizing compounds will be described in detail using chemical formulas including the synthesis processes of the compounds.

[Materials used]

Raw materials of compositions used in the Examples and Comparative Examples are as follows.

<Isocyanate>

- DURANATE (registered trademark) TSS-100: hexamethylene diisocyanate-based polyisocyanate (NCO: 17.6%) (manufactured by Asahi Kasei Corporation)

<Polyol>

- PLACCEL PCL-205U: polycaprolactone diol (molecular weight: 530) (manufactured by Daicel Corporation)

- PLACCEL PCL-305: polycaprolactone triol (molecular weight: 550) (manufactured by Daicel Corporation)

<Photopolymerization initiator>

- HLI02: 1-(9-ethyl-6-cyclohexanoyl-9H-carbazol-3-yl)-1-(O-acetyloxime)glutaric acid methyl ester

<Radical scavenger>

- TEMPOL: 4-hydroxy-2,2,6,6-tetramethylpiperidine-1-oxyl free radicals (manufactured by TOKYO CHEMICAL INDUSTRY Co., Ltd.)

<Light stabilizer>

- ADEKA STAB LA-63P (manufactured by ADEKA Co., Ltd.)

<Urethane polymerization catalyst>

- Octylic acid solution of bismuth tris(2-ethylhexanoate) (amount of effective component: 56% by weight)

(Synthesis Example 1)

A method described in JP2017-14213A was used. Specifically, the following synthesis method was used to produce bis(4-dibenzothiophenyl)disulfide as a compound S-1 and then produce 4-dibenzothiophenethiol as a compound S-2.

[Chem. 21]

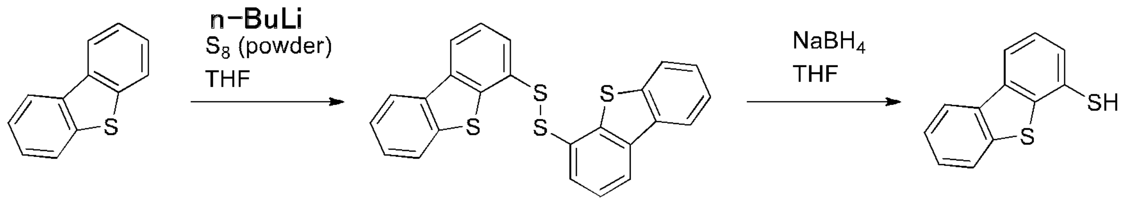


Compound S-1 Compound S-2

20 g of dibenzothiophene was dissolved in 300 mL of THF. While the mixture was cooled to 0°C, 74.6 mL of a n-butyllithium hexane solution with a concentration of 1.6 M was added, and the resulting mixture was heated to 20°C and stirred for 2 hours. The obtained brown reaction solution was cooled to -40°C, and 3.8 g of sulfur (a powder form, product of Wako Pure Chemical Industries, Ltd.) was added. The resulting reaction solution was stirred at -40°C for 30 minutes, and 5 mL of water was added to the reaction mixture to stop the reaction.
The obtained solution was concentrated using an evaporator, and the obtained solid was washed with 100 mL of toluene for 39 minutes. The yellow solid was separated by filtration to thereby produce the compound S-1.
The compound S-1 was dispersed in 200 mL of THF. Then 4.5 g of sodium borohydride was added, and the mixture was stirred at 50°C for 1 hour. Then the reaction solution was filtrated. The resulting solution was concentrated using an evaporator, and 200 mL of toluene was added. The toluene solution was washed with water, 1N hydrochloric acid, a 1N aqueous sodium hydroxide solution, and the resulting solution was concentrated. The concentrate was subjected to recrystallization with hexane, and 9.4 g of the compound S-2 was obtained (yield: 40%).
Several milliliters of deuterochloroform was added to an appropriate amount (about 10 mg) of the compound

S-2 obtained to dissolve it. If insoluble matter was found, the mixture was filtered through a cotton plug. The filtrate was transferred to a special-purpose sample tube, and the sample tube was covered with a lid. This sample was used for measurement of resonance states of hydrogen using a 400 MHz nuclear magnetic resonance (NMR) apparatus. The attribution of each of the resonance lines to hydrogen in the compound was determined to confirm that the target compound was obtained. The measurement data is shown below. For each of the following target compounds, the same measurement was performed to confirm the target compound was obtained. The measurement data is shown below.
$^1$H NMR (400 MHz, $CDCl_3$, δ, ppm) 3.63 (s, 1H), 7.35 (Ar, 1H), 7.45 (Ar, 3H), 7.89 (Ar, 1H), 8.05 (Ar, 1H), 8.14 (Ar, 1H)

(Example 1)

A compound M-1 was produced by the following synthesis method.

[Chem. 22]

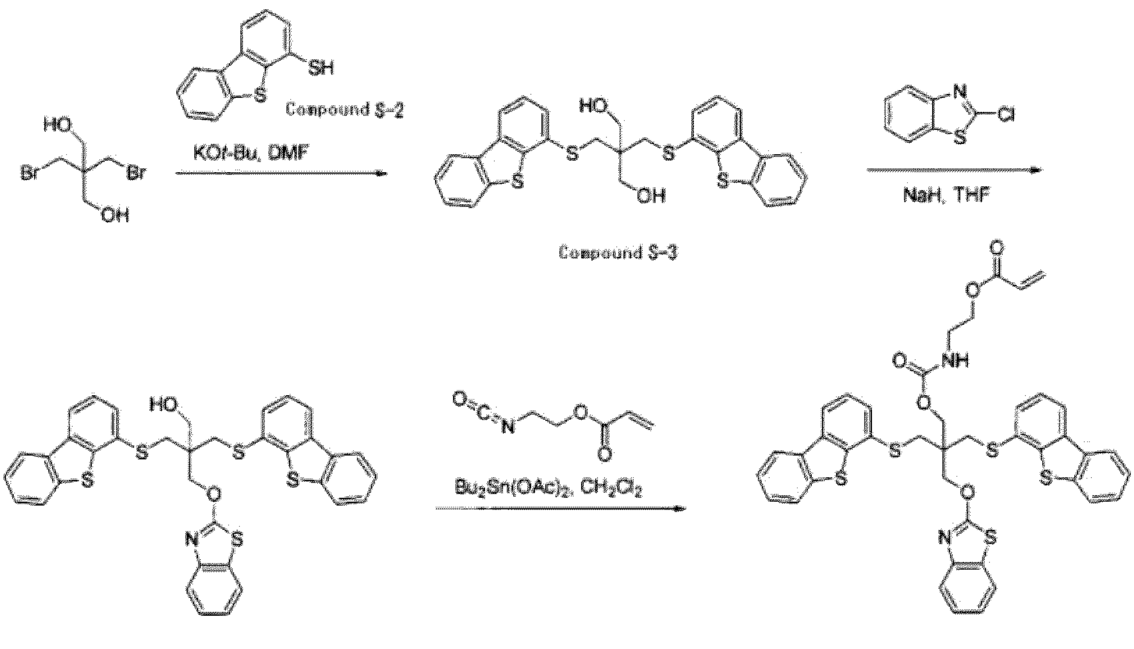


Compound S-3

Compound S-4

Compound M-1

The compound S-2 (20 g), 2,2-bis(bromomethyl)-1,3-propanediol (12.11 g), and DMF (150 mL) were mixed in a nitrogen atmosphere. A DMF (50 mL) solution of potassium tert-butoxide (10.37 g) was added dropwise to the mixture at 75°C, and the resulting mixture was continuously stirred for 30 minutes.

After completion of the reaction, the reaction solution was poured into 1 L of water, and the mixture was extracted twice with 500 mL of ethyl acetate, washed with 1 L of saturated brine, dried over sodium sulfate, filtered, and concentrated. The obtained crude product was purified by silica gel column chromatography to thereby obtain 9.7 g of a compound S-3 (yield: 97%).

The NMR measurement data of the compound S-3 is as follows.
$^1$H-NMR (400 MHz, $CDCl_3$) δ8.31 - 8.29 (m, 2H), 8.15 (d, J=9.5Hz, 2H), 7.99 - 7.97 (m, 2H), 7.53 - 7.50 (m, 6H), 7.39 (t, J=3.5Hz, 2H), 4.78 (t, J=4.8Hz, 2H), 3.51 (d, J=4.8Hz, 4H), 3.27 (s, 4H)

The compound S-3 (3.0 g), sodium tert-butoxide (0.80 g), and THF (30 mL) were mixed in a nitrogen atmosphere. 2-Chloro-1,3-benzothiazole (1.34 g) was added dropwise to the mixture at 30°C over 1 hour, and the resulting mixture was continuously stirred for 1 hour.

After completion of the reaction, the reaction solution was poured into 50 mL of water, and the resulting mixture was extracted with 200 mL of ethyl acetate, washed with 100 mL of saturated brine, dried over anhydrous magnesium sulfate, filtered, and concentrated. The obtained crude product was purified by silica gel column chromatography to thereby obtain 1.9 g of a compound S-4 (yield: 51%).

The NMR measurement data of the compound S-4 is as follows.
$^1$H NMR (400 MHz, $CDCl_3$ δ, ppm) 3.14 (t, OH, 1H), 3.38 (d, 2H), 3.40 (d, 2H), 3.71 (d, 2H), 4.63 (s, 2H), 7.20 (Ar, 1H), 7.29 (Ar, 3H), 7.45 (Ar, 5H), 7.53 (Ar, 3H), 7.82 (Ar, 4H), 8.00 (Ar, 2H)

The compound S-4 (1.9 g) was dissolved in 15 mL of dichloromethane, and 19 mg of dibutyltin diacetate was added. 0.65 g of 2-isocyanatoethyl acrylate was added to the mixture, and the resulting mixture was allowed to react at room temperature for about 50 hours. After completion of the reaction, 20 mL of dichloromethane was added, and the resulting mixture was caused to pass through a silica gel short-path column. Then the obtained solution was concentrated to a total weight of 10 g at 30°C or lower. The resulting solution was added dropwise to 100 mL of ice-cooled methanol, and the mixture was stirred in this state for 1 hour. The precipitate was separated by filtration, washed with methanol, and dried to thereby obtain 1.6 g of a compound M-1 (yield: 71%).

The NMR measurement data of the compound M-1 is as follows.

$^1$H NMR (400 MHz, $CDCl_3$, $\delta$, ppm) 3.29 (m, 2H), 3.40 (s, 4H), 4.09 (m, 2H), 4.29 (s, 2H), 4.53 (s, 2H), 4.64 (brt, NH, 1H), 5.80 (dd, 1H), 6.06 (dd, 1H), 6.37 (dd, 1H), 7.17 (Ar, 1H), 7.22 (Ar, 2H), 7.26 (Ar, 7.36 (Ar, 1H), 7.44 (Ar, 5H), 7.52 (Ar, 2H), 7.79 (Ar, 4H), 7.94 (Ar, 2H)

<Production of holographic recording medium >

0.269 g of the compound M-1 used as the polymerizable monomer, 0.0096 g of a photopolymerization initiator HLI02, 3.30 mg of the radical scavenger TEMPOL, and 2.6 mg of light stabilizer LA-63P were dissolved in 2.53 g of DURANATE (registered trademark) TSS-100 to prepare solution A.

Separately, 1.73 g of PLACCEL PCL-205U and 0.74 g of PLACCEL PCL-305 (PLACCEL PCL-205U:PLACCEL PCL-305 = 70:30 (mass ratio)) were mixed, and 0.2 mg of an octylic acid solution of bismuth tris(2-ethylhexanoate) was dissolved in the mixture to thereby prepare solution B.

The solutions A and B were separately degassed at room temperature or 45°C under reduced pressure for 2 hours. Then 2.39 g of the solution A and 2.11 g of the solution B were mixed under stirring and further degassed in a vacuum for several minutes.

Then the vacuum degassed solution mixture was poured onto a microscope slide with 0.5 mm-thick spacer sheets placed on two opposite edges, and another microscope slide was placed thereon. Clips were used to fix the edges, and heating was performed at 80°C for 24 hours to produce a holographic recording medium as an evaluation sample. In this evaluation sample, a recording layer with a thickness of 0.5 mm was formed between the microscope slides used as covers.

In the holographic recording medium, the ratio of the number of isocyanate groups in the solution A to the number of isocyanate reactive groups in the solution B was 1.0. The holographic recording medium 2 contained 58.3 $\mu$mol/g of the polymerizable monomer, 3.05 $\mu$mol/g of the photopolymerization initiator, and 3.05 $\mu$mol/g of the radical scavenger.

[Holographic recording and evaluation method]

Each of the holographic recording medium produced as the evaluation samples was used to perform holographic recording and evaluate the holographic recording performance of each holographic recording medium using procedures described below.

Holographic recording was performed using a semiconductor laser with a wavelength of 405 nm. An exposure device shown in Fig. 1 was used to perform two-beam plane-wave holographic recording at an exposure power density per beam of 10.2 mW/cm$^2$. The medium was rotated from -22.5° to 22.5°, and angular multiple recording was performed in the same position. The diffraction efficiency for each multiple recording operation was measured. The obtained diffraction efficiency was used to compute $\Delta$n, and the total over the entire multiple recording was used as the total $\Delta$n.

Details will next be described.

(Holographic recording)

Fig. 1 is a structural diagram showing the outline of the device used for holographic recording.

In Fig. 1, S represents a holographic recording medium sample, and M1 to M3 represent mirrors. PBS represents a polarizing beam splitter. L1 represents a recording laser light source emitting light with a wavelength of 405 nm (a single mode laser ("L1" in Fig. 1) manufactured by TOPTICA Photonics and capable of emitting light with a wavelength of about 405 nm). L2 represents a reproduction laser light source emitting light with a wavelength of 633 nm. PD1, PD2, and PD3 represent photodetectors. 1 represents an LED unit.

As shown in Fig. 1, a light beam with a wavelength of 405 nm was split using the polarizing beam splitter ("PBS" in the figure) into two beams intersecting on a recording surface such that the angle therebetween was 59.3°. In this case, the light beam was split such that a bisector of the angle between the two beams was perpendicular to the recording surface, and the two beams obtained by splitting the light beam were applied such that the vibration planes of the electric field vectors of the two beams were perpendicular to a plane including the two intersecting beams.

After the holographic recording, a He-Ne laser capable of emitting light with a wavelength of 633 nm (V05-LHP151 manufactured by Melles Griot: "L2" in the figure) was used to apply the light to the holographic recording medium at an angle of 50.7°. The diffracted light was detected using a photo diode and a photosensor amplifier (S2281 and C9329: manufactured by Hamamatsu Photonics Co.,Ltd., "PD1" in the figure) to determine whether the holographic recording was correctly performed.

(Measurement of diffraction efficiency)

Multiple recording was performed while the sample was moved with respect to the optical axes such that the angle of the sample (the angle between the normal to the sample and a bisector of the interior angle of the two beams, i.e., incident light beams from the mirrors M1 and M2 in Fig. 1, at the intersection of the beams) was changed from -22.5° to 22.5°. Specifically, the multiple recording was performed 151 times while the angle of the sample was changed in steps of 0.3°.
After the multiple recording, the LED unit (1 in the figure, center wavelength: 405 nm) was turned on for a given period of time to completely consume the remaining initiator and the remaining monomer. This process is referred to as postexposure. The power of the LED was 100 mW/$cm^2$, and the irradiation was performed such that the integrated energy was 12 J/$cm^2$.
The diffraction efficiency of a hologram is given as the ratio of the intensity of the diffracted light to the sum of the intensity of the diffracted light and the intensity of the transmitted light. Light (wavelength: 405 nm) from the mirror M1 in Fig. 1 was directed to the sample, and the diffraction efficiency was measured at angles of - 23° to 23°. Δn was computed from the following formula in Coupled Wave Theory (H. Kogelnik, The Bell System Technical Journal (1969), 48, 2909-2947) using the obtained diffraction efficiency, and the total over the entire multiple recording was used as the total Δn.

[Math. 2]

$$\eta = \sin^2\left(\frac{\pi \cdot T \cdot \Delta n}{\lambda \cdot \cos\theta}\right)$$

$$total\Delta n = \sum \Delta n$$

Here, η is the diffraction efficiency, and T is the thickness of the medium. λ is the wavelength of the reference light, and θ is the incident angle of the reference light (29.65°).
A plurality of samples prepared were used. The evaluation was performed a plurality of times under different irradiation energy conditions, i.e., while the irradiation energy at the beginning of irradiation was increased or decreased and the total irradiation energy was increased or decreased. A search for conditions under which the polymerizable monomer was almost completely consumed (the total Δn substantially reached equilibrium by the multiple recording) was performed in order to maximize the total Δn. The maximum value obtained was used as the total Δn of the medium.

(Measurement of transmittance before recording and transmittance after recording)

The transmittance before recording of an evaluation sample was determined before recording by measuring the ratio of the power of the transmission light to the power of the incident light.
Moreover, the transmittance after recording of the evaluation sample subjected to postexposure was determined after recording by measuring the ratio of the power of the transmission light to the power of the incident light.

(Measurement of haze after recording)

Each evaluation sample subjected to holographic recording and postexposure was used to measure a haze value with respect to white light using a haze meter NDH 7000SPII manufactured by NIPPON DENSHOKU INDUSTRIES Co., Ltd. (JIS K7136).
The ratio of the haze value to the total Δn described above (haze % / total Δn) was computed.
The lower the ratio, the higher the degree of balance between the diffraction efficiency and the transparency.

(Example 2)

A compound M-2 was produced by the following synthesis method.

[Chem. 23]

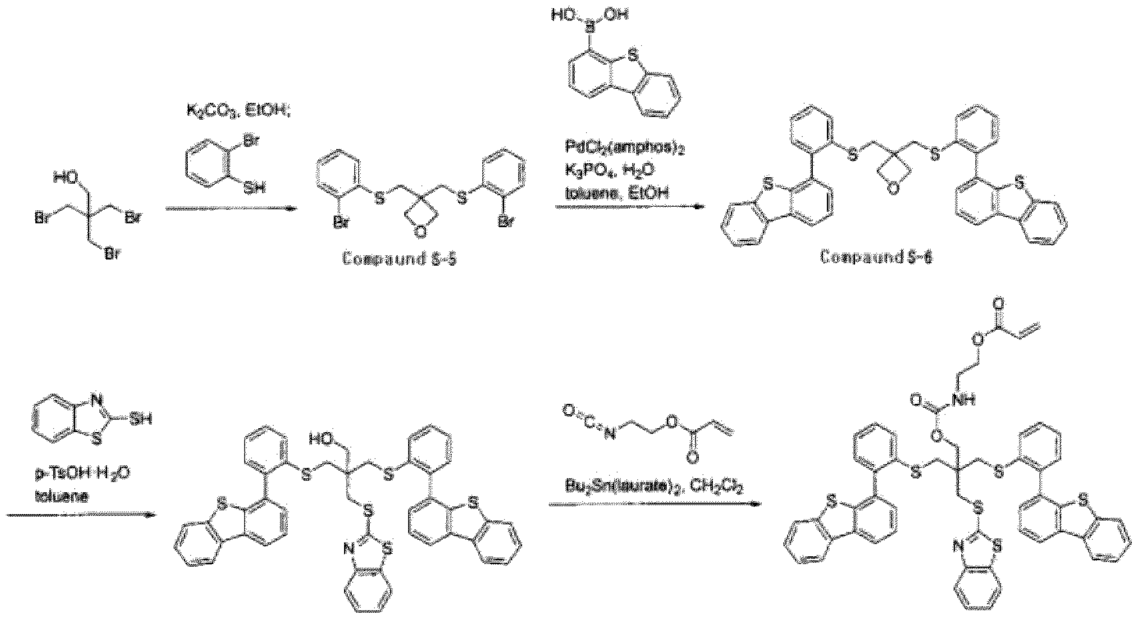


Compound S-5

Compound S-6

Compound S-7

Compound M-2

10 g of pentaerythritoltribromide and 12.8 g of potassium carbonate were suspended in 50 mL of ethanol. The reaction mixture was heated to 100°C and stirred for 4 hours while the progress of the reaction was checked by TLC and HPLC analysis. The mixture was cooled to room temperature, and then 12.2 g of 2-bromobenzenethiol was slowly added. The white suspension was again heated to 100°C and stirred for 1 hour. The suspension was cooled to room temperature, and the white solid was separated by filtration and washed with 100 mL of ethyl acetate. The obtained organic layer was concentrated, and the obtained crude product was purified using a silica gel column (hexane/ethyl acetate) to thereby obtain 12.4 g of a compound S-5 (yield: 88%).

The NMR measurement data of the compound S-5 is as follows.

$^1H$ NMR (400 MHz, $CDCl_3$, $\delta$, ppm) 3.50 (s, 4H), 4.49 (s, 4H), 7.04 (ddd, Ar, 2H), 7.24 (ddd, Ar, 2H), 7.38 (dd, Ar, 2H), 7.53 (dd, Ar, 2H)

The compound S-5 (3 g), dibenzothiophene-4-boronic acid (4.5 g), and potassium phosphate (5.5 g) were suspended in 20 mL of toluene, 10 mL of ethanol, and 10 mL of water, and nitrogen gas was bubbled through the suspension to degas the suspension. 138 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 6 hours. The resulting mixture was cooled to room temperature, extracted with ethyl acetate, and washed with water. The aqueous layer was extracted twice with ethyl acetate. 0.4 g of activated carbon was added to the obtained organic layer, and the mixture was stirred for 30 minutes. The mixture was filtered through celite and concentrated, and the crude product obtained was purified using a silica gel column (hexane/ethyl acetate) to thereby obtain 4.4 g of a compound S-6.

The NMR measurement data of the compound S-6 is as follows.

$^1H$ NMR (400 MHz, $CDCl_3$, $\delta$, ppm) 3.01 (s, 4H), 4.03 (s, 4H), 7.17 (Ar, 2H) 7.27 (Ar, 4H), 7.35 (Ar, 4H), 7.45 (Ar, 6H), 7.76 (Ar, 2H), 8.16 (Ar, 4H)

The compound S-6 (4 g), 2-mercaptobenzothiazole (1.2 g), and p-toluenesulfonic acid monohydrate (30 mg) were suspended in 50 mL of toluene. The reaction solution was heated to 120°C in a nitrogen atmosphere and stirred at reflux for 1 hour. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 4.1 g of a compound S-7 (yield: 82%).

The NMR measurement data of the compound S-7 is as follows.
$^1H$ NMR (400 MHz, $CDCl_3$, $\delta$, ppm) 2.69 (brs, 2H), 2.93 (d, 2H), 3.14 (brs, 4H), 5.04 (s, OH, 1H), 7.27 (Ar, 7H), 7.34 (Ar, 3H), 7.44 (Ar, 8H), 7.65 (Ar, 2H), 7.74 (Ar, 2H), 8.14 (Ar, 4H)

The compound S-7 (5 g) was dissolved in 15 mL of dichloromethane, and 100 mg of dibutyltin dilaurate was added. 0.85 g of 2-isocyanatoethyl acrylate (Karenz AOI manufactured by Showa Denko Co.,Ltd.) was added to the prepared solution, and the mixture was allowed to react at room temperature. After a lapse of 24 hours, an additional 0.3 g of 2-isocyanatoethyl acrylate was added, and the mixture was allowed to further react for 24 hours. A saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 3.6 g of a compound M-2 (yield: 62%).

The NMR measurement data of the compound M-2 is as follows.
$^1H$ NMR (400 MHz, $CDCl_3$, $\delta$, ppm) 2.77 (brs, 4H), 3.12 (dd, 2H), 3.25 (brs, 2H), 3.78 (brs, 2H), 4.02 (dd, 2H), 4.42 (brs, NH, 1H), 5.83 (d, 1H), 6.10 (dd, 1H), 6.40 (d, 1H), 5.04 (s, OH, 1H), 7.23 (Ar, 5H), 7.31 (Ar, 5H), 7.37 (Ar, 2H), 7.43 (Ar, 6H), 7.63 (Ar, 2H), 7.73 (Ar, 2H), 8.12 (Ar, 4H)

A holographic recording medium was produced and evaluated in the same manner as in Example 1, except that compound M-2 was used as the polymerizable monomer. The results are shown in Table 1 below.

(Example 3)

A compound M-3 was produced by the following synthesis method.

[Chem. 24]

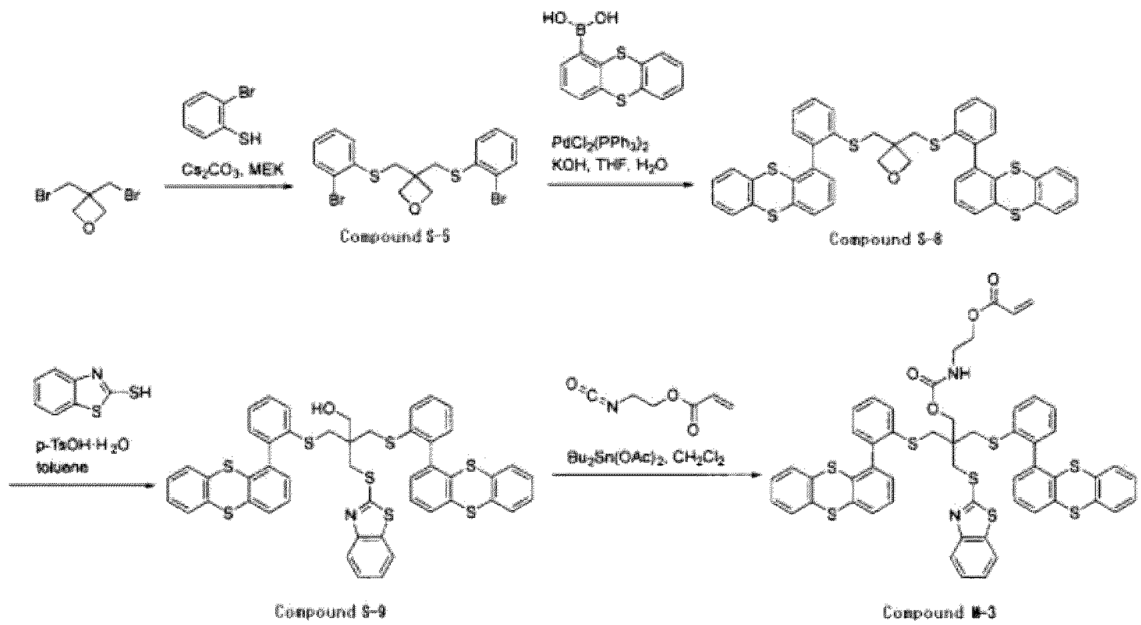


10 g of bis(bromomethyl)oxetane, 16.3 g of 2-bromobenzenethiol, and 28 g of cesium carbonate were suspended in 300 mL of methyl ethyl ketone (MEK). The reaction mixture was heated to 90°C and stirred at reflux for 3 hours while the progress of the reaction was checked by LC analysis. Then the mixture was cooled to room temperature and extracted with 600 mL of water and twice with 300 mL of ethyl acetate. The crude product obtained was purified using a silica gel column (hexane/ethyl acetate) to thereby obtain 26.5 g of the compound S-5 (yield: 96%).

The NMR measurement data of the compound S-5 obtained was as described above.

The compound S-5 (9 g), thianthrene-1-boronic acid (11.2 g), 1.37 g of dichlorobis[triphenylphosphino]palladium (II), and potassium hydroxide (11 g) were suspended in 90 mL of tetrahydrofuran and 23 mL of water, and nitrogen gas was bubbled through the suspension to degas the suspension. The reaction solution was heated to 80°C in a nitrogen atmosphere and stirred for 12 hours. The reaction solution was cooled to room temperature. Then 125 mL of ethyl acetate was added, and the mixture was extracted with 250 mL of water. The organic layer was combined with an organic layer in a different lot subjected to the same reaction, and the combined organic layer was concentrated. The crude

product obtained was purified using a silica gel column (heptane/ethyl acetate) to thereby obtain 16 g of a compound S-8 (yield: 66%) .

The NMR measurement data of the compound S-8 is as follows.

$^1$H NMR (400 MHz, $CDCl_3$, $\delta$, ppm) 2.97 (brs, 4H), 3.95 (m, 4H), 7.10 (Ar, 4H) 7.17 (Ar, 4H), 7.23 (Ar, 2H), 7.29 (Ar, 4H), 7.33 (Ar, 2H), 7.36 (Ar, 2H), 7.47 (Ar, 2H), 7.51 (Ar, 2H)

The compound S-8 (9.5 g), 2-mercaptobenzothiazole (2.4 g), and toluenesulfonic acid monohydrate (100 mg) were suspended in 30 mL of toluene. The reaction solution was heated to 120°C in a nitrogen atmosphere and stirred at reflux for 1 hour. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 10.3 g of a compound S-9 (yield: 88%).

The NMR measurement data of the compound S-9 is as follows.

$^1$H NMR (400 MHz, $CDCl_3$, $\delta$, ppm) 2.55 (dd, 2H), 2.68 (dd, 2H), 2.82 (dd, 2H) 3.04 (d, 2H), 5.04 (brs, OH, 1H), 5.83 (d, 1H), 6.10 (dd, 1H), 6.40 (d, 1H), 5.04 (s, OH, 1H), 7.11 (Ar, 5H), 7.28 (Ar, 5H), 7.43 (Ar, 12H), 7.70 (Ar, 4H)

The compound S-9 (6.3 g) was dissolved in 30 mL of dichloromethane, and 100 mg of dibutyltin diacetate was added. 2-Isocyanatoethyl acrylate (1.1 g) was added to the prepared solution, and the mixture was allowed to react at room temperature. After a lapse of 24 hours, an additional 0.5 g of 2-isocyanatoethyl acrylate was added, and the mixture was allowed to further react for 24 hours. A saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 5.1 g of a compound M-3 (yield: 70%).

The NMR measurement data of the compound M-3 is as follows.

$^1$H NMR (400MHz, $CDCl_3$, $\delta$, ppm) 2.71 (m, 4H), 3.13 (d, 1H), 3.23 (m, 3H), 3.77 (m, 2H), 4.08 (m, 2H), 4.69 (brs, NH, 1H), 5.83 (d, 1H), 6.10 (dd, 1H), 6.41 (d, 1H), 7.12 (Ar, 10H), 7.35 (Ar, 14H), 7.73 (Ar, 2H)

A holographic recording medium was produced and evaluated in the same manner as in Example 1, except that compound M-3 was used as the polymerizable monomer. The results are shown in Table 1 below.

(Example 4)

A compound M-4 was produced by the following synthesis method.

[chem. 25]

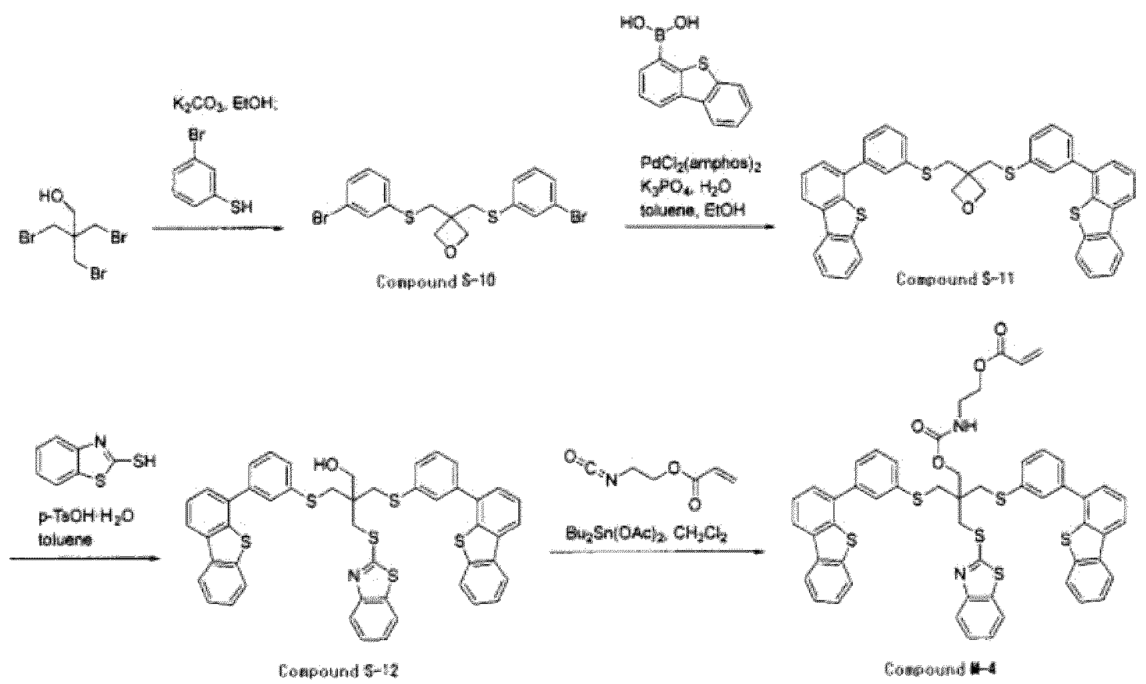


Compound S-10

Compound S-11

Compound S-12

Compound M-4

10 g of pentaerythritol tribromide and 12.8 g of potassium carbonate were suspended in 50 mL of ethanol. The

reaction mixture was heated to 100°C and stirred for 4 hours while the progress of the reaction was checked by TLC analysis. The mixture was cooled to room temperature, and 11.6 g of 3-bromobenzenethiol was slowly added. The white suspension was again heated to 100°C and stirred for 1 hour. The mixture was cooled to room temperature, and the white solid was separated by filtration. Then the solid was washed with 100 mL of ethyl acetate. The organic layer obtained was concentrated. The crude product obtained was purified using a silica gel column (hexane/ethyl acetate) to thereby obtain 11.8 g of a compound S-10 (yield: 83%).

The NMR measurement data of the compound S-10 is as follows.

$^1$H NMR (400MHz, $CDCl_3$, $\delta$, ppm) 3.44 (s, 4H), 4.43 (s, 4H), 7.12 (dd, 2H), 7.27 (Ar, 2H), 7.31 (Ar, 2H), 7.49 (dd, 2H)

The compound S-10 (2 g), dibenzothiophene-4-boronic acid (3.9 g), and potassium phosphate (4.6 g) were suspended in 20 mL of toluene, 10 mL of ethanol, and 10 mL of water, and nitrogen gas was bubbled through the suspension to degas the suspension. 31 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 6 hours. The resulting mixture was cooled to room temperature, extracted with ethyl acetate, and washed with water. The aqueous layer was extracted twice with ethyl acetate. 0.4 g of activated carbon was added to the obtained organic layer, and the mixture was stirred for 30 minutes. The mixture was filtered through celite and concentrated, and the crude product obtained was purified using a silica gel column (hexane/ethyl acetate) to thereby obtain 2.9 g of a compound S-11.

The NMR measurement data of the compound S-11 is as follows.

$^1$H NMR (400MHz, $CDCl_3$, $\delta$, ppm) 3.57 (s, 4H), 4.51 (s, 4H), 7.36 (Ar, 4H), 7.45 (Ar, 10H), 7.74 (Ar, 4H), 8.09 (dd, 2H), 8.14 (Ar, 2H)

The compound S-11 (2.9 g), 2-mercaptobenzothiazole (0.87 g), and toluenesulfonic acid monohydrate (100 mg) were suspended in 50 mL of toluene. The reaction solution was heated to 120°C in a nitrogen atmosphere and stirred at reflux for 1 hour. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 2.8 g of a compound S-12 (yield: 76%).

The NMR measurement data of the compound S-12 is as follows.

$^1$H NMR (400MHz, $CDCl_3$, $\delta$, ppm) 3.23 (d, 2H), 3.44 (d, 2H), 3.64 (brd, 2H), 3.68 (s, 2H), 5.47 (brt, OH, 1H), 7.30 (ddd, 1H), 7.39 (Ar, 8H), 7.48 (Ar, 7H), 7.70 (d, 1H), 7.76 (Ar, 5H), 8.10 (dd, 2H), 8.15 (brd, 2H)

The compound S-12 (2.7 g) was dissolved in 15 mL of dichloromethane, and 60 mg of dibutyltin diacetate was added. 0.55 g of 2-isocyanatoethyl acrylate (Karenz AOI manufactured by Showa Denko Co.,Ltd.) was added to the prepared solution, and the mixture was allowed to react at room temperature. After a lapse of 24 hours, an additional 0.2 g of 2-isocyanatoethyl acrylate was added, and the mixture was allowed to further react for 24 hours. A saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 1.5 g of a compound M-4 (yield: 48%).

The NMR measurement data of the compound M-4 is as follows.

$^1$H NMR (400MHz, $CDCl_3$, $\delta$, ppm) 3.21 (dd, 2H), 3.39 (s, 4H), 3.84 (s, 2H), 4.01 (dd, 2H), 4.30 (s, 2H), 4.76 (brt, NH, 1H), 5.69 (d, 1H), 5.92 (dd, 1H), 6.29 (d, 1H), 7.21 (dd, 1H), 7.31 (Ar, 3H), 7.44 (Ar, 12H), 7.62 (brd, 1H), 7.76 (Ar, 5H), 8.11 (dd, 2H), 8.15 (brd, 2H)

A holographic recording medium was produced and evaluated in the same manner as in Example 1, except that compound M-4 was used as the polymerizable monomer. The results are shown in Table 1 below.

(Example 5)

A compound M-5 was produced by the following synthesis method.

[Chem. 26]

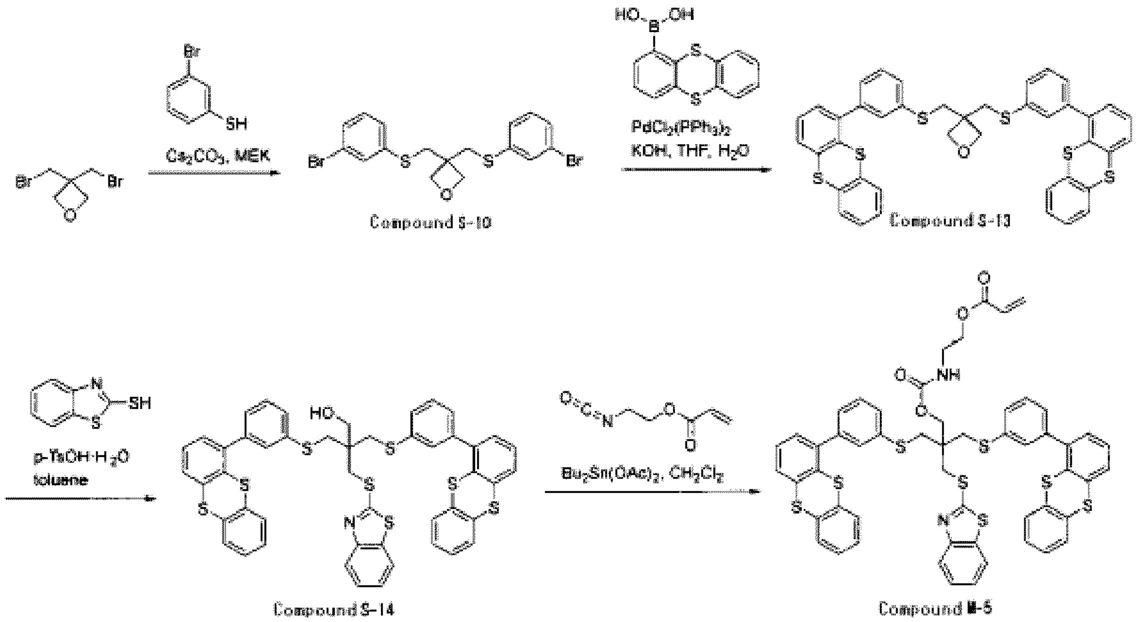


20 g of bis(bromomethyl)oxetane, 32.6 g of 2-bromobenzenethiol, and 56.1 g of cesium carbonate were suspended in 600 mL of methyl ethyl ketone (MEK). The reaction mixture was heated to 90°C and stirred at reflux for 3 hours while the progress of the reaction was checked by LC analysis. Then the mixture was cooled to room temperature and extracted with 600 mL of water and twice with 300 mL of ethyl acetate. The crude product obtained was purified using a silica gel column (hexane/ethyl acetate) to thereby obtain 35 g of the compound S-10 (yield: 94%). The NMR measurement data of the compound S-10 obtained was as described above.

The compound S-10 (29.2 g), thianthrene-1-boronic acid (36.3 g), 4.45 g of dichlorobis[triphenylphosphino]palladium (II), and potassium hydroxide (35.6 g) were suspended in 360 mL of tetrahydrofuran and 90 mL of water, and nitrogen gas was bubbled through the suspension to degas the suspension. The reaction solution was heated to 80°C in a nitrogen atmosphere and stirred for 12 hours. The reaction solution was cooled to room temperature. Then 200 mL of ethyl acetate was added, and the mixture was extracted with 450 mL of water. The organic layer was concentrated. The crude product obtained was purified using a silica gel column (heptane/ethyl acetate) to thereby obtain 33.4 g of a compound S-13 (yield: 66%).

The NMR measurement data of the compound S-13 is as follows.
$^1$H NMR (400MHz, $CDCl_3$, $\delta$, ppm) 3.56 (s, 4H), 4.48 (s, 4H), 7.14 (Ar, 4H), 7.21 (Ar, 6H), 7.33 (Ar, 4H), 7.43 (Ar, 4H), 7.49 (Ar, 4H)

The compound S-13 (8.5 g), 2-mercaptobenzothiazole (2.3 g), and toluenesulfonic acid monohydrate (50 mg) were suspended in 130 mL of toluene. The reaction solution was heated to 110°C in a nitrogen atmosphere and stirred at reflux for 1 hour. The mixture was cooled to room temperature. Then 100 mL of toluene was added, and the resulting mixture was washed with a 1M aqueous sodium hydroxide solution. The organic layer was washed with 0.1N hydrochloric acid and washed again with water. Chloroform was added to dissolve insoluble matter in the organic layer, and the resulting mixture was dried over anhydrous magnesium sulfate and concentrated. The crude product obtained was purified using a silica gel column (hexane/ethyl acetate) to thereby obtain 7.9 g of a compound S-14 (yield: 76%).

The NMR measurement data of the compound S-14 is as follows.
$^1$H NMR (400MHz, $CDCl_3$, $\delta$, ppm) 3.22 (d, 2H), 3.42 (d, 2H), 3.63 (d, 2H), 3.67 (s, 2H), 5.46 (t, OH, 1H), 7.09 (ddd, 2H), 7.20 (Ar, 8H), 7.29 (ddd, 1H), 7.30 (d, 2H), 7.34 (d, 2H), 7.40 (ddd, 1H), 7.47 (Ar, 8H), 7.69 (Ar, 1H), 7.77 (Ar, 1H)

The compound S-14 (4.0 g) was dissolved in 40 mL of dichloromethane, and 38 mg of dibutyltin diacetate was added. 2-Isocyanatoethyl acrylate (1.1 g) was added to the prepared solution, and the mixture was allowed to react at room temperature for 50 hours. After the reaction, the solution was concentrated at 30°C or lower. The crude product obtained by concentration was purified using a silica gel column (hexane/ethyl acetate) to thereby obtain 3.6 g of a compound M-5 (yield: 80%).

The NMR measurement data of the compound M-5 is as follows.
$^1$H NMR (400MHz, $CDCl_3$, $\delta$, ppm) 3.24 (m, 2H), 3.37 (s, 4H), 3.83 (s, 2H), 4.04 (m, 2H), 4.28 (s, 2H), 4.89 (brt, NH, 1H), 5.74 (d, 1H), 5.94 (dd, 1H), 6.31 (d, 1H), 7.12 (Ar, 6H), 7.20 (Ar, 4H), 7.27 (Ar, 5H), 7.33 (ddd, 1H), 7.40 (Ar, 4H), 7.48 (Ar, 4H), 7.65 (Ar, 1H), 7.77 (Ar, 1H)

A holographic recording medium was produced and evaluated in the same manner as in Example 1, except that compound M-5 was used as the polymerizable monomer. The results are shown in Table 1 below.

(Example 6)

A compound M-6 was produced by the following synthesis method.

[Chem. 27]

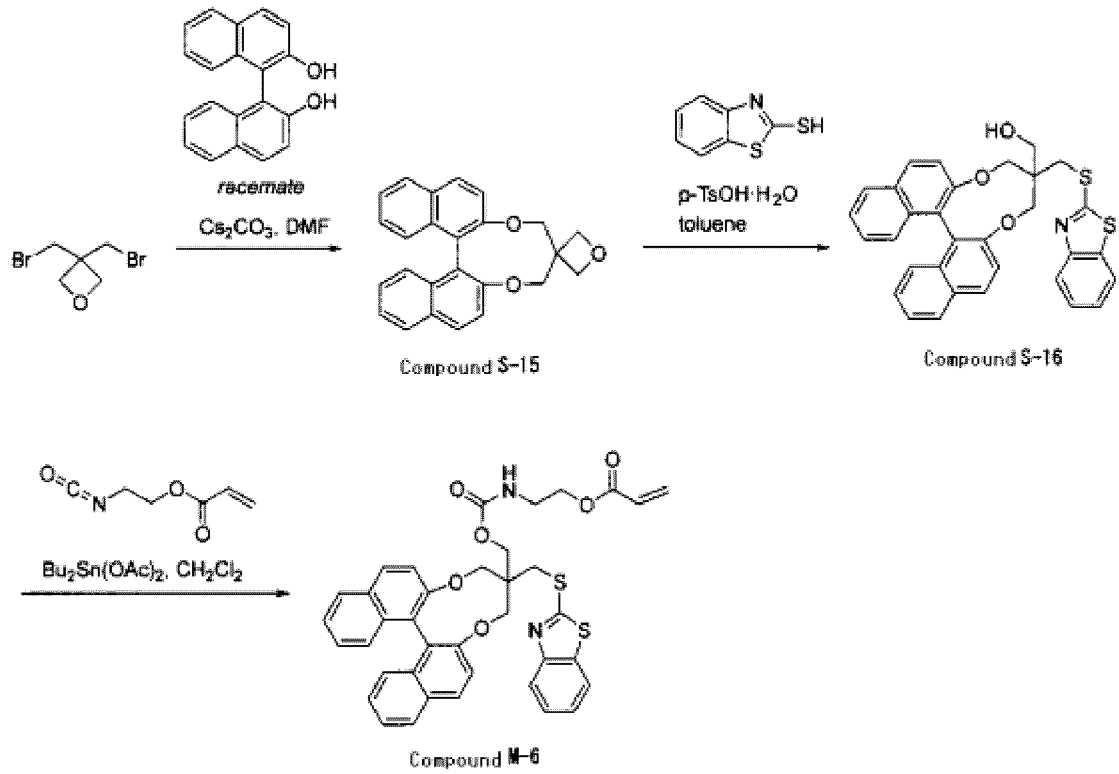


Compound S-15

Compound S-16

Compound M-6

1.0 g of 1,1'-bi-2-naphthol, 1.0 g of bis(bromomethyl)oxetane, and 1.2 g of cesium carbonate were suspended in 8 mL of N,N-dimethylformamide (DMF). The reaction mixture was heated to 120°C and stirred for 3 hours. The resulting mixture was cooled to room temperature, and the white solid generated was separated by filtration, washed with methanol, and dried under reduced pressure to thereby obtain 1.3 g of a compound S-15.

The NMR measurement data of the compound S-15 is as follows.

$^1$H NMR (400MHz, $CDCl_3$, $\delta$, ppm) 4.28 (d, 2H), 4.39 (d, 2H), 4.49 (d, 2H), 4.76 (d, 2H), 7.21 (Ar, 4H), 7.37 (Ar, 2H), 7.55 (d, 2H), 7.87 (d, 2H), 7.95 (d, 2H)

The compound S-15 (5.0 g), 2-mercaptobenzothiazole (2.3 g), and toluenesulfonic acid monohydrate (100 mg) were suspended in 50 mL of toluene. The reaction solution was heated to 120°C in a nitrogen atmosphere and stirred at reflux for 1 hour. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 5.5 g of a compound S-16 (yield: 78%).

The NMR measurement data of the compound S-16 is as follows.

$^1$H NMR (400MHz, $CDCl_3$, $\delta$, ppm) 3.34 (m, 3H), 3.51 (d, 1H), 4.08 (d, 1H), 4.32 (d, 1H), 4.54 (brd, 1H), 4.82 (brd, 1H), 5.58 (t, OH, 1H), 7.21 (Ar, 2H), 7.27 (Ar, 2H), 7.31 (Ar, 1H), 7.38 (Ar, 3H), 7.45 (d, 1H), 7.58 (d, 1H), 7.72 (d, 1H), 7.78 (d, 1H), 7.89 (Ar, 2H), 7.98 (Ar, 2H)

The compound S-16 (2.5 g) was dissolved in 16 mL of dichloromethane, and 29 mg of dibutyltin diacetate was added. 2-Isocyanatoethyl acrylate (0.98 g) was added to the prepared solution, and the mixture was allowed to react at 30°C for 30 hours. After the reaction the solution was concentrated at 30°C or lower. The crude product obtained by concentration was purified using a silica gel column (hexane/ethyl acetate) to thereby obtain 2.0 g of a compound M-6 (yield: 70%).

The NMR measurement data of the compound M-6 is as follows.

$^1$H NMR (400MHz, $CDCl_3$, $\delta$, ppm) 3.37 (m, 2H), 3.42 (d, 1H), 3.52 (d, 1H), 3.95 (d, 1H), 4.05 (d, 1H), 4.17 (m, 2H), 4.31 (d, 1H), 4.49 (s, 2H), 4.69 (d, 1H), 4.95 (brt, NH, 1H), 5.83 (d, 1H), 6.11 (dd, 1H), 6.41 (d, 1H), 7.22 (Ar, 4H), 7.28 (Ar,

1H), 7.36 (Ar, 3H), 7.45 (d, 1H), 7.51 (d, 1H), 7.66 (d, 1H), 7.72 (d, 1H), 7.78 (d, 1H), 7.86 (Ar, 2H), 7.95 (d, 1H)

A holographic recording medium was produced and evaluated in the same manner as in Example 1, except that compound M-6 was used as the polymerizable monomer. The results are shown in Table 1 below.

(Example 7)

A compound M-7 was produced by the following synthesis method.

[Chem. 28]

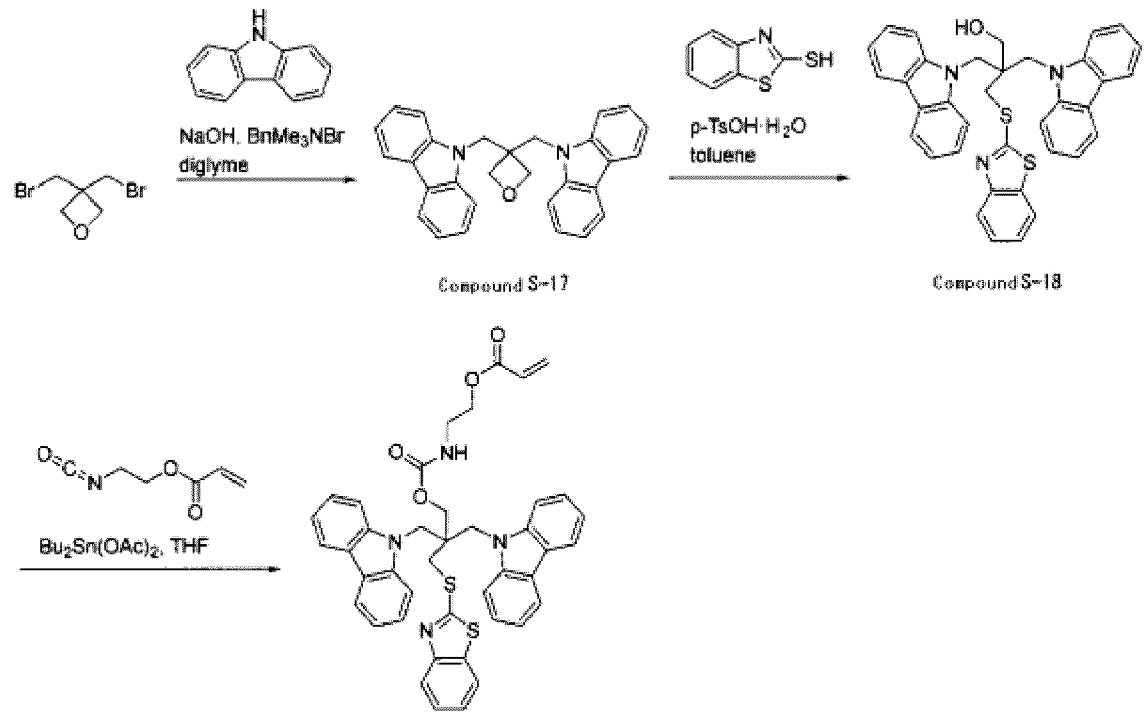


Compound S-17

Compound S-18

Compound M-7

1.4 g of 9H-carbazole, 1.0 g of bis(bromomethyl)oxetane, 0.66 g of sodium hydroxide, and 20 mg of benzyltrimethylammonium bromide were suspended in 5 mL of diethylene glycol dimethyl ether (diglyme). The reaction mixture was heated to 120°C and stirred for 3 hours. The resulting mixture was cooled to room temperature, and the white solid generated was separated by filtration, washed with methanol, and dried under reduced pressure to thereby obtain 1.3 g of a compound S-17 (yield: 76%).

The NMR measurement data of the compound S-17 is as follows.

$^1$H NMR (400MHz, $CDCl_3$, $\delta$, ppm) 4.64 (s, 4H), 4.69 (s, 4H), 7.27 (Ar, 8H), 7.43 (Ar, 4H), 8.13 (brd, 4H)

The compound S-17 (5.3 g), 2-mercaptobenzothiazole (2.1 g), and toluenesulfonic acid monohydrate (100 mg) were suspended in 30 mL of toluene. The reaction solution was heated to 120°C in a nitrogen atmosphere and stirred at reflux for 1 hour. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was washed with a small amount of methanol to thereby obtain 6.9 g of a compound S-18 (yield: 94%).

The NMR measurement data of the compound S-18 is as follows.

$^1$H NMR (400MHz, $CDCl_3$, $\delta$, ppm) 3.89 (s, 2H), 4.18 (d, 2H), 4.50 (d, 2H), 4.88 (d, 2H), 5.20 (t, OH, 1H), 7.25 (Ar,

5H), 7.44 (Ar, 9H), 7.61 (d, 1H), 7.82 (d, 1H), 8.11 (brd, 4H)

The compound S-18 (3.8 g) was dissolved in 30 mL of tetrahydrofuran, and 28 mg of dibutyltin diacetate was added. 2-Isocyanatoethyl acrylate (1.0 g) was added to the prepared solution, and the mixture was allowed to react at room temperature. After a lapse of 48 hours, a saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using

a silica gel column (hexane/ethyl acetate) to thereby obtain 3.4 g of a compound M-7 (yield: 72%).

The NMR measurement data of the compound M-7 is as follows.

$^1$H NMR (400MHz, $CDCl_3$, $\delta$, ppm) 3.24 (m, 2H), 3.75 (m, 2H), 3.99 (s, 2H), 4.06 (t, 2H), 4.40 (t, OH, 1H), 4.43 (s, 2H), 4.68 (d, 2H), 4.73 (d, 2H), 5.86 (d, 1H), 6.13 (dd, 1H), 6.42 (d, 1H), 7.23 (Ar, 4H), 7.29 (Ar, 1H), 7.39 (Ar, 9H), 7.72 (d, 1H), 7.80 (d, 1H), 8.08 (d, 4H)

A holographic recording medium was produced and evaluated in the same manner as in Example 1, except that compound M-7 was used as the polymerizable monomer. The results are shown in Table 1 below.

(Example 8)

A compound M-8 was produced by the following synthesis method.

[Chem. 29]

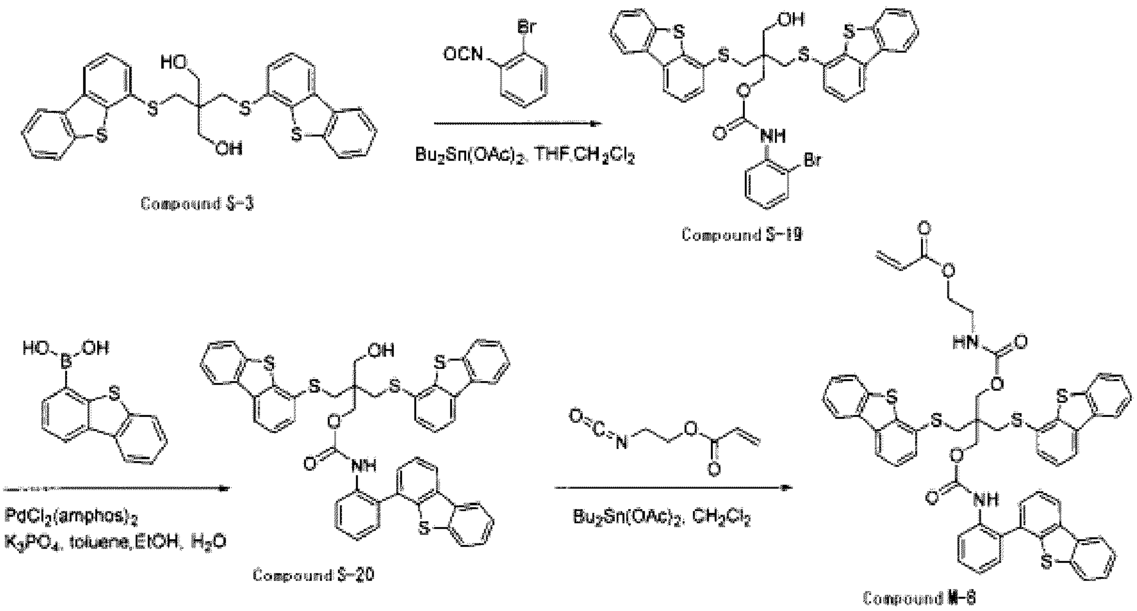


Compound S-3

Compound S-19

Compound S-20

Compound M-8

The compound S-3 (2.0 g) obtained in Example 1 was dissolved in 8 mL of tetrahydrofuran in nitorogen atmosphere, and 32 mL of dichloromethane and 26 mg of dibutyltin diacetate were added. 1.3 g of 2-bromobenzene isocyanate was added in three portions to the prepared solution, and the mixture was allowed to react at an internal temperature of 5°C for 5 hours. After completion of the reaction, the solvent was removed by evaporation under reduced pressure, and the crude product obtained was purified using a silica gel column to thereby obtain 1.85 g of a compound S-19 (yield: 68%).

The NMR measurement data of the compound S-19 is as follows.

$^1$H NMR (400MHz, $CDCl_3$, $\delta$, ppm) 2.29 (t, OH, 1H), 3.33 (s, CH2, 4H), 3.68 (d, CH2, 2H), 4.29 (s, CH2, 2H), 6.79 (s, NH, 1H), 6.91 (Ar, 1H), 7.24 (Ar, 1H), 7.32 (Ar, 2H), 7.40-7.48 (Ar, 5H), 7.54 (Ar, 2H), 7.82 (Ar, 2H), 7.92 (Ar, 2H), 7.95 (Ar, 1H), 8.02 (Ar, 2H)

The compound S-19 (1.84 g), dibenzothiophene-4-boronic acid (1.21 g), and potassium phosphate (1.28 g) were suspended in 14 mL of toluene, 14 mL of ethanol, and 7 mL of water, and nitrogen gas was bubbled through the suspension to degas the suspension. 34 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 2 hours. The resulting solution was cooled to room temperature, extracted with ethyl acetate, and washed with a 1N aqueous NaOH solution. The solution was further washed with water and brine, and the organic layer was dried over anhydrous magnesium sulfate. The resulting solution was concentrated, and the crude product obtained was purified using a silica gel column to thereby obtain 1.8 g of a compound S-20 (yield: 89%).

The NMR measurement data of the compound S-20 is as follows.

$^1$H NMR (400MHz, $CDCl_3$, $\delta$, ppm) 2.65 (t, OH, 1H), 2.81-3.28 (br, CH2, 4H), 3.49 (d, CH2, 2H), 4.14 (br, CH2, 2H), 6.25 (s, NH, 1H), 6.95-7.53 (Ar, 15H) 7.70-7.85 (Ar, 5H), 7.94-8.05 (Ar, 3H), 8.07-8.15 (Ar, 2H)

The compound S-20 (1.7 g) was dissolved in 17 mL of dichloromethane, and 16 mg of dibutyltin diacetate was added. 0.42 g of 2-isocyanatoethyl acrylate was added to the prepared solution, and the mixture was allowed to react at room temperature for about 72 hours. After completion of the reaction, the product was purified using a silica gel column (dichloromethane/ethyl acetate), and the fractions obtained were concentrated at 30°C or lower to a total weight of 10 g. The resulting solution was added dropwise to 100 mL of ice-cooled methanol, and the mixture was stirred in this state for 1 hour. The precipitate was separated by filtration, washed with methanol, and dried to thereby obtain 1.2 g of a compound M-8 (yield: 68%).

The NMR measurement data of the compound M-8 is as follows.
$^1$H NMR (400MHz, $CDCl_3$, $\delta$, ppm) 2.92-3.20 (brm, CH2, 4H), 3.24 (brq, CH2, 2H), 3.84-4.16 (brm, CH2, 4H), 4.20 (s, CH2, 2H), 4.55 (brt, NH, 1H), 5.79 (dd, 1H), 6.05 (dd, 1H), 6.24 (s, NH, 1H), 6.37 (dd, 1H), 7.11-7.25 (Ar, 5H), 7.30-7.48 (Ar, 10H), 7.71-7.81 (Ar, 3H), 7.85 (Ar, 2H), 7.95-8.06 (Ar, 3H), 8.07-8.14 (Ar, 2H)

A holographic recording medium was produced and evaluated in the same manner as in Example 1, except that compound M-8 was used as the polymerizable monomer. The results are shown in Table 1 below.

(Example 9)

A compound M-9 was produced by the following synthesis method.

[Chem. 30]

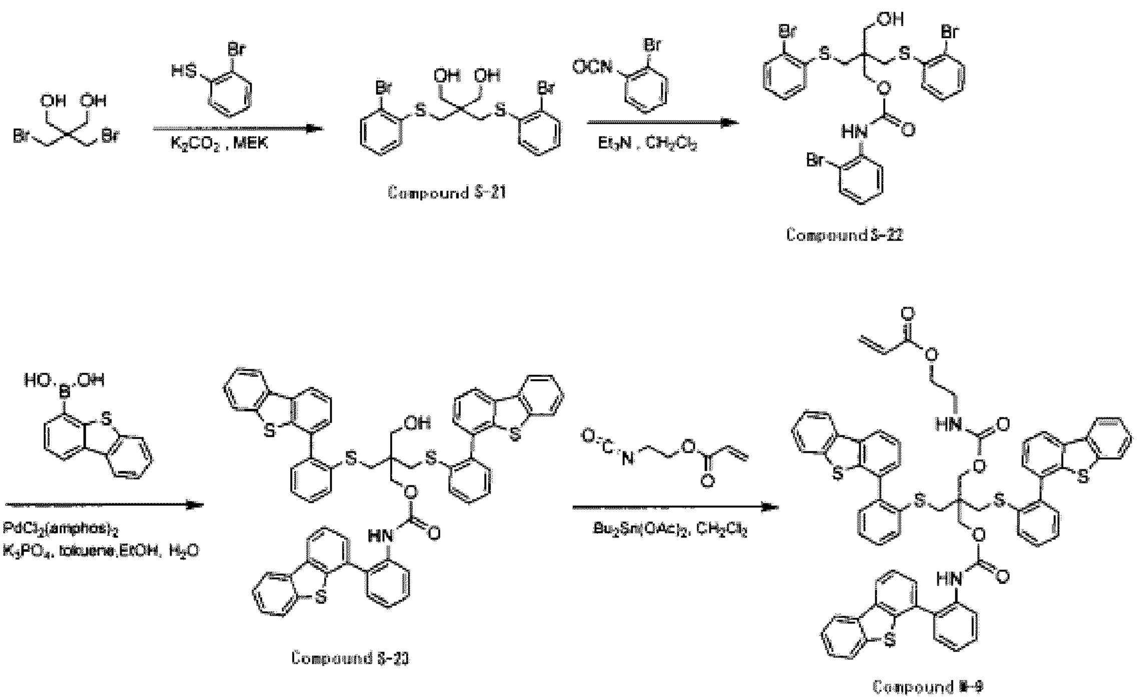


Compound S-21

Compound S-22

Compound S-23

Compound M-9

8.1 g of pentaerythritol dibromide was dissolved in 80 mL of methyl ethyl ketone (MEK) in a nitrogen atmosphere. 12.9 g of 2-bromobenzenethiol and 9.2 g of potassium carbonate were added, and the resulting mixture was allowed to react for 4 hours under reflux conditions. The resulting mixture was cooled to room temperature, and water was added. The mixture was extracted with ethyl acetate, washed with a 1N aqueous sodium hydroxide solution, and washed with brine. The solution obtained was dried over anhydrous magnesium sulfate, concentrated, and then purified using a silica gel column (dichloromethane, ethyl acetate) to thereby obtain 13.4 g of a compound S-21 (yield: 89%).

The NMR measurement data of the compound S-21 is as follows.
$^1$H NMR (400MHz, $CDCl_3$, $\delta$, ppm) 2.13 (t, OH, 2H), 3.16 (s, CH2, 4H), 3.82 (d, CH2, 4H), 7.02 (Ar, 2H), 7.24 (Ar, 2H), 7.38 (Ar, 2H), 7.52 (Ar, 2H)

The compound S-21 (5.2 g) was dissolved in 75 mL of dichloromethane, and 22 mg of triethylamine was added. 2.3 g of 2-bromobenzene isocyanate was added in three portions to the prepared solution, and the mixture was allowed to react at room temperature for 7 hours. Water was added to terminate the reaction, and the mixture was subjected to separation. Then the organic layer was washed with 0.1N hydrochloric acid and water. The solution obtained was dried

over anhydrous magnesium sulfate, and the solvent was removed by evaporation under reduced pressure. The crude product obtained was purified using a silica gel column (dichloromethane, ethyl acetate) to thereby obtain 3.4 g of a compound S-22 (yield: 47%).

The NMR measurement data of the compound S-22 is as follows.

$^1$H NMR (400MHz, $CDCl_3$, $\delta$, ppm) 2.51 (t, OH, 1H), 3.19 (m, CH2, 4H), 3.69 (d, CH2, 2H), 4.33 (s, CH2, 2H), 6.96-7.03 (Ar, 4H), 7.20-7.35 (Ar, 3H), 7.40-7.43 (Ar, 2H), 7.50-7.55 (Ar, 3H), 8.05 (brd, NH,1H)

The compound S-22 (3.3 g), dibenzothiophene-4-boronic acid (3.7 g), and potassium phosphate (3.6 g) were suspended in 24 mL of toluene, 24 mL of ethanol, and 12 mL of water, and nitrogen gas was bubbled through the suspension to degas the suspension. 101 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 2 hours. The resulting mixture was cooled to room temperature, extracted with ethyl acetate, and washed with a 1N aqueous sodium hydroxide solution. The resulting mixture was further washed with water and brine, and the organic layer was dried over anhydrous magnesium sulfate. The resulting solution was concentrated, and the crude product obtained was purified using a silica gel column (dichloromethane, ethyl acetate) to thereby obtain 4.1 g of a compound S-23 (yield: 88%).

The NMR measurement data of the compound S-23 is as follows.

$^1$H NMR (400MHz, $CDCl_3$, $\delta$, ppm) 2.24 (brs, OH, 1H), 2.54 (brd, CH2, 4H), 3.02 (d, CH2, 2H), 3.76 (brs, CH2, 2H), 6.18 (s, NH, 1H), 6.98-7.27 (Ar, 11H), 7.31-7.49 (Ar, 11H), 7.53 (Ar, 1H), 7.67-7.75 (Ar,3H), 7.94 (Ar, 1H), 8.07 (Ar, 2H), 8.14 (Ar, 2H), 8.16-8.22 (Ar, 2H)

The compound S-23 (4.1 g) was dissolved in 40 mL of dichloromethane, and 36 mg of dibutyltin diacetate was added. 0.97 g of 2-isocyanatoethyl acrylate was added to the prepared solution, and the mixture was allowed to react at room temperature for about 72 hours. After completion of the reaction, the product was purified using a silica gel column (dichloromethane/ethyl acetate), and the fractions obtained were concentrated at 30°C or lower to a total weight of 20 g. The resulting solution was added dropwise to 350 mL of ice-cooled methanol, and the mixture was stirred in this state for 2 hours. The precipitate was separated by filtration, washed with methanol, and dried to thereby obtain 3.4 g of a compound M-9 (yield: 72%).

The NMR measurement data of the compound M-9 is as follows.

$^1$H NMR (400MHz, $CDCl_3$, $\delta$, ppm) 2.52 (brs, CH2, 4H), 2.88+3.16 (brs+d, CH2, 2H), 3.60 (brs, CH2, 2H), 3.75 (s, CH2, 2H), 3.85+4.03 (brs+t, CH2, 2H), 4.39 (brs, NH, 1H), 5.81 (d, 1H), 6.08 (dd, 1H), 6.18 (s, NH, 1H), 7.39 (d, 1H), 7.03-7.34 (Ar, 12H), 7.34-7.55 (Ar, 11H), 7.68-7.76 (Ar, 3H), 7.98 (Ar, 1H), 8.07 (Ar, 2H), 8.12-8.19 (Ar, 4H)

A holographic recording medium was produced and evaluated in the same manner as in Example 1, except that compound M-9 was used as the polymerizable monomer. The results are shown in Table 1 below.

(Example 10)

A compound M-10 was produced by the following synthesis method.

[Chem. 31]

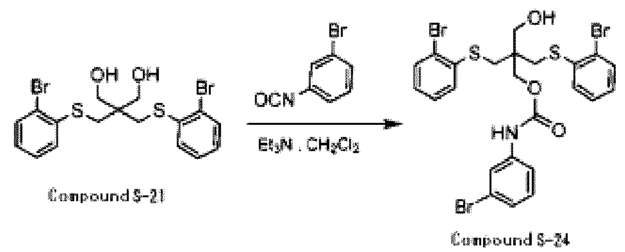


Compound S-21

Compound S-24

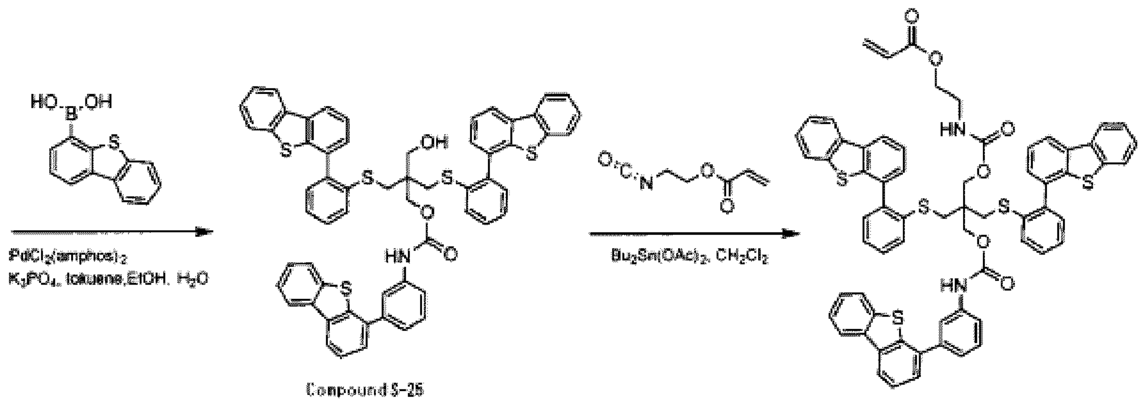


Compound S-25

Compound M-10

The compound S-21 (3.0 g) obtained in Example 9 was dissolved in 45 mL of dichloromethane, and 12 mg of triethylamine was added. 2.0 g of 3-bromobenzene isocyanate was added in three portions to the solution obtained, and the mixture was allowed to react at room temperature for 12 hours. Water was added to terminate the reaction, and the mixture was subjected to separation. Then the organic layer was washed with 0.1N hydrochloric acid and water. The solution obtained was dried over anhydrous magnesium sulfate, and the solvent was removed by evaporation under reduced pressure. The crude product obtained was purified using a silica gel column (dichloromethane, ethyl acetate) to thereby obtain 2.7 g of a compound S-24 (yield: 64%).

The NMR measurement data of the compound S-24 is as follows.

$^1$H NMR (400MHz, $CDCl_3$, $\delta$, ppm) 2.46 (t, OH, 1H), 3.16 (m, CH2, 4H), 3.68 (d, CH2, 2H), 4.32 (s, CH2, 2H), 6.55 (s, NH, 1H), 7.01 (Ar, 2H), 7.13-7.25 (Ar, 5H), 7.40 (Ar, 2H), 7.51 (Ar, 2H), 7.59 (Ar, 1H)

The compound S-24 (2.7 g), dibenzothiophene-4-boronic acid (3.0 g), and potassium phosphate (2.9 g) were suspended in 19 mL of toluene, 19 mL of ethanol, and 10 mL of water, and nitrogen gas was bubbled through the suspension to degas the suspension. 82 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 2 hours. The resulting solution was cooled to room temperature, extracted with ethyl acetate, and washed with a 1N aqueous sodium hydroxide solution. The solution was further washed with water and brine, and the organic layer was dried over anhydrous magnesium sulfate. The resulting solution was concentrated, and the crude product obtained was purified using a silica gel column (dichloromethane, ethyl acetate) to thereby obtain 3.4 g of a compound S-25 (yield: 85%).

The NMR measurement data of the compound S-25 is as follows.

$^1$H NMR (400MHz, $CDCl_3$, $\delta$, ppm) 1.99 (brs, OH, 1H), 2.67-2.79 (m, CH2, 4H), 3.17 (brd, CH2, 2H), 3.86 (brs, CH2, 2H), 6.29 (s, NH, 1H), 7.22-7.58 (Ar, 23H), 7.70-7.74 (Ar, 2H), 7.80-7.84 (Ar, 1H), 8.08-8.22 (Ar,7H)

The compound S-25 (2.9 g) was dissolved in 30 mL of dichloromethane, and 24 mg of dibutyltin diacetate was added. 0.63 g of 2-isocyanatoethyl acrylate was added to the prepared solution, and the mixture was allowed to react at room temperature for about 72 hours. After completion of the reaction, the product was purified using a silica gel column (dichloromethane/ethyl acetate), and the fractions obtained were concentrated at 30°C or lower to a total weight of 20 g. The resulting solution was added dropwise to 350 mL of ice-cooled methanol, and the mixture was stirred in this state for 2 hours. The precipitate was separated by filtration, washed with methanol, and dried to thereby obtain 2.9 g of a compound M-10 (yield: 89%).

The NMR measurement data of the compound M-10 is as follows.

$^1H$ NMR (400MHz, $CDCl_3$, δ, ppm) 2.68 (brs, CH2, 4H), 3.00+3.22 (brs+m, CH2, 2H), 3.76 (brs, CH2, 2H), 3.82 (brs, CH2, 2H), 3.92+4.07 (brs+t, CH2, 2H), 4.48 (brt, NH, 1H), 5.80 (d, 1H), 6.11 (dd, 1H), 6.33 (s, NH, 1H), 6.39 (d, 1H), 7.19-7.50 (Ar, 22H), 7.53-7.60 (Ar, 2H), 7.71-7.76 (Ar, 2H), 7.80-7.85 (Ar, 1H), 8.08-8.23 (Ar, 6H)

A holographic recording medium was produced and evaluated in the same manner as in Example 1, except that compound M-10 was used as the polymerizable monomer. The results are shown in Table 1 below.

(Example 11)

A compound M-11 was produced by the following synthesis method.

[Chem. 32]

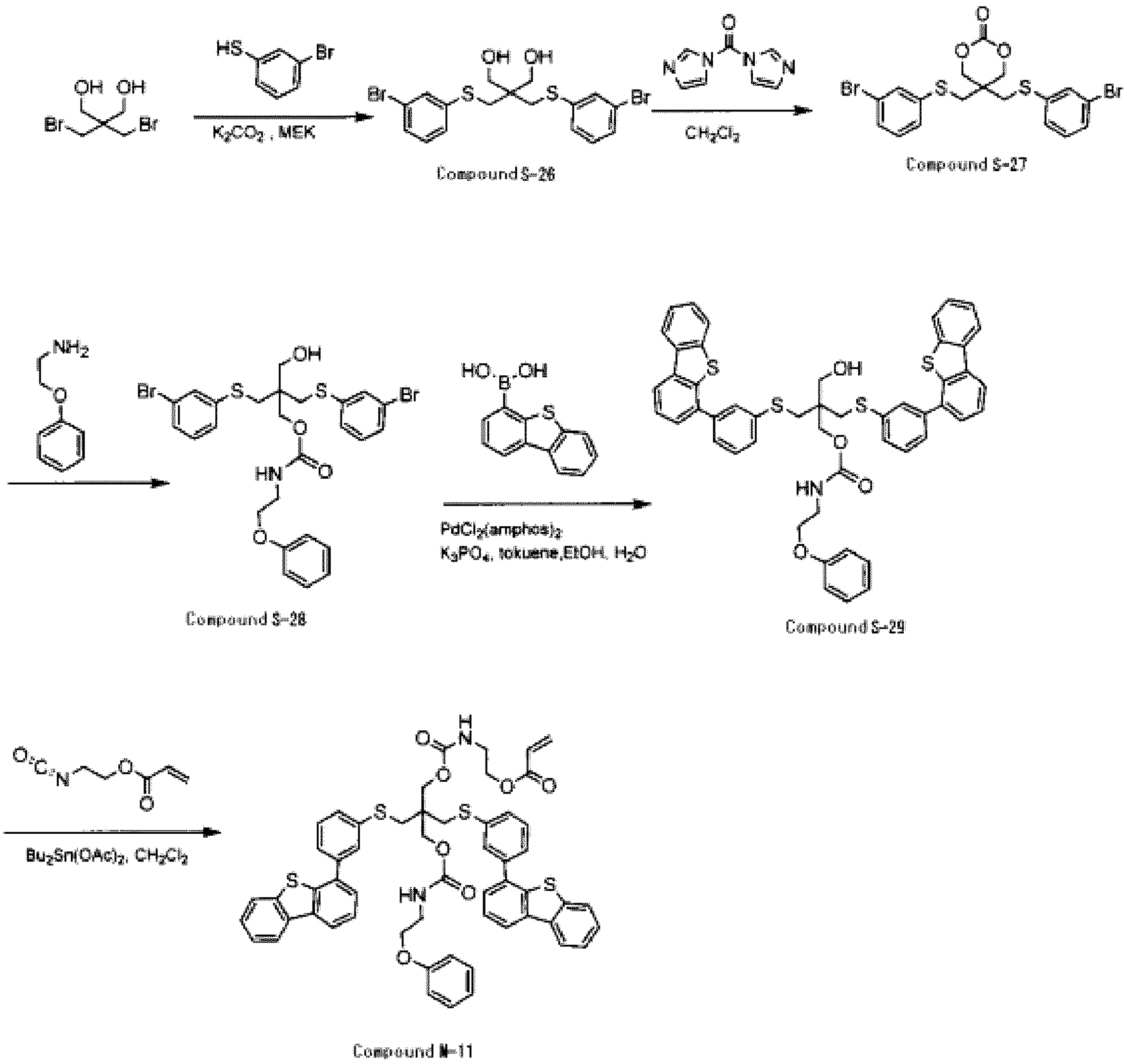

Compound S-26
Compound S-27
Compound S-28
Compound S-29
Compound M-11

8.0 g of pentaerythritol dibromide was dissolved in 80 mL of methyl ethyl ketone (MEK) in a nitrogen atmosphere. 12.7 g of 3-bromobenzenethiol and 9.1 g of potassium carbonate were added, and the resulting mixture was allowed to react for 4 hours under reflux conditions. The resulting mixture was cooled to room temperature, and water was added. The mixture was extracted with ethyl acetate, washed with a 1N aqueous sodium hydroxide solution, and washed with brine. The solution obtained was dried over anhydrous magnesium sulfate, concentrated, and then purified using a silica gel column (dichloromethane, ethyl acetate) to thereby obtain 13.0 g of a compound S-26 (yield: 87%).

The NMR measurement data of the compound S-26 is as follows.

$^1H$ NMR (400MHz, $CDCl_3$, δ, ppm) 2.06 (t, OH, 2H), 3.12 (s, CH2, 4H), 3.73 (d, CH2, 2H), 7.12 (Ar, 2H), 7.26-7.32 (Ar,

4H), 7.49 (Ar, 2H)

The compound S-26 (6.0 g) was dissolved in 120 mL of dichloromethane, and 2.25 g of carbonyldiimidazole was added. The mixture was allowed to react at room temperature for 3 hours. Water was added to terminate the reaction, and the mixture was subjected to separation. Then the organic layer was washed with 0.1N hydrochloric acid. The resulting organic layer was further washed with brine and with water and dried over anhydrous magnesium sulfate. The resulting solution was concentrated, and the crude product obtained was purified using a silica gel column (dichloromethane, ethyl acetate) to thereby obtain 5.2 g of a compound S-27 (yield: 79%).

The NMR measurement data of the compound S-27 is as follows.

$^1$H NMR (400MHz, $CDCl_3$, $\delta$, ppm) 3.19 (s, CH2, 4H), 4.31 (s, CH2, 4H), 7.16 (Ar, 2H), 7.27 (Ar, 2H), 7.36 (Ar, 2H), 7.48 (Ar, 2H)

The compound S-27 (1.5 g) was dissolved in 10 mL of dichloromethane, and 0.55 g of 2-phenoxyethylamine was added. The mixture was allowed to react at room temperature for 4 hours, and the resulting mixture was washed with water and dried over anhydrous magnesium sulfate. The product was concentrated, and the crude product obtained was purified using a silica gel column (dichloromethane, ethyl acetate) to thereby obtain 1.7 g of a compound S-28 (yield: 87%).

The NMR measurement data of the compound S-28 is as follows.

$^1$H NMR (400MHz, $CDCl_3$, $\delta$, ppm) 2.97 (t, OH, 1H), 3.00-3.15 (m, CH2, 4H), 3.50 (d, CH2 2H), 3.44+3.59 (brs+q, CH2, 2H), 3.97+4.03 (brs+t, CH2, 2H), 4.14+4.23 (s+brs, CH2, 2H), 5.00+5.23 (brs+brt, NH, 1H), 6.89 (Ar, 2H), 6.98 (Ar, 1H), 7.09 (Ar, 2H), 7.26-7.33 (Ar, 6H), 7.49 (Ar, 2H)

The compound S-28 (1.7 g), dibenzothiophene-4-boronic acid (1.2 g), and potassium phosphate (1.1 g) were suspended in 11 mL of toluene, 11 mL of ethanol, and 5 mL of water, and nitrogen gas was bubbled through the suspension to degas the suspension. 45 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 4 hours. The resulting mixture was cooled to room temperature, extracted with ethyl acetate, and washed with a 1N aqueous sodium hydroxide solution. The resulting mixture was further washed with water and brine, and the organic layer was dried over anhydrous magnesium sulfate. The resulting solution was concentrated, and the crude product obtained was purified using a silica gel column (dichloromethane, ethyl acetate) to thereby obtain 1.7 g of a compound S-29 (yield: 84%).

The NMR measurement data of the compound S-29 is as follows.

$^1$H NMR (400MHz, $CDCl_3$, $\delta$, ppm) 2.92 (t, OH, 1H), 3.22 (dd, CH2, 4H), 3.28+3.45 (brs+q, CH2, 2H), 3.58 (d, CH2, 2H), 3.69+3.89 (brs+t, CH2, 2H), 4.24+4.32 (s+brs, CH2, 2H), 4.89+5.06 (brs+brt, NH, 1H), 6.70+6.76 (Ar, 2H), 6.92 (Ar, 1H), 7.21 (Ar, 2H), 7. 7.33 (Ar, 2H), 7.38-7.53 (Ar, 12H), 7.76 (Ar, 4H), 8.12 (Ar, 4H)

The compound S-29 (1.8 g) was dissolved in 17 mL of dichloromethane, and 17 mg of dibutyltin diacetate was added. 0.46 g of 2-isocyanatoethyl acrylate was added to the prepared solution, and the mixture was allowed to react at room temperature for about 72 hours. After completion of the reaction, the product was purified using a silica gel column (dichloromethane/ethyl acetate), and the fractions obtained were concentrated at 30°C or lower to a total weight of 10 g. The resulting solution was added dropwise to 180 mL of ice-cooled methanol, and the mixture was stirred in this state for 2 hours. The precipitate was separated by filtration, washed with methanol, and dried to thereby obtain 1.2 g of a compound M-11 (yield: 61%).

The NMR measurement data of the compound M-11 is as follows.

$^1$H NMR (400MHz, $CDCl_3$, $\delta$, ppm), 3.21-3.35 (br, CH2, 6H), 3.41 (q, CH2, 2H), 3.77+3.86 (brs+t, CH2, 2H), 4.07 (t, CH2, 2H), 4.24 (brs, CH2, 2H), 4.80 (brt, NH, 1H), 4.67+4.99 (brs+brt, NH, 1H), 5.70 (d, 1H), 5.94 (dd, 1H), 6.30 (d, 1H), 6.72 (Ar, 2H), 6.92 (Ar, 1H), 7.18 (Ar, 2H), 7.32 (Ar, 2H), 7.38-7.53 (Ar, 12H), 7.75-7.82 (Ar, 4H), 8.12 (Ar, 4H)

A holographic recording medium was produced and evaluated in the same manner as in Example 1, except that compound M-11 was used as the polymerizable monomer. The results are shown in Table 1 below.

(Example 12)

A compound M-12 was produced by the following synthesis method.

[Chem. 33]

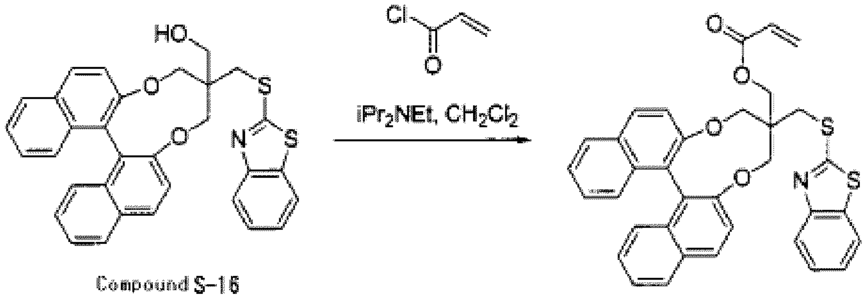


Compound S-16

Compound M-12

The compound S-16 (1.3 g) obtained in Example 6 and 0.6 mL of diisopropylethylamine were dissolved in 10 mL of dichloromethane, and the mixture was cooled to 0°C. 1 mL of a dichloromethane solution of 300 mg of acryloyl chloride was added to the solution obtained, and the mixture was stirred for 2 hours. After completion of the reaction, 10 mL of a saturated aqueous sodium hydrogencarbonate solution was added, and the mixture was extracted twice with 50 mL of dichloromethane. The organic layer was dried over mirabilite, filtered, and concentrated. The crude product obtained was purified using a silica gel column (hexane/ethyl acetate) to thereby obtain 770 mg of a compound M-12 (yield: 54%).

The NMR measurement data of the compound M-12 is as follows.
$^1$H NMR (400MHz, $CDCl_3$, $\delta$, ppm) 3.34 (d, 1H), 3.53 (d, 1H), 4.06 (d, 1H), 4.09 (d, 1H), 4.37 (d, 1H), 4.47 (d, 1H), 4.56 (d, 1H), 4.70 (d, 1H), 5.83 (dd, 1H), 6.08 (dd, 1H), 6.37 (dd, 1H), 7.22 (Ar, 4H), 7.28 (Ar, 1H), 7.37 (Ar, 3H), 7.45 (d, 1H), 7.51 (d, 1H), 7.66 (brd, 1H), 7.73 (brd, 1H), 7.80 (d, 1H), 7.87 (Ar, 2H), 7.94 (d, 1H)

(Example 13)

A compound M-13 was produced by the following synthesis method.

[Chem. 34]

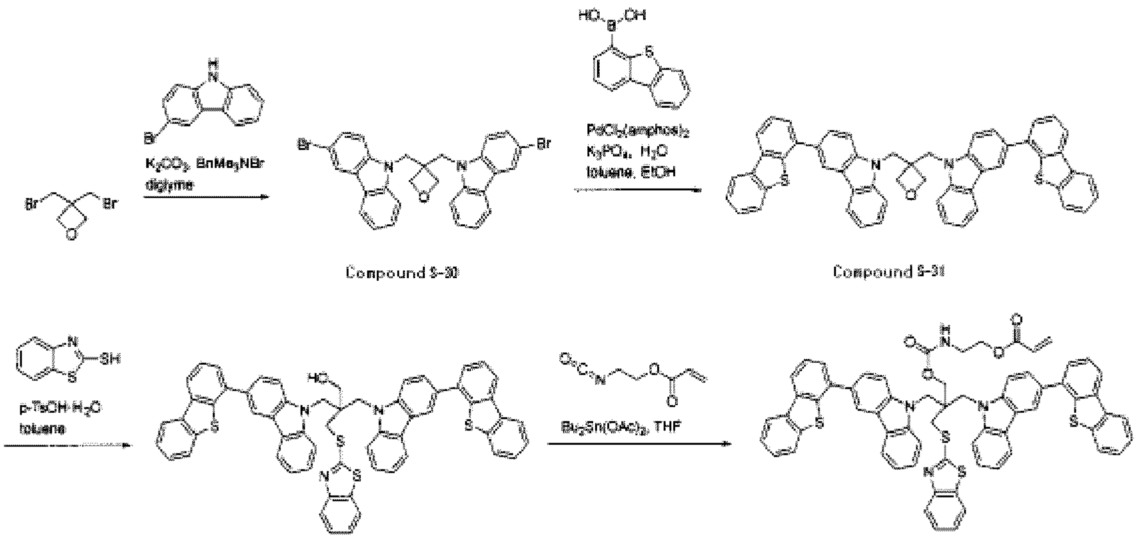


Compound S-30

Compound S-31

Compound S-32

Compound M-13

5.1 g of 3-bromo-9H-carbazole, 2.5 g of bis(bromomethyl)oxetane, 2.9 g of potassium carbonate, and 0.1 g of benzyltrimethylammonium bromide were suspended in 5 mL of diethylene glycol dimethyl ether (diglyme). The reaction mixture was heated to 120°C and stirred for 3 hours. Then the mixture was cooled to room temperature, the resulting solid was filtered off, washed with ethyl acetate/hexane mixture, and dried under reduced pressure to thereby obtain

5.2 g of the compound S-30.

The NMR measurement data of the compound S-30 is as follows.

$^1$H NMR (400MHz, $CDCl_3$, $\delta$, ppm) 4.58 (s, 4H), 4.63 (s, 4H), 7.14 (d, 2H), 7.26 (d, 2H), 7.29 (d, 2H), 7.45 (Ar, 2H), 7.50 (dd, 2H), 8.07 (d, 2H), 8.23 (d, 2H)

The compound S-30 (3.5 g), dibenzothiophene-4-boronic acid (2.8 g), and potassium phosphate (5.2 g) were suspended in 60 mL of toluene, 30 mL of ethanol, and 30 mL of water, and nitrogen gas was bubbled through the suspension to degas the suspension. 31 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 6 hours. The resultant solution was cooled to room temperature, extracted with ethyl acetate, and washed with water. The aqueous layer was extracted twice with ethyl acetate. 0.4 g of activated carbon was added to the organic layer obtained, and the mixture was stirred for 30 minutes. The mixture was filtered through celite and concentrated, and the crude product obtained was purified using a silica gel column (hexane/ethyl acetate) to thereby obtain 4.3 g of a compound S-31 (yield: 90%).

The NMR measurement data of the compound S-31 is as follows.

$^1$H NMR (400MHz, $CDCl_3$, $\delta$, ppm) 4.77 (s, 4H), 4.81 (s, 4H), 7.32 (t, 2H), 7.38 (d, 2H), 7.47 (Ar, 8H), 7.59 (d, 4H), 7.83 (Ar, 4H), 8.19 (Ar, 6H), 8.51 (d, 2H)

The compound S-31 (4.8 g), 2-mercaptobenzothiazole (1.1 g), and toluenesulfonic acid monohydrate (106 mg) were suspended in 30 mL of toluene. The reaction solution was heated to 100°C in a nitrogen atmosphere and stirred for 5 minutes. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 3.8 g of a compound S-32 (yield: 65%).

The NMR measurement data of the compound S-32 is as follows.

$^1$H NMR (400MHz, $CDCl_3$, $\delta$, ppm) 3.99 (s, 2H), 4.27 (s, 2H), 4.62 (d, 2H), 4.98 (d, 2H), 7.29 (t, 2H), 7.46 (Ar, 9H), 7.58 (Ar, 7H), 7.66 (Ar, 2H), 7.80 (Ar, 2H), 7.85 (Ar, 2H) 8.19 (Ar, 6H), 8.49 (d, 2H)

The compound S-32 (3.8 g) was dissolved in 30 mL of tetrahydrofuran, and 60 mg of dibutyltin diacetate was added. 2-Isocyanatoethyl acrylate (0.9 g) was added to the prepared solution, and the mixture was allowed to react at room temperature. After a lapse of 48 hours, a saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 3.4 g of a compound M-13 (yield: 78%).

The NMR measurement data of the compound M-13 is as follows.

$^1$H NMR (400MHz, $CDCl_3$, $\delta$, ppm) 3.32 (m, 2H), 4.11 (m, 4H), 4.55 (s, 2H), 4.61 (brt, NH, 1H), 4.82 (s, 4H), 5.74 (d, 1H), 6.08 (dd, 1H), 6.36 (d, 1H), 7.29 (t, 2H), 7.44 (Ar, 10H), 7.58 (Ar, 6H), 7.73 (Ar, 2H), 7.82 (Ar, 4H), 8.17 (Ar, 6H), 8.47 (d, 2H)

(Example 14)

A compound M-14 was produced by the following synthesis method.

[Chem. 35]

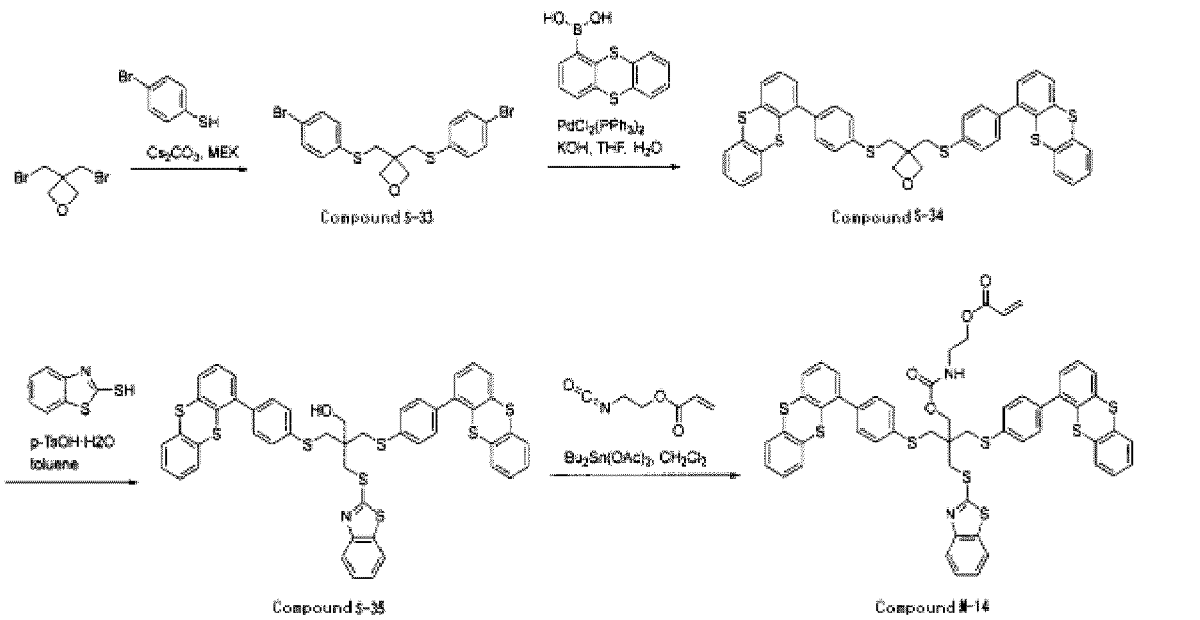


Compound S-33 Compound S-34 Compound S-35 Compound M-14

10 g of bis(bromomethyl)oxetane, 16.3 g of 4-bromobenzenethiol, and 28 g of cesium carbonate were suspended in 150 mL of methyl ethyl ketone (MEK). The reaction mixture was heated to 90°C and stirred at reflux for 3 hours while the progress of the reaction was checked by LC analysis. The mixture was cooled to room temperature and extracted with 300 mL of water and twice with 150 mL of ethyl acetate. The crude product obtained was purified using a silica gel column (hexane/ethyl acetate) to thereby obtain 18.1 g of a compound S-33 (yield: 94%).

The NMR measurement data of the compound S-33 is as follows.

$^1$H NMR (400MHz, $CDCl_3$, $\delta$, ppm) 3.42 (s, 4H), 4.41 (s, 4H), 7.22 (td, 4H), 7.40 (td, 4H)

The compound S-33 (6.8 g), thianthrene-1-boronic acid (8.5 g), 1.04 g of dichlorobis[triphenylphosphino]palladium (II), and potassium hydroxide (8.3 g) were suspended in 360 mL of tetrahydrofuran and 90 mL of water, and nitrogen gas was bubbled through the suspension to degas the suspension. The reaction solution was heated to 80°C in a nitrogen atmosphere and stirred for 12 hours. The reaction solution was cooled to room temperature. Then 100 mL of ethyl acetate was added, and the mixture was extracted with 200 mL of water. The organic layer was concentrated. The crude product obtained was purified using a silica gel column (heptane/ethyl acetate) to thereby obtain 6.4 g of a compound S-34 (yield: 59%).

The NMR measurement data of the compound S-34 is as follows.

$^1$H NMR (400MHz, $CDCl_3$, $\delta$, ppm) 3.60 (s, 4H), 4.53 (s, 4H), 7.16 (Ar, 4H), 7.22 (Ar, 4H), 7.34 (Ar, 6H), 7.49 (Ar, 8H)

The compound S-34 (5.5 g), 2-mercaptobenzothiazole (1.5 g), and toluenesulfonic acid monohydrate (100 mg) were suspended in 30 mL of toluene. The reaction solution was heated to 120°C in a nitrogen atmosphere and stirred at reflux for 1 hour. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 5.5 g of a compound S-35 (yield: 81%).

The NMR measurement data of the compound S-35 is as follows.

$^1$H NMR (400MHz, $CDCl_3$, $\delta$, ppm) 3.26 (d, 2H), 3.46 (d, 2H), 3.68 (d, 2H), 3.70 (s, 2H), 5.58 (t, OH, 1H), 7.20 (Ar, 5H), 7.33 (Ar, 8H), 7.46 (Ar, 7H), 7.55 (d, 4H), 7.74 (brd, 1H), 7.83 (brd, 1H)

The compound S-35 (3.6 g) was dissolved in 30 mL of dichloromethane, and 28 mg of dibutyltin diacetate was added. 2-Isocyanatoethyl acrylate (0.63 g) was added to the prepared solution, and the mixture was allowed to react at room temperature. After a lapse of 24 hours, an additional 0.5 g of 2-isocyanatoethyl acrylate was added, and the mixture was allowed to further react for 24 hours. A saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 1.0 g of a compound M-14 (yield: 24%).

The NMR measurement data of the compound M-14 is as follows.

$^1$H NMR (400MHz, $CDCl_3$, $\delta$, ppm) 3.39 (s, 4H), 3.84 (s, 2H), 4.17 (m, 4H), 4.32 (s, 2H), 5.00 (brt, OH, 1H), 5.80 (d, 1H), 6.12 (dd, 1H), 6.40 (d, 1H), 7.13 (Ar, 4H), 7.21 (Ar, 5H), 7.28 (Ar, 8H), 7.40 (Ar, 1H), 7.48 (d, 6H), 7.71 (brd, 1H), 7.87 (brd, 1H)

(Example 15)

A compound M-15 was produced by the following synthesis method.

[Chem. 36]

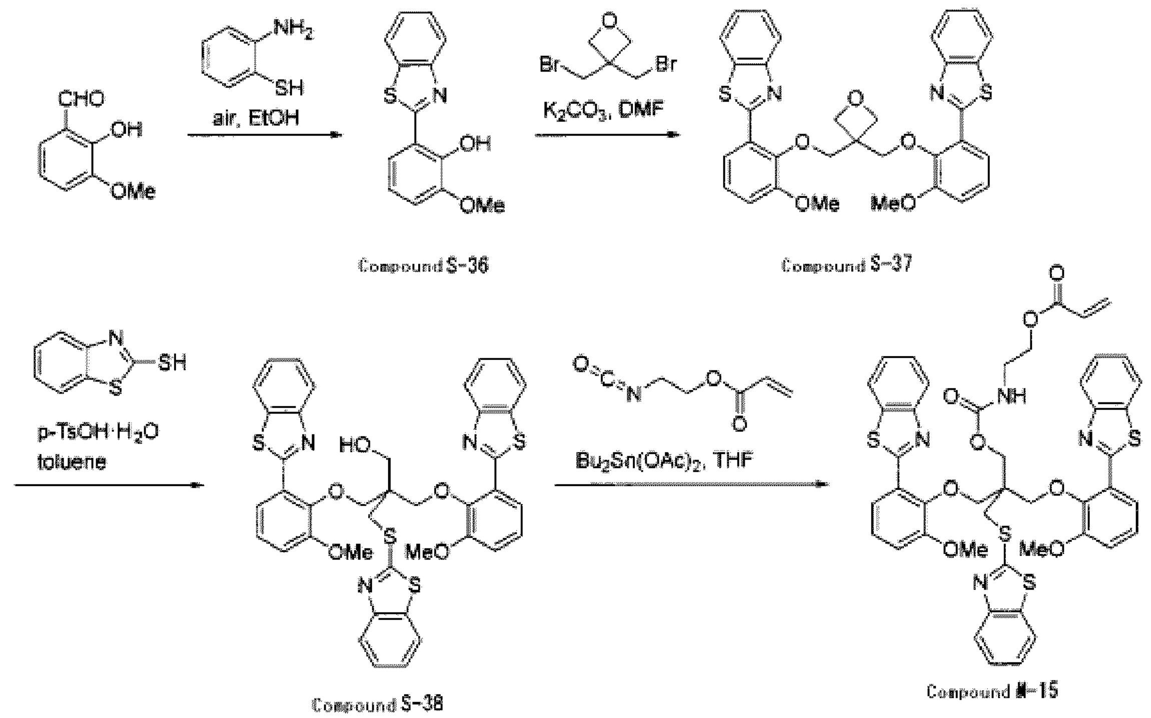


Compound S-36

Compound S-37

Compound S-38

Compound M-15

6.7 g of o-vanillin and 5.0 g of 2-aminobenzenethiol were dissolved in 10 mL of ethanol. The reaction mixture was heated and stirred at reflux for 5 hours. The resulting mixture was cooled to room temperature and stirred in air at room temperature for 48 hours. The reaction mixture was concentrated, and the solid generated was separated by filtration, washed with 2-butanone, and dried under reduced pressure to thereby obtain 5.4 g of a compound S-36 (yield: 53%) .

The NMR measurement data of the compound S-36 is as follows.

$^1H$ NMR (400MHz, $CDCl_3$, $\delta$, ppm) 3.95 (s, 6H), 6.90 (dd, 1H), 6.99 (dd, 1H), 7.32 (dd, 1H), 7.41 (ddd, 1H), 7.51 (ddd, 1H), 7.90 (d, 1H), 8.01 (d, 1H), 12.74 (brs, HO, 1H)

The compound S-36 (4.2 g), 2.0 g of bis(bromomethyl)oxetane, and 2.3 g of potassium carbonate were dissolved in 10 mL of dimethylformamide (DMF). The reaction mixture was heated to 120°C and stirred for 2 hours. The resulting mixture was cooled to room temperature, and ethyl acetate and water were added to the reaction mixture to perform an extraction operation. The organic layer was concentrated, and the crude product obtained was purified using a silica gel column (hexane/ethyl acetate) to thereby obtain 2.9 g of a compound S-37 (yield: 59%).

The NMR measurement data of the compound S-37 is as follows.

$^1H$ NMR (400MHz, $CDCl_3$, $\delta$, ppm) 3.85 (s, 6H), 4.59 (s, 4H), 4.97 (s, 4H), 7.04 (dd, 2H), 7.20 (dd, 2H), 7.34 (ddd, 2H), 7.45 (ddd, 2H), 7.81 (d, 2H), 7.96 (dd, 2H), 8.05 (d, 2H)

The compound S-37 (2.9 g), 2-mercaptobenzothiazole (0.82 g), and toluenesulfonic acid monohydrate (50 mg) were suspended in 10 mL of toluene. The reaction solution was heated to 120°C in a nitrogen atmosphere and stirred at reflux for 3 hours. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 3.5 g of a compound S-38 (yield: 94%).

The NMR measurement data of the compound S-38 is as follows.

$^1H$ NMR (400MHz, $CDCl_3$, $\delta$, ppm) 3.85 (s, 6H), 4.18 (s, 2H), 4.22 (s, 2H), 4.43 (d, 2H), 4.45 (d, 2H), 4.66 (brs, 1H), 7.01 (dd, 2H), 7.19 (dd, 2H), 7.29 (Ar, 3H), 7.40 (Ar, 3H), 7.71 (d, 2H), 7.72 (d, 1H), 7.78 (dd, 2H), 7.80 (d, 1H), 8.05 (d, 2H)

The compound S-38 (3.5 g) was dissolved in 10 mL of tetrahydrofuran, and 38 mg of dibutyltin diacetate was added. 2-Isocyanatoethyl acrylate (0.65 g) was added to the prepared solution, and the mixture was allowed to react at

room temperature. After a lapse of 48 hours, a saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 1.3 g of a compound M-15 (yield: 32%).

The NMR measurement data of the compound M-15 is as follows.
$^1H$ NMR (400MHz, $CDCl_3$, $\delta$, ppm) 3.32 (m, 2H), 3.85 (s, 6H), 4.06 (m, 2H), 4.23 (s, 2H), 4.41 (m, 4H), 4.79 (brt, 1H), 4.48 (s, 2H), 5.77 (d, 1H), 6.02 (dd, 1H), 6.35 (d, 1H), 7.00 (dd, 2H), 7.18 (dd, 2H), 7.30 (Ar, 3H), 7.40 (Ar, 3H), 7.76 (Ar, 4H), 7.86 (dd, 2H), 8.03 (d, 2H)

(Example 16)

A compound M-16 was produced by the following synthesis method.

[Chem. 37]

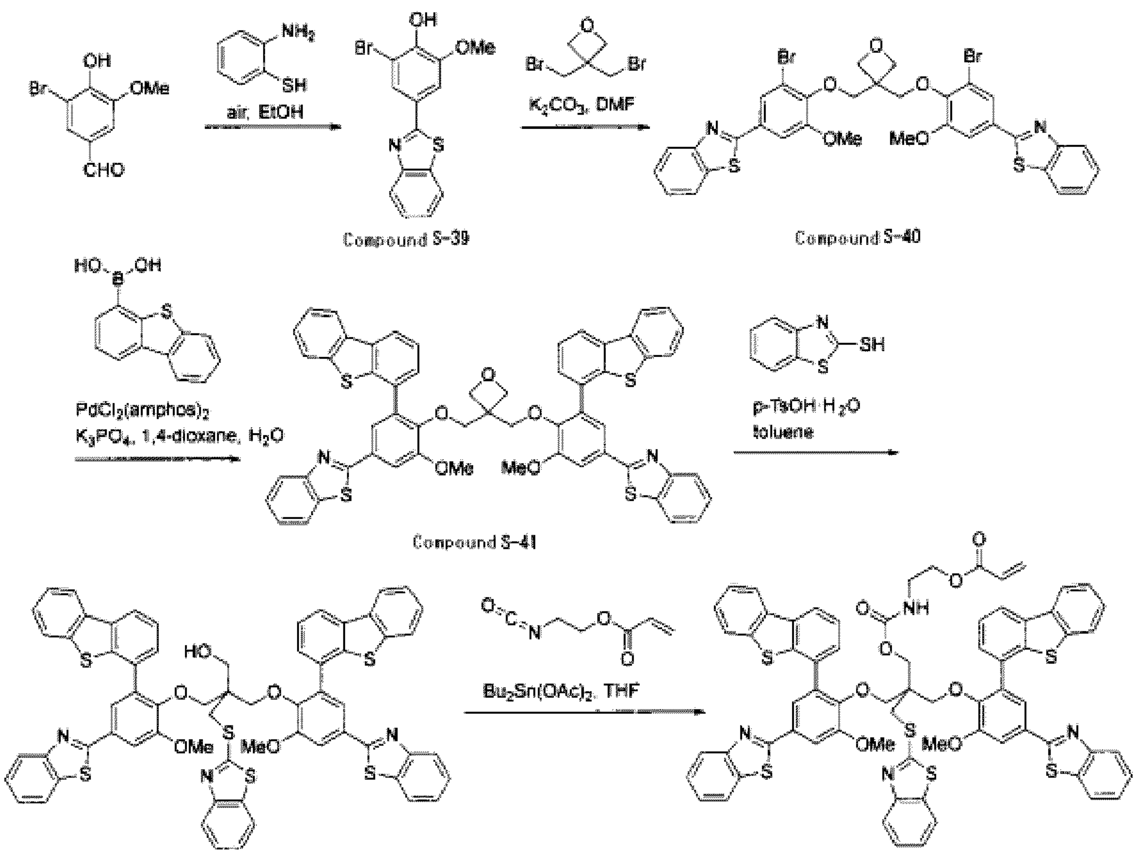

Compound S-39
Compound S-40
Compound S-41
Compound S-42
Compound M-16

3.7 g of 5-bromovanillin and 2.0 g of 2-aminobenzenethiol were dissolved in 10 mL of ethanol. The reaction mixture was heated and stirred at reflux for 5 hours. The mixture was cooled to room temperature and stirred in air at room temperature for 72 hours. The reaction mixture was concentrated, and the solid generated was separated by filtration, washed with 2-butanone, and dried under reduced pressure to thereby obtain 3.5 g of a compound S-39 (yield: 65%).

The NMR measurement data of the compound S-39 is as follows.
$^1H$ NMR (400MHz, $CDCl_3$, $\delta$, ppm) 4.40 (s, 3H), 6.23 (s, OH, 1H), 7.38 (dd, 1H), 7.49 (dd, 1H), 7.65 (d, 1H), 7.80 (d, 1H), 7.89 (d, 1H), 8.04 (d, H)

The compound S-39 (3.3 g), 1.2 g of bis(bromomethyl)oxetane, and 1.4 g of potassium carbonate were dissolved in 10 mL of dimethylformamide (DMF). The reaction mixture was heated to 120°C and stirred for 2 hours. The resulting mixture was cooled to room temperature, and ethyl acetate and water were added to the reaction mixture to perform an extraction operation. The organic layer was concentrated, to thereby obtain 3.7 g of the crude product of a compound S-40.

The NMR measurement data of the compound S-40 is as follows.
$^1$H NMR (400MHz, $CDCl_3$, $\delta$, ppm) 3.99 (s, 6H), 4.57 (s, 4H), 4.86 (s, 4H), 7.39 (dd, 2H), 7.50 (dd, 2H), 7.66 (s, 2H), 7.81 (s, 2H), 7.89 (d, 2H), 8.04 (d, 2H)

The compound S-40 (3.7 g), dibenzothiophene-4-boronic acid (2.5 g), and potassium phosphate (3.2 g) were suspended in 60 mL of dioxane and 10 mL of water, and nitrogen gas was bubbled through the suspension to degas the suspension. 81 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 6 hours. The resulting mixture was cooled to room temperature, extracted with ethyl acetate, and washed with water. The aqueous layer was extracted twice with ethyl acetate. 0.4 g of activated carbon was added to the obtained organic layer, and the mixture was stirred for 30 minutes. The mixture was filtered through celite and concentrated, and the crude product obtained was purified using a silica gel column (hexane/ethyl acetate) to thereby obtain 4.4 g of a compound S-41.

The NMR measurement data of the compound S-41 is as follows.
$^1$H NMR (400MHz, $CDCl_3$, $\delta$, ppm) 3.56 (s, 4H), 3.84 (s, 6H), 3.96 (s, 4H), 7.22 (Ar, 2H), 7.39 (Ar, 8H), 7.51 (Ar, 2H), 7.72 (Ar, 6H), 7.91 (d, 2H), 8.01 (dd, 2H), 8.09 (Ar, 4H)

The compound S-41 (4.7 g), 2-mercaptobenzothiazole (0.90 g), and toluenesulfonic acid monohydrate (100 mg) were suspended in 10 mL of toluene. The reaction solution was heated to 120°C in a nitrogen atmosphere and stirred at reflux for 3 hours. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 5.5 g of a compound S-42.

The NMR measurement data of the compound S-42 is as follows.
$^1$H NMR (400MHz, $CDCl_3$, $\delta$, ppm) 2.96 (s, 2H), 3.22 (d, 2H), 3.46 (s, 4H), 3.53 (brt, OH, 1H), 3.81 (s, 6H), 7.20 (Ar, 2H), 7.24 (dd, 1H), 7.36 (Ar, 9H), 7.51 (ddd, 2H), 7.58 (d, 1H), 7.64 (Ar, 3H), 7.69 (Ar, 4H), 7.90 (d, 2H), 7.97 (dd, 2H), 8.02 (Ar, 2H), 8.09 (d,2H)

The compound S-42 (5.5 g) was dissolved in 10 mL of tetrahydrofuran, and 34 mg of dibutyltin diacetate was added. 2-Isocyanatoethyl acrylate (0.83 g) was added to the prepared solution, and the mixture was allowed to react at room temperature. After a lapse of 48 hours, a saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 2.8 g of a compound M-16 (yield: 45%).

The NMR measurement data of the compound M-16 is as follows.
$^1$H NMR (400MHz, $CDCl_3$, $\delta$, ppm) 3.14 (m, 4H), 3.45 (s, 4H), 3.79 (s, 6H), 3.88 (s, 2H), 4.03 (brt, 2H), 4.40 (t, NH, 1H), 5.82 (d, 1H), 6.09 (dd, 1H), 6.40 (d, 1H), 7.22 (Ar, 3H), 7.37 (Ar, 9H), 7.50 (ddd, 2H), 7.56 (d, 1H), 7.66 (Ar, 5H), 7.72 (Ar, 2H), 7.90 (d, 2H), 7.95 (dd, 2H), 8.02 (Ar, 2H), 8.08 (d, 2H)

(Example 17)

A compound M-17 was produced by the following synthesis method.

[Chem. 38]

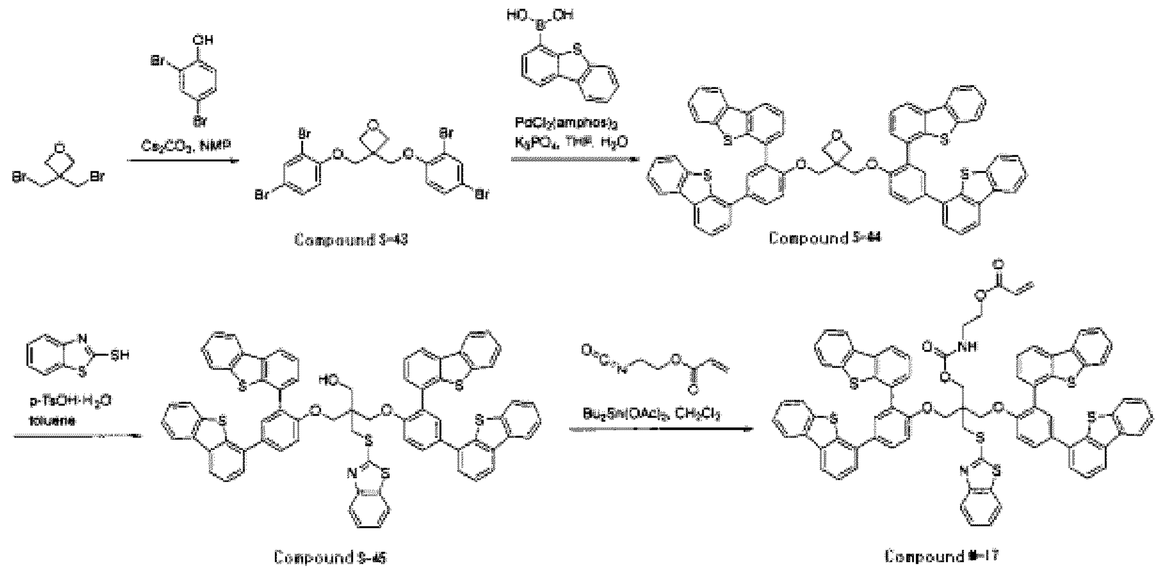


Compound S-43

Compound S-44

Compound S-45

Compound M-17

2,4-Dibromophenol (2.3 g), 1.0 g of bis(bromomethyl)oxetane, and 2.7 g of cesium carbonate were dissolved in 5 mL of 1-methyl-2-pyrrolidone (NMP). The reaction mixture was heated to 130°C and stirred for 2 hours. The resulting reaction mixture was cooled to room temperature, and ethyl acetate and water were added to the reaction mixture to perform an extraction operation. The organic layer was concentrated, and the crude product obtained was purified using a silica gel column (hexane/ethyl acetate) to thereby obtain 1.9 g of a compound S-43 (yield: 79%).

The NMR measurement data of the compound S-43 is as follows.

$^1$H NMR (400MHz, $CDCl_3$, $\delta$, ppm) 4.41 (s, 4H), 4.71 (s, 4H), 6.85 (d, 2H), 7.38 (dd, 2H), 7.65 (d, 2H)

The compound S-43 (8.9 g), dibenzothiophene-4-boronic acid (15 g), and potassium phosphate (19.3 g) were suspended in 150 mL of tetrahydrofuran and 30 mL of water, and nitrogen gas was bubbled through the suspension to degas the suspension. 181 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 6 hours. The resulting mixture was cooled to room temperature, extracted with ethyl acetate, and washed with water. The aqueous layer was extracted twice with ethyl acetate. 0.4 g of activated carbon was added to the obtained organic layer, and the mixture was stirred for 30 minutes. The mixture was filtered through celite and concentrated, and the crude product obtained was purified using a silica gel column (hexane/ethyl acetate) to thereby obtain 15 g of a compound S-44.

The NMR measurement data of the compound S-44 is as follows.

$^1$H NMR (400MHz, $CDCl_3$, $\delta$, ppm) 3.98 (s, 4H), 4.15 (s, 4H), 6.75 (d, 2H), 7.36 (dd, 2H), 7.52 (Ar, 16H), 7.71 (Ar, 2H), 7.85 (Ar, 4H), 8.17 (Ar, 8H)

The crude product of the compound S-44 (5.0 g), 2-mercaptobenzothiazole (0.90 g), and toluenesulfonic acid monohydrate (100 mg) were suspended in 70 mL of toluene. The reaction solution was heated to 120°C in a nitrogen atmosphere and stirred at reflux for 2 hours. The mixture was cooled to room temperature, and 20 mL of tetrahydrofuran was added to obtain a uniform solution. The solution was washed with a 1M aqueous sodium hydroxide solution. Then the resulting solution was washed with 0.1N hydrochloric acid and water, and the organic layer was dried over anhydrous magnesium sulfate and concentrated. The crude product obtained was dissolved in 20 mL of dichloromethane, and the mixture was slowly added dropwise to 150 mL of acetonitrile under stirring. The mixture was further stirred for 2 hours, and the precipitate was separated by filtration. The solid obtained was again dissolved in 80 mL of dichloromethane, and the solution was caused to pass through a silica gel short-path column and concentrated to thereby obtain 3.8 g of a compound S-45 (yield: 73%).

The NMR measurement data of the compound S-45 is as follows.

$^1$H NMR (400MHz, $CDCl_3$, $\delta$, ppm) 3.03 (s, 2H), 3.22 (d, 2H), 3.61 (d, 2H), 3.81 (d, 2H), 4.69 (brt, OH, 1H), 6.67 (d, 2H), 4.15 (s, 4H), 6.75 (d, 2H), 7.28 (Ar, 3H), 7.39 (Ar, 3H), 7.52 (Ar, 13H), 7.67 (Ar, 3H), 7.83 (Ar, 4H), 8.17 (Ar, 8H)

The compound S-45 (3.5 g) was dissolved in 35 mL of dichloromethane, and 24 mg of dibutyltin diacetate was added. 2-Isocyanatoethyl acrylate (0.68 g) was added to the prepared solution, and the mixture was allowed to react at room temperature for 50 hours. After completion of the reaction, the reaction solution was concentrated at 30°C or lower. The crude product obtained by concentration was purified using a silica gel column (hexane/ethyl acetate) to thereby obtain 3.0 g of a compound M-17 (yield: 80%).

The NMR measurement data of the compound M-17 is as follows.

$^1H$ NMR (400MHz, $CDCl_3$, δ, ppm) 3.21 (m, 4H), 3.83 (s, 4H), 3.87 (s, 2H), 4.06 (m, 2H), 4.59 (brt, NH, 2H), 5.81 (d, 1H), 6.08 (dd, 1H), 6.39 (d, 1H), 6.62 (d, 2H), 7.24 (Ar, 1H), 7.32 (Ar, 3H), 7.39 (Ar, 2H), 7.39 (Ar, 3H), 7.48 (Ar, 11H), 7.55 (Ar, 2H), 7.62 (Ar, 2H), 7.69 (Ar, 2H), 7.82 (Ar, 2H), 7.85 (Ar, 2H), 8.17 (Ar, 8H)

(Example 18)

A compound M-18 was produced by the following synthesis method.

[Chem. 39]

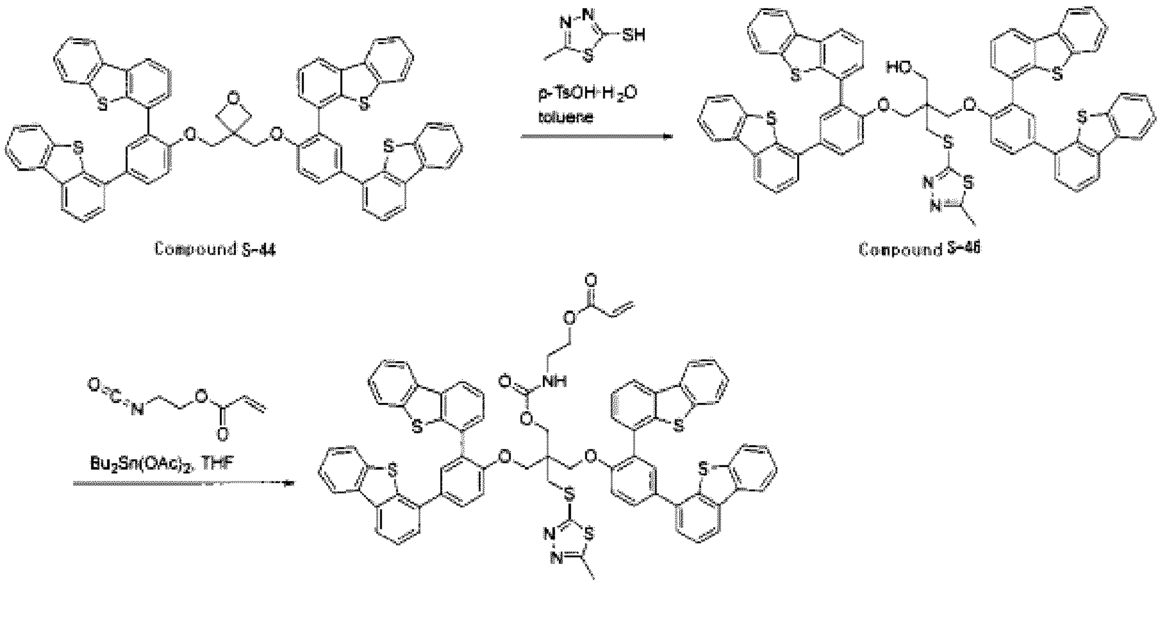


Compound S-44

Compound S-46

Compound M-18

The compound S-44 (20 g) obtained in Example 17, 5-methyl-1,3,4-thiadiazole-2-thiol (3.2 g), and toluenesulfonic acid monohydrate (200 mg) were suspended in 150 mL of toluene. The reaction solution was heated to 120°C in a nitrogen atmosphere and stirred at reflux for 7 hours. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 15.5 g of a compound S-46 (yield: 68%).

The NMR measurement data of the compound S-46 is as follows.

$^1H$ NMR (400MHz, $CDCl_3$, δ, ppm) 2.62 (s, 3H), 2.99 (s, 2H), 3.22 (d, 2H), 3.62 (d, 2H), 3.82 (d, 2H), 3.85 (brt, OH, 1H), 6.76 (d, 2H), 7.30 (dd, 2H), 7.48 (Ar, 16H), 7.68 (Ar, 2H), 7.81 (Ar, 2H), 7.85 (Ar, 2H), 8.18 (Ar, 8H)

The compound S-46 (3.2 g) was dissolved in 10 mL of tetrahydrofuran, and 20 mg of dibutyltin diacetate was added. 2-Isocyanatoethyl acrylate (0.60 g) was added to the prepared solution, and the mixture was allowed to react at room temperature. After a lapse of 48 hours, a saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 1.3 g of a compound M-18 (yield: 36%).

The NMR measurement data of the compound M-18 is as follows.

$^1H$ NMR (400MHz, $CDCl_3$, δ, ppm) 2.56 (s, 3H), 3.11 (s, 2H), 3.30 (m, 2H), 3.75 (m, 4H), 3.82 (s, 2H), 3.84 (s, 2H), 4.68 (brt, NH, 1H), 5.81 (d, 1H), 6.11 (dd, 1H), 6.40 (d, 1H), 6.62 (d, 2H), 7.36 (Ar, 2H), 7.49 (Ar, 16H), 7.73 (Ar, 2H), 7.82 (Ar, 2H), 7.86 (Ar, 2H), 8.18 (Ar, 8H)

(Example 19)

A compound M-19 was produced by the following synthesis method.

[Chem. 40]

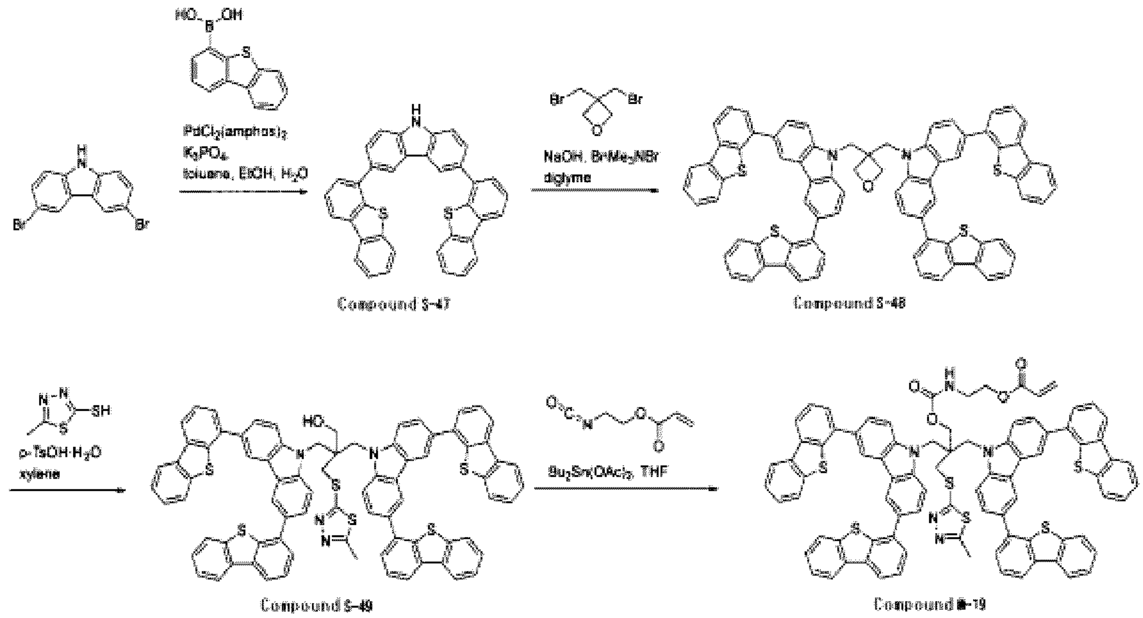


Compound S-47

Compound S-48

Compound S-49

Compound M-19

3,6-Dibromo-9H-carbazole (10 g), dibenzothiophene-4-boronic acid (14.7 g), and potassium phosphate (19.6 g) were suspended in 100 mL of toluene, 100 mL of ethanol, and 50 mL of water, and nitrogen gas was bubbled through the suspension to degas the suspension. 11 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 2 hours. The resulting mixture was cooled to room temperature. The resulting solid was filtered off and washed with ethanol to thereby obtain 15 g of a compound S-47 (yield: 92%).

The NMR measurement data of the compound S-47 is as follows.

$^1$H NMR (400MHz, $CDCl_3$, $\delta$, ppm) 7.46 (Ar, 4H), 7.60 (Ar, 6H), 7.84 (Ar, 4H), 8.18 (dd, 4H), 8.50 (s, 2H)

The compound S-47 (4.4 g), 1.0 g of bis(bromomethyl)oxetane, 0.41 g of sodium hydroxide, and 21 mg of benzyltrimethylammonium bromide were suspended in 10 mL of diethylene glycol dimethyl ether (diglyme). The reaction mixture was heated to 150°C and stirred for 3 hours. The resulting mixture was cooled to room temperature, and the solid generated was separated by filtration, and washed with acetone to thereby obtain 3.3 g of a compound S-48 (yield: 70%).

The NMR measurement data of the compound S-48 is as follows.

$^1$H NMR (400MHz, $CDCl_3$, $\delta$, ppm) 4.89 (m, 8H), 7.44 (Ar, 8H), 7.85 (Ar, 12H), 7.79 (Ar, 4H), 7.92 (Ar, 4H), 8.18 (Ar, 8H), 8.57 (Ar, 4H)

The compound S-48 (1.0 g), 5-methyl-1,3,4-thiadiazole-2-thiol (0.18 g), and toluenesulfonic acid monohydrate (100 mg) were suspended in 10 mL of xylene. The reaction solution was heated to 150°C in a nitrogen atmosphere and stirred at reflux for 2 hours. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 0.92 g of a compound S-49 (yield: 83%).

The NMR measurement data of the compound S-49 is as follows.

$^1$H NMR (400MHz, $CDCl_3$, $\delta$, ppm) 1.53 (s, 3H), 4.01 (m, 4H), 4.81 (d, 2H), 4.93 (d, 2H), 7.40 (Ar, 4H), 7.46 (Ar, 4H), 7.58 (Ar, 8H), 7.74 (Ar, 8H), 7.90 (Ar, 4H), 8.17 (Ar, 8H), 8.53 (Ar, 4H)

The compound S-49 (2.2 g) was dissolved in 10 mL of tetrahydrofuran, and 34 mg of dibutyltin diacetate was added. 2-Isocyanatoethyl acrylate (0.37 g) was added to the prepared solution, and the mixture was allowed to react at room temperature. After a lapse of 72 hours, a saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 1.3 g of a compound M-19 (yield: 53%).

The NMR measurement data of the compound M-19 is as follows.

$^1$H NMR (400MHz, $CDCl_3$, $\delta$, ppm) 1.53 (s, 3H), 3.48 (m, 2H), 3.95 (s, 2H), 4.24 (m, 2H), 4.56 (s, 2H), 4.71 (d, 2H), 4.95

(m, 3H), 5.68 (d, 1H), 6.05 (dd, 1H), 6.33 (d, 1H), 7.43 (Ar, 8H), 7.58 (Ar, 12H), 7.75 (Ar, 4H), 7.90 (Ar, 4H), 8.16 (Ar, 8H), 8.53 (Ar, 4H)

(Example 20)

A compound M-20 was produced by the following synthesis method.

[Chem. 41]

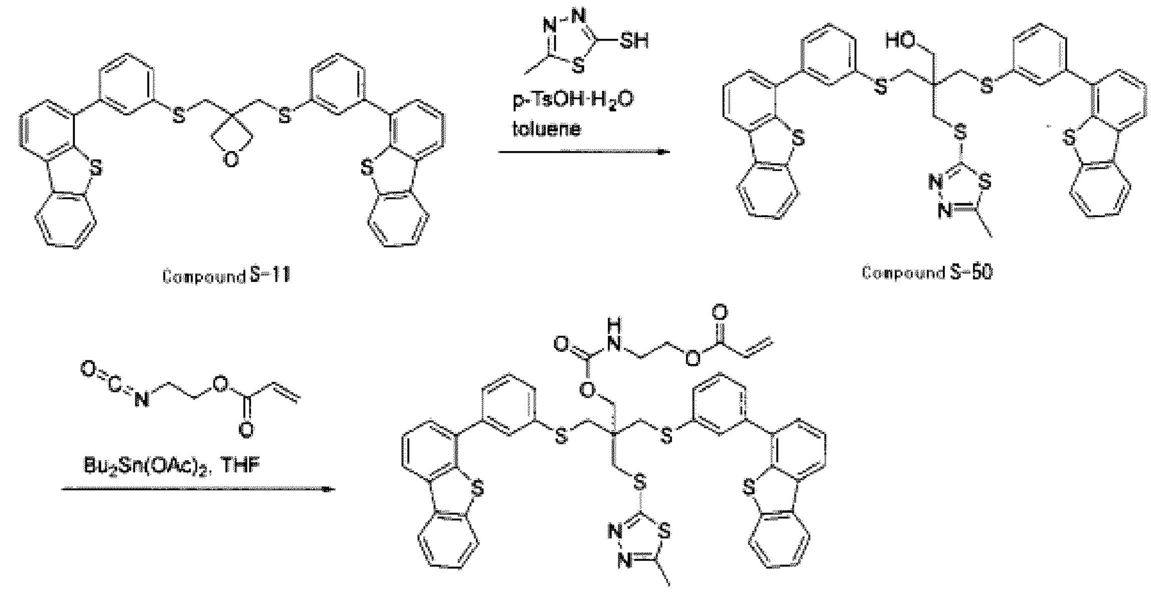


Compound S-11

Compound S-50

Compound M-20

The compound S-11 (3.5 g) obtained in Example 4, 5-methyl-1,3,4-thiadiazole-2-thiol (0.84 g), and toluenesulfonic acid monohydrate (100 mg) were suspended in 10 mL of toluene. The reaction solution was heated to 120°C in a nitrogen atmosphere and stirred at reflux for 2 hours. The mixture was cooled to room temperature, and then an additional of toluenesulfonic acid monohydrate (100 mg) was added, and the mixture was stirred under heat and reflux for 3 hours. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 3.1 g of a compound S-50 (yield: 74%).

The NMR measurement data of the compound S-50 is as follows.

$^1H$ NMR (400MHz, $CDCl_3$, $\delta$, ppm) 2.67 (s, 3H), 3.20 (d, 2H), 3.42 (d, 2H), 3.62 (d, 2H), 3.64 (s, 2H), 4.74 (t, OH, 1H), 7.35 (Ar, 2H), 7.45 (Ar, 12H), 7.76 (Ar, 4H), 8.09 (dd, 2H), 8.15 (dd, 2H)

The compound S-50 (3.1 g) was dissolved in 10 mL of tetrahydrofuran, and 27 mg of dibutyltin diacetate was added. 2-Isocyanatoethyl acrylate (0.82 g) was added to the prepared solution, and the mixture was allowed to react at room temperature. After a lapse of 72 hours, a saturated aqueous sodium hydrogencarbonate solution was added to the reaction solution, and 150 mL of ethyl acetate was added for extraction. The organic layer obtained was dried over magnesium sulfate and concentrated at 30°C or lower. The crude product obtained by concentration was purified using a silica gel column (hexane/ethyl acetate) to thereby obtain 1.3 g of a compound M-20 (yield: 36%).

The NMR measurement data of the compound M-20 is as follows.

$^1H$ NMR (400MHz, $CDCl_3$, $\delta$, ppm) 2.60 (s, 3H), 3.29 (m, 2H), 3.34 (m, 4H), 3.79 (s, 2H), 4.08 (t, 2H), 4.26 (s, 2H), 4.86 (brs, NH, 1H), 5.71 (d, 1H), 5.95 (dd, 1H), 6.31 (d, 1H), 7.33 (Ar, 2H), 7.45 (Ar, 12H), 7.74 (Ar, 2H), 7.79 (Ar, 2H), 8.10 (d, 2H), 8.14 (d, 2H)

(Example 21)

A compound M-21 was produced by the following synthesis method.

[Chem. 42]

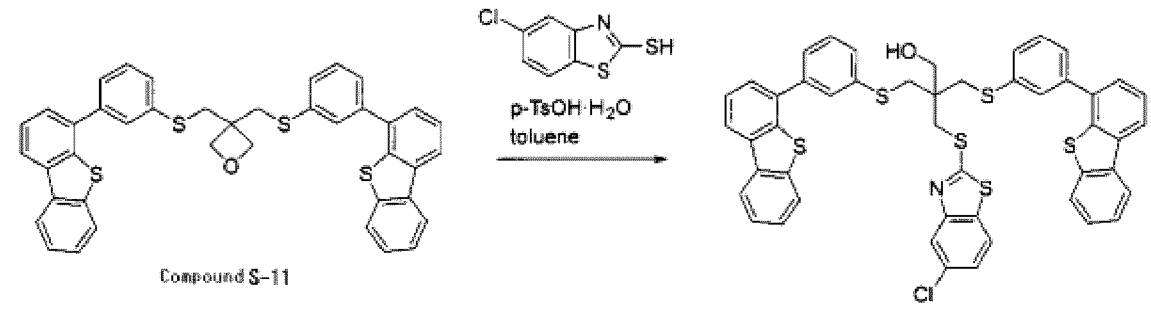


Compound S-11

Compound S-51

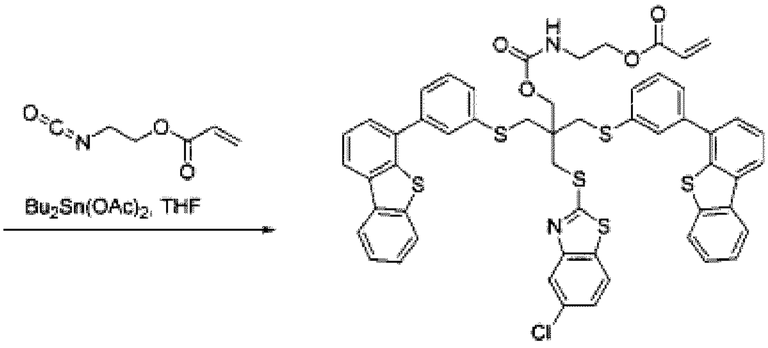


Compound M-21

The compound S-11 (4 g) obtained Example 4, 5-chloro-2-mercaptobenzothiazole (1.81 g), and toluenesulfonic acid monohydrate (100 mg) were suspended in 20 mL of toluene. The reaction solution was heated to 120°C in a nitrogen atmosphere and stirred at reflux for 2 hours. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 4.4 g of a compound S-51 (yield: 68%).

The NMR measurement data of the compound S-51 is as follows.

$^1H$ NMR (400MHz, $CDCl_3$, $\delta$, ppm) 3.23 (d, 2H), 3.43 (d, 2H), 3.63 (d, 2H), 3.68 (s, 2H), 5.05 (t, OH, 1H), 7.43 (Ar, 15H), 7.59 (d, 1H), 7.76 (Ar, 5H), 8.10 (dd, 2H), 8.15 (dd, 2H)

The compound S-51 (4.4 g) was dissolved in 10 mL of tetrahydrofuran, and 30 mg of dibutyltin dilaurate was added. 2-Isocyanatoethyl acrylate (0.93 g) was added to the prepared solution, and the mixture was allowed to react at room temperature. After a lapse of 22 hours, a saturated aqueous sodium hydrogencarbonate solution was added to the reaction solution, and 150 mL of ethyl acetate was added for extraction. The organic layer obtained was dried over magnesium sulfate and concentrated at 30°C or lower. The crude product obtained by concentration was purified using a silica gel column (hexane/ethyl acetate) to thereby obtain 1.9 g of a compound M-21 (yield: 37%).

The NMR measurement data of the compound M-21 is as follows.

$^1H$ NMR (400MHz, $CDCl_3$, $\delta$, ppm) 3.24 (brdd, 2H), 3.38 (s, 4H), 3.82 (s, 2H), 4.04 (t, 2H), 4.29 (s, 2H), 4.79 (brt, NH, 1H), 5.70 (d, 1H), 5.93 (dd, 1H), 6.30 (d, 1H), 7.15 (dd, 1H), 7.33 (dd, 2H), 7.44 (Ar, 13H), 7.75 (Ar, 5H), 8.11 (dd, 2H), 8.15 (Ar, 2H)

(Example 22)

A compound M-22 was produced by the following synthesis method.

[Chem. 43]

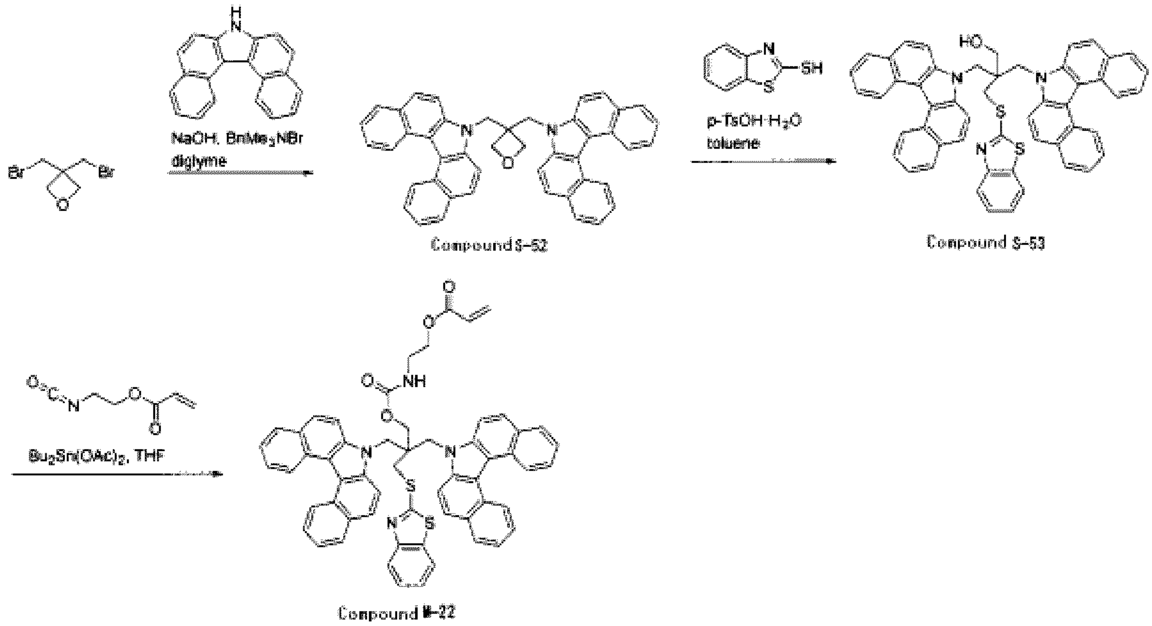


Compound S-52

Compound S-53

Compound M-22

2.6 g of 7H-dibenzo[c,g]carbazole, 1.2 g of bis(bromomethyl)oxetane, 0.66 g of sodium hydroxide, and 20 mg of benzyltrimethylammonium bromide were suspended in 5 mL of diethylene glycol dimethyl ether (diglyme). The reaction mixture was heated to 120°C and stirred for 3 hours. The mixture was cooled to room temperature, and the white solid generated was separated by filtration, washed with methanol, and dried under reduced pressure to thereby obtain 1.6 g of a compound S-52 (yield: 53%).

The NMR measurement data of the compound S-52 is as follows.
$^1$H NMR (400MHz, $CDCl_3$, δ, ppm) 4.60 (s, 4H), 5.00 (s, 4H), 7.53 (Ar, 4H), 7.55 (Ar, 4H), 7.70 (Ar, 4H), 7.85 (d, 4H), 8.02 (d, 4H), 9.22 (d, 4H)

The compound S-52 (1.0 g), 2-mercaptobenzothiazole (0.28 g), and toluenesulfonic acid monohydrate (100 mg) were suspended in 30 mL of toluene. The reaction solution was heated to 100°C in a nitrogen atmosphere and stirred for 5 minutes. The mixture was cooled to room temperature, extracted with ethyl acetate, and washed with a 1M aqueous sodium hydroxide solution. The aqueous layer was re-extracted with ethyl acetate, and the combined organic layer was concentrated. The crude product obtained was purified using a silica gel column (hexane/ethyl acetate) to thereby obtain 0.9 g of a compound S-53 (yield: 71%).

The NMR measurement data of the compound S-53 is as follows.
$^1$H NMR (400MHz, $CDCl_3$, δ, ppm) 2.35 (s, 2H), 3.79 (s, 2H), 4.70 (d, 2H), 5.08 (d, 2H), 5.09 (brs, OH, 1H), 7.23 (Ar, 1H), 7.35 (t, 1H), 7.51 (Ar, 5H), 7.69 (Ar, 9H), 7.80 (d, 4H), 7.98 (d, 4H), 9.20 (d, 4H)

The compound S-53 (1.7 g) was dissolved in 30 mL of tetrahydrofuran, and 55 mg of dibutyltin diacetate was added. 2-Isocyanatoethyl acrylate (0.5 g) was added to the prepared solution, and the mixture was allowed to react at room temperature. After a lapse of 48 hours, a saturated aqueous sodium hydrogencarbonate solution was added to the reaction solution, and 50 mL of chloroform was added for extraction. The organic layer obtained was dried over magnesium sulfate and concentrated at 30°C or lower. The crude product obtained by concentration was purified using a silica gel column (hexane/ethyl acetate) to thereby obtain 1.1 g of a compound M-22 (yield: 55%).

The NMR measurement data of the compound M-22 is as follows.
$^1$H NMR (400MHz, $CDCl_3$, δ, ppm) 2.93 (m, 2H), 3.78 (s, 2H), 3.82 (m, 2H), 4.26 (s, 2H), 4.30 (brt, OH, 1H), 4.74 (s, 4H), 5.70 (d, 1H), 5.96 (dd, 1H), 6.29 (d, 1H), 7.19 (t,

1H), 7.29 (t, 1H), 7.47 (Ar, 8H), 7.60 (Ar, 6H), 7.69 (Ar, 4H), 7.90 (d, 4H), 9.11 (d, 4H)

(Example 23)

A compound M-23 was produced by the following synthesis method.

[Chem. 44]

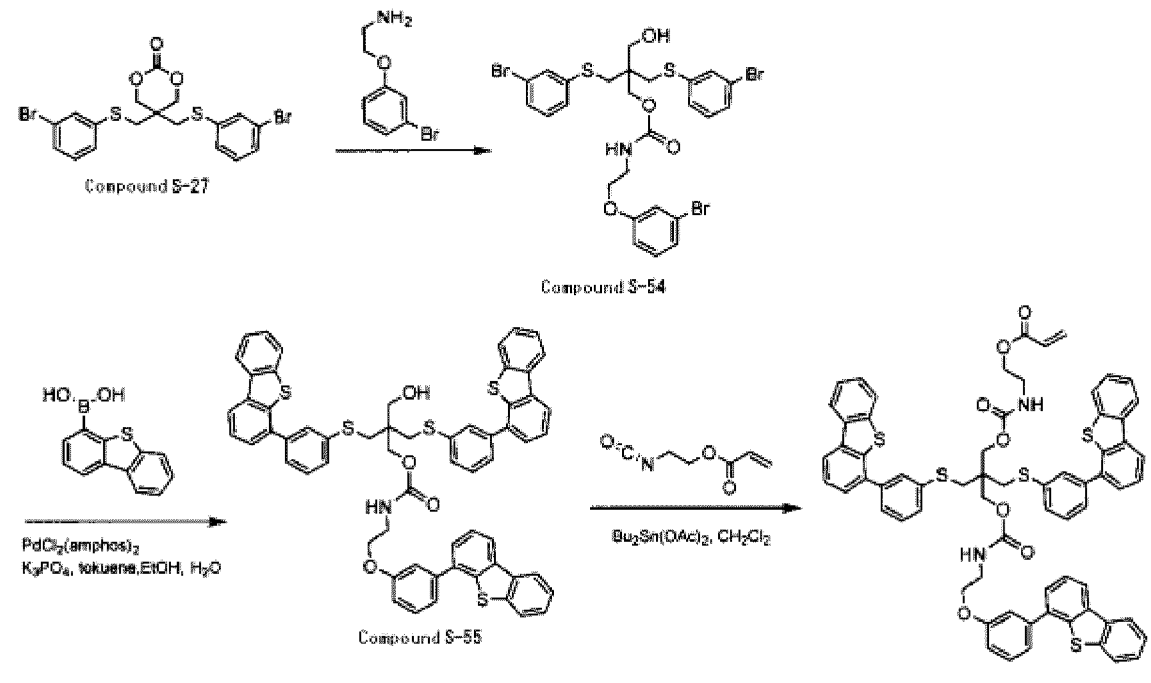


Compound S-27

Compound S-54

Compound S-55

Compound M-23

The compound S-27 (2.0 g) obtained in Example 11 was dissolved in 10 mL of dichloromethane in a nitrogen atmosphere, and 1.2 g of 2-(3-bromophenoxy)ethylamine was added. The mixture was allowed to react at room temperature for 3 hours. 0.3 g of 2-(3-bromophenoxy)ethylamine was further added, and the mixture was allowed to react under reflux conditions for 1 hour. The solution obtained was washed with water and dried over anhydrous magnesium sulfate. The resulting solution was concentrated, and the crude product obtained was purified using a silica gel column (dichloromethane, ethyl acetate) to thereby obtain 2.5 g of a compound S-54 (yield: 84%).

The NMR measurement data of the compound S-54 is as follows.

$^1$H NMR (400MHz, $CDCl_3$, $\delta$, ppm) 2.90 (t, OH, 1H), 3.01-3.15 (m, CH2, 4H), 3.50 (d, CH2, 2H), 3.45+3.58 (brs+q, CH2, 2H), 3.95+4.01 (brs+t, CH2, 2H), 4.14+4.23 (s+brs, CH2, 2H), 4.97+5.18 (brs+brt, NH, 1H), 6.82 (Ar, 1H), 7.00-7.18 (Ar, 5H), 7.27 (Ar, 4H), 7.50 (Ar, 2H)

The compound S-54 (2.5 g), dibenzothiophene-4-boronic acid (2.2 g), and potassium phosphate (2.2 g) were suspended in 16 mL of toluene, 16 mL of ethanol, and 8 mL of water, and nitrogen gas was bubbled through the suspension to degas the suspension. 62 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 2 hours. The resulting solution was cooled to room temperature, extracted with ethyl acetate, and washed with a 1N aqueous sodium hydroxide solution. The solution was further washed with water and brine, and the organic layer was dried over anhydrous magnesium sulfate. The resulting solution was concentrated, and the crude product obtained was purified using a silica gel column (dichloromethane/ethyl acetate) to thereby obtain 2.5 g of a compound S-55 (yield: 75%).

The NMR measurement data of the compound S-55 is as follows.

$^1$H NMR (400MHz, $CDCl_3$, $\delta$, ppm) 2.86+3.01 (t+brs, OH, 1H), 3.23 (dd, CH2, 4H), 3.28+3.49 (brs+q, CH2, 2H), 3.60 (d, CH2, 2H), 3.79+3.98 (brs+t, CH2, 2H), 4.26+4.32 (s+brs, CH2, 2H), 4.92+5.09 (brs+t, NH, 1H), 6.75+6.86 (Ar, 1H), 7.09-7.18 (Ar, 1H), 7.28-7.56 (Ar, 20H), 7.72-7.83 (Ar, 5H), 8.04-8.21 (Ar, 6H)

The compound S-55 (2.5 g) was dissolved in 24 mL of dichloromethane, and 19 mg of dibutyltin diacetate was added. 0.51 g of 2-isocyanatoethyl acrylate was added to the prepared solution, and the mixture was allowed to react at room temperature for about 150 hours. After completion of the reaction, the product was purified using a silica gel column (dichloromethane/ethyl acetate), and the fractions obtained were concentrated at 30°C or lower to a total weight of 13 g. The resulting solution was added dropwise to 160 mL of ice-cooled methanol, and the mixture was stirred in this state for 2 hours. The precipitate was separated by filtration, washed with methanol, and dried to thereby obtain 1.9 g of a compound M-23 (yield: 71%).

The NMR measurement data of the compound M-23 is as follows.

$^1$H NMR (400MHz, $CDCl_3$, δ, ppm), 3.23-3.34 (br, CH2, 6H), 3.44 (q, CH2, 2H), 3.85+3.93 (brs+t, CH2, 2H), 4.06 (t, CH2, 2H), 4.24 (brt, CH2, 4H), 4.80 (t, NH, 1H), 4.63+5.02 (brs+t, NH, 1H), 5.69 (d, 1H), 5.93 (dd, 1H), 6.29 (dd, 1H), 6.78 (Ar, 1H), 7.10 (Ar, 1H), 7.26-7.55 (Ar, 20H), 7.73-7.85 (Ar, 5H), 8.04-8.20 (Ar, 6H)

(Comparative Example 1)

A compound M-24 was produced by the following synthesis method.

[Chem. 45]

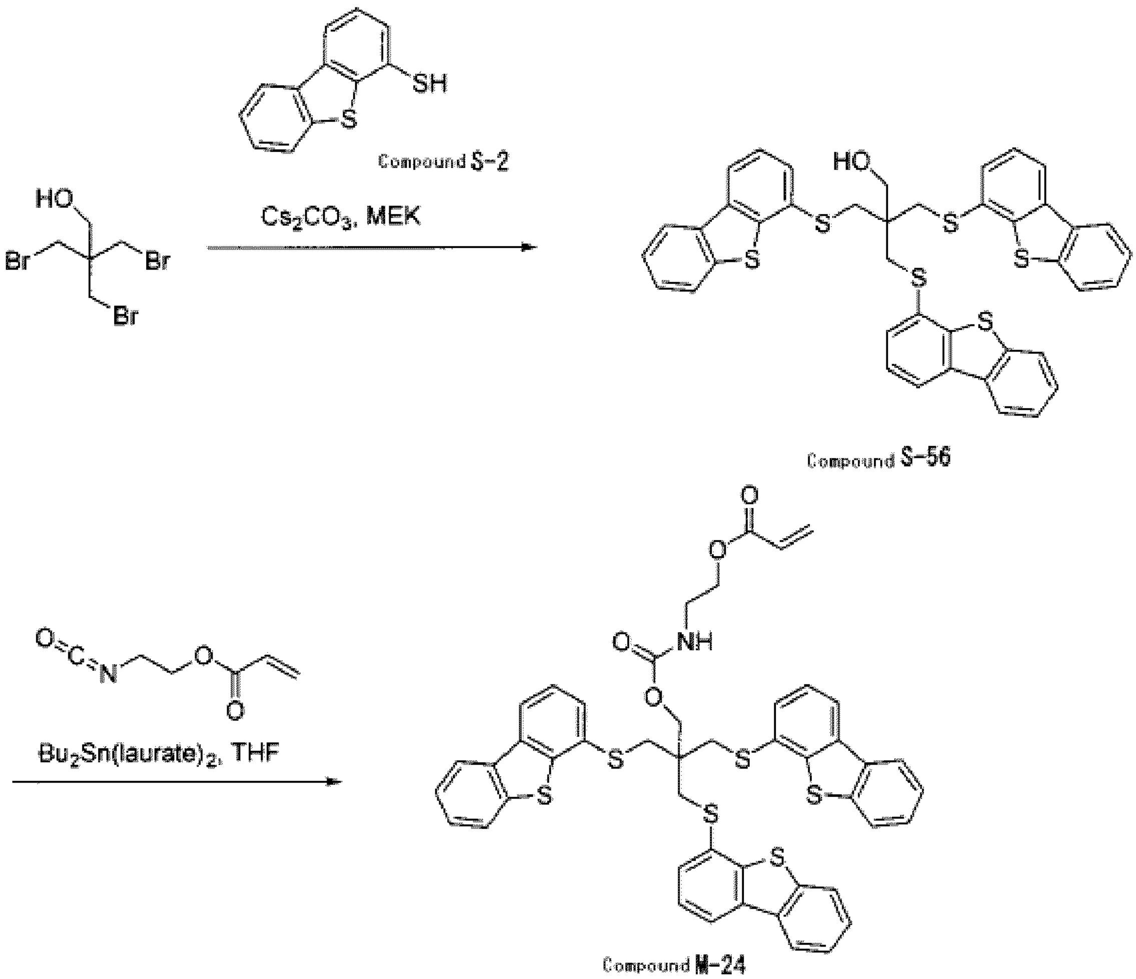


20 g of the compound S-2 obtained in Synthesis Example 1 and 50 g of cesium carbonate were dissolved in 200 mL of methyl ethyl ketone (MEK). 9 g of pentaerythritoltribromide was added to the prepared solution, and the mixture was heated to 90°C and stirred for 5 hours while the progress of the reaction was checked by LC analysis. Water was added to the reaction solution, and then 100 mL of ethyl acetate was added to extract the organic layer. The organic layer obtained was extracted twice with 50 mL of water, and the resulting aqueous layer was back-extracted twice with 100 mL of ethyl acetate. The organic layer obtained was dried over mirabilite and concentrated. The crude product obtained by concentration was purified using a silica gel column (hexane/ethyl acetate) to thereby obtain 10 g of a compound S-56 (yield: 49%).

The NMR measurement data of the compound S-56 is as follows.

$^1$H NMR (400MHz, $CDCl_3$, δ, ppm) 3.38 (s, 6H), 3.75 (d, 2H), 7.22 (dd, 3H), 7.43 (Ar, 9H), 7.77 (Ar, 3H), 7.86 (dd, 3H), 8.02 (Ar, 3H)

The compound S-56 (11 g) was dissolved in 55 mL of tetrahydrofuran (THF), and 190 mg of dibutyltin dilaurate was added. 2.55 g of 2-isocyanatoethyl acrylate (Karenz AOI manufactured by Showa Denko Co.,Ltd.) was added to the prepared solution, and the mixture was allowed to react at room temperature. After a lapse of 24 hours, an additional

0.4 g of 2-isocyanatoethyl acrylate was added, and the mixture was allowed to further react for 24 hours. 100 mL of ethyl acetate was added to the reaction solution, and the solution was concentrated to about 50 mL. Insoluble matter was removed, and the mixture was concentrated. The crude product obtained was purified using a silica gel column (hexane/ethyl acetate) to thereby obtain 7.6 g of a compound M-24 (yield: 58%).

The NMR measurement data of the compound M-24 is as follows.

$^1$H NMR (400MHz, $CDCl_3$, $\delta$, ppm) 3.19 (dt, 2H), 3.36 (s, 6H), 4.02 (m, 2H), 4.21 (s, 2H), 4.42 (m, 2H), 5.79 (dd, 1H), 6.03 (dd, 1H), 6.37 (d, 1H), 7.19 (Ar, 3H), 7.38 (Ar, 3H), 7.43 (Ar, 6H), 7.76 (Ar, 3H), 7.84 (Ar, 3H), 8.01 (Ar, 3H)

A holographic recording medium was produced and evaluated in the same manner as in Example 1, except that compound M-24 was used as the polymerizable monomer. The results are shown in Table 1 below.

(Comparative Example 2)

A compound M-25 was produced by the following synthesis method.

[Chem. 46]

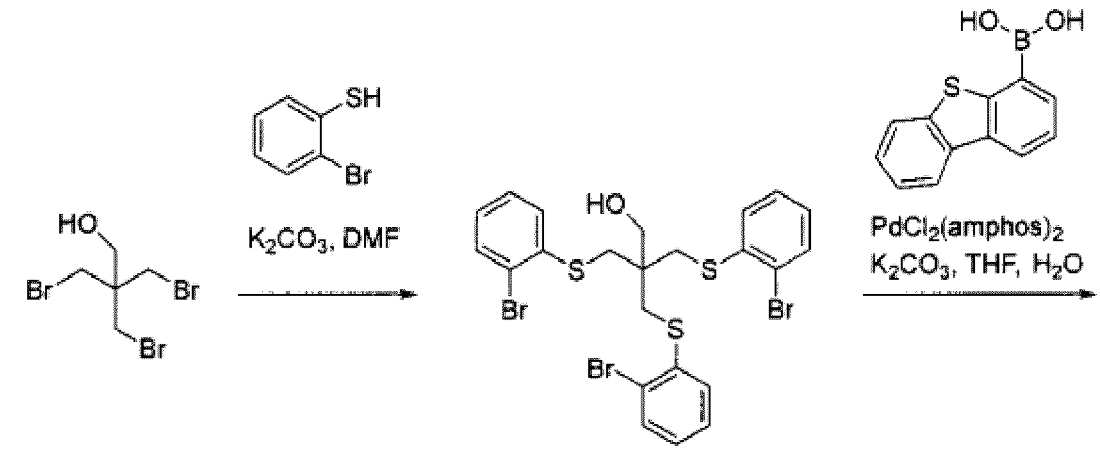


Compound S-57

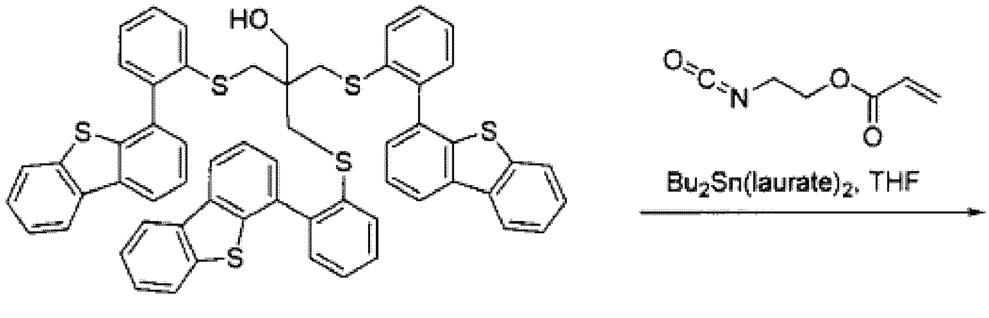


Compound S-58

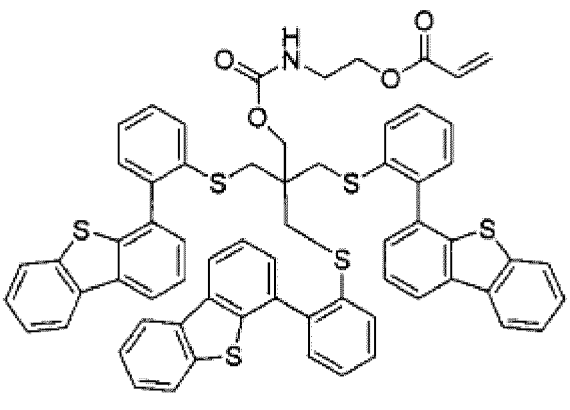

Compound M-25

13.60 g of 2-bromobenzenethiol, 7.08 g of pentaerythritoltribromide, and 9.04 g of potassium carbonate were suspended in 21 mL of N,N-dimethylformamide (DMF). The reaction mixture was heated to 100°C and stirred for 4 hours while the progress of the reaction was checked by LC analysis. The mixture was cooled to room temperature, extracted with ethyl acetate, and washed with water. The aqueous layer was back-extracted twice with ethyl acetate. The organic layer obtained was dried over mirabilite and concentrated. The crude product obtained by concentration was purified using a silica gel column (hexane/ethyl acetate) to thereby obtain 13.2 g of a compound S-57 (yield: 93%) .

The NMR measurement data of the compound S-57 is as follows.

$^1H$ NMR (400MHz, $CDCl_3$, $\delta$, ppm) 3.23 (s, 6H), 3.79 (d, 2H), 6.98 (Ar, 3H), 7.19 (Ar, 3H), 7.33 (Ar, 3H), 7.47 (Ar, 3H)

The compound S-57 (2.0 g), 3.5 g of dibenzothiophene-4-boronic acid, and 2.5 g of potassium phosphate were suspended in 20 mL of THF and 2.0 mL of water, and nitrogen gas was bubbled through the suspension to degas the suspension. 30 mg of dichlorobis[di-t-butyl(p-dimethylaminophenyl)phosphino]palladium (II) was added to the reaction

solution, and nitrogen gas was further bubbled through the mixture for 10 minutes. The reaction solution was heated in a nitrogen atmosphere and stirred at reflux for 6 hours. The resulting mixture was cooled to room temperature, extracted with ethyl acetate, and washed with water. The aqueous layer was extracted twice with ethyl acetate. 0.4 g of activated carbon was added to the obtained organic layer, and the mixture was stirred for 30 minutes. The mixture was filtered through celite and concentrated, and the crude product obtained was purified using a silica gel column (hexane/ethyl acetate) to thereby obtain 2.9 g of a compound S-58 (yield: 98%).

The NMR measurement data of the compound S-58 is as follows.

$^1$H NMR (400MHz, $CDCl_3$, $\delta$, ppm) 2.62 (s, 6H), 3.00 (d, 2H), 7.16 (Ar, 12H), 7.41 (Ar, 12H), 7.70 (Ar, 3H), 8.08 (Ar, 3H), 8.15 (Ar, 3H)

The compound S-58 (2.9 g) was dissolved in 15 mL of tetrahydrofuran (THF), and 40 mg of dibutyltin dilaurate was added. 510 mg of 2-isocyanatoethyl acrylate (Karenz AOI manufactured by Showa Denko Co.,Ltd.) was added to the prepared solution, and the mixture was allowed to react at room temperature. After a lapse of 24 hours, an additional 500 mg of 2-isocyanatoethyl acrylate was added, and the mixture was allowed to further react for 24 hours. After water was added to the reaction solution, 50 mL of ethyl acetate was added and the organic layer was extracted. The organic layer obtained was dried over magnesium sulfate and concentrated at 30°C or lower. The crude product obtained by concentration was purified using a silica gel column (hexane/ethyl acetate) to thereby obtain 1.5 g of a compound M-25 (yield: 45%).

The NMR measurement data of the compound M-25 is as follows.

$^1$H NMR (400MHz, $CDCl_3$, $\delta$, ppm) 2.53 (s, 6H), 3.07 (m, 2H), 3.53 (s, 2H), 3.99 (t, 2H), 4.14 (dd, 1H), 5.81 (d, 1H), 6.10 (dd, 1H), 6.40 (d, 1H), 7.11 (Ar, 12H), 7.24 (Ar, 3H), 7.36 (Ar, 3H), 7.43 (Ar, 6H), 7.71 (Ar, 3H), 8.08 (Ar, 3H). 8.16 (Ar, 3H)

A holographic recording medium was produced and evaluated in the same manner as in Example 1, except that compound M-25 was used as the polymerizable monomer. The results are shown in Table 1 below.

(Comparative Example 3)

A compound M-26 was produced by the following synthesis method.

[Chem. 47]

Compound S-57

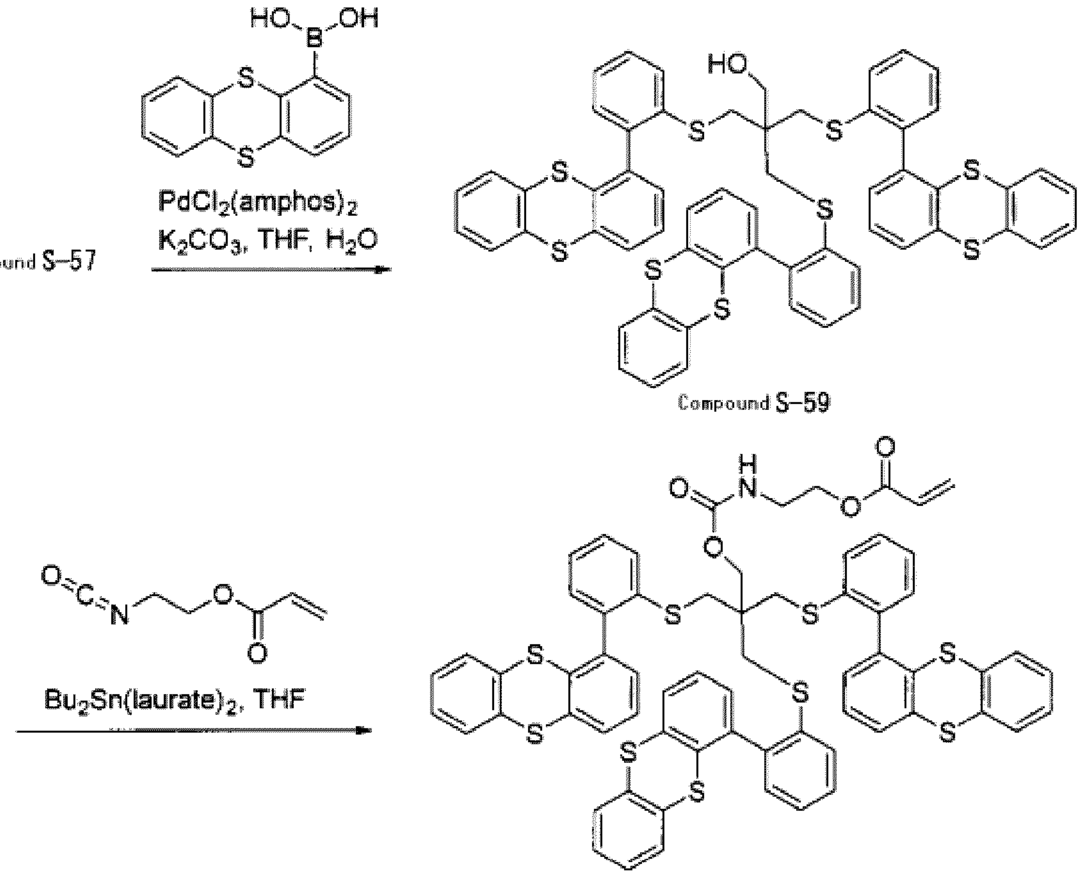


Compound S-59

Compound M-26

The compound S-57 (2 g) obtained in Comparative Example 2, 2.7 g of thiansulene-1-boronic acid, and 2.5 g of potassium carbonate were suspended in 20 mL of THF and 10 mL of water, and nitrogen gas was bubbled through the suspension to degas the suspension. 30 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 6 hours. The resulting mixture was cooled to room temperature, extracted with ethyl acetate, and washed with water. The aqueous layer was extracted twice with ethyl acetate. 0.4 g of activated carbon was added to the obtained organic layer, and the mixture was stirred for 30 minutes. The mixture was filtered through celite and concentrated, and the crude product obtained was purified using a silica gel column (hexane/ethyl acetate) to thereby obtain 2.2 g of a compound S-59 (yield: 68%).

The NMR measurement data of the compound S-59 is as follows.

$^1H$ NMR (400MHz, $CDCl_3$, $\delta$, ppm) 2.57 (m, 6H), 2.98 (d, 2H), 6.99 (Ar, 6H), 7.07 (Ar, 3H), 7.14 (Ar, 12H), 7.22 (Ar, 6H), 7.44 (Ar, 6H)

The compound S-59 (1.1 g) was dissolved in 7.5 mL of tetrahydrofuran (THF), and 10 mg of dibutyltin dilaurate was added. 290 mg of 2-isocyanatoethyl acrylate (Karenz AOI manufactured by Showa Denko Co.,Ltd.) was added to the prepared solution, and the mixture was allowed to react at room temperature. After a lapse of 24 hours, an additional 300 mg of 2-isocyanatoethyl acrylate was added, and the mixture was allowed to further react for 24 hours. Water was added to the reaction solution, and then 50 mL of ethyl acetate was added to extract the organic layer. The organic layer obtained was extracted twice with 30 mL of water, and the resulting aqueous layer was back-extracted twice with 50 mL of ethyl acetate. The organic layer obtained was dried over magnesium sulfate and concentrated at 30°C or lower. The crude product obtained by concentration was purified using a silica gel column (hexane/ethyl acetate) to thereby obtain 600 mg of a compound M-26 (yield: 50%).

The NMR measurement data of the compound M-26 is as follows.

$^1H$ NMR (400MHz, $CDCl_3$, $\delta$, ppm) 2.49 (m, 6H), 3.23 (m, 2H), 3.53 (s, 2H), 4.08 (t, 2H), 4.52 (dd, 1H), 5.82 (d, 1H), 6.08 (dd, 1H), 6.40 (d, 1H), 7.10 (Ar, 27H), 7.44 (Ar, 6H)

A holographic recording medium was produced and evaluated in the same manner as in Example 1, except that compound M-26 was used as the polymerizable monomer. The results are shown in Table 1 below.

(Comparative Example 4)

A compound M-27 was produced by the following synthesis method.

[Chem. 48]

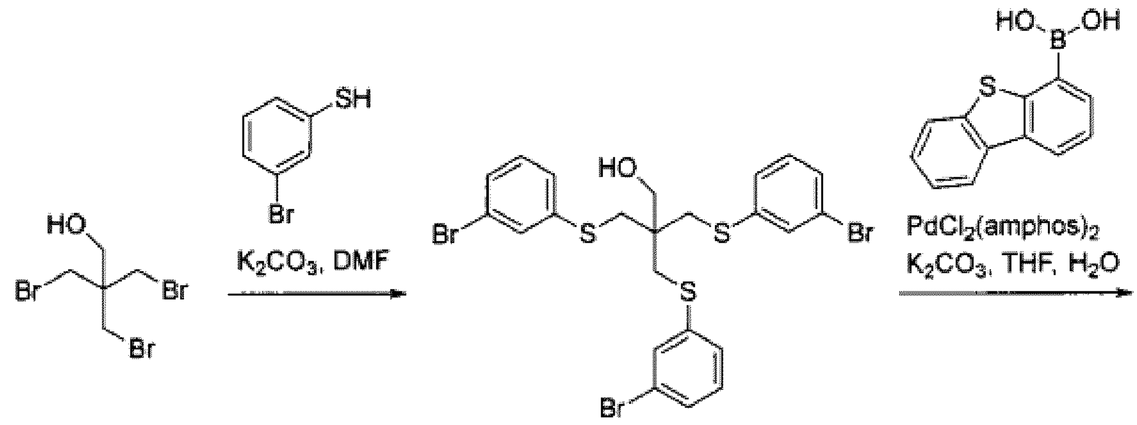


Compound S-60

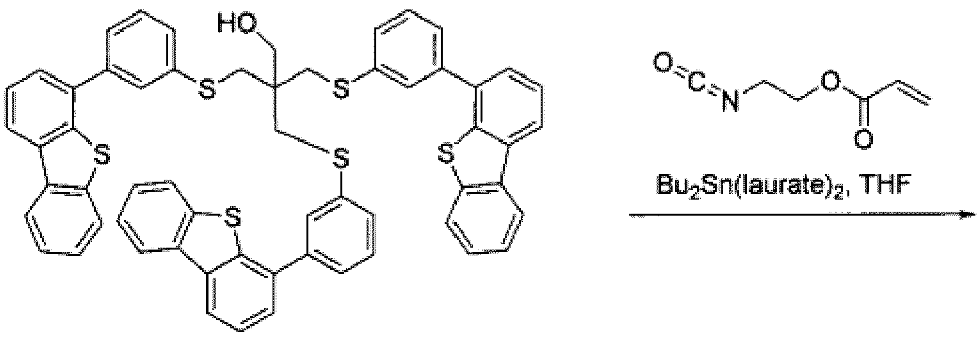


Compound S-61

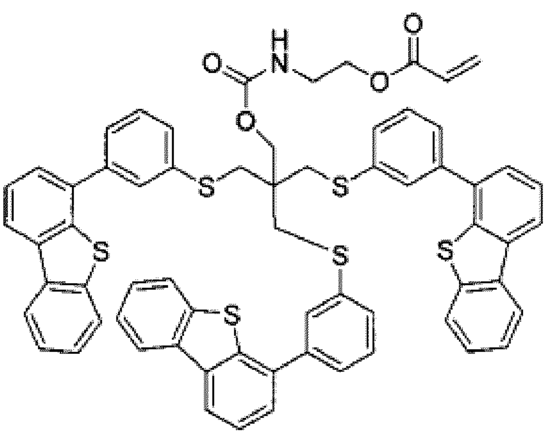

Compound M-27

4.80 g of 3-bromobenzenethiol, 2.50 g of pentaerythritoltribromide, and 3.19 g of potassium carbonate were

suspended in 13 mL of N,N-dimethylformamide (DMF). The reaction mixture was heated to 100°C and stirred for 4 hours while the progress of the reaction was checked by LC analysis. The mixture was cooled to room temperature, extracted with ethyl acetate, and washed with water. The aqueous layer was back-extracted twice with ethyl acetate. The organic layer obtained was dried over mirabilite and concentrated. The crude product obtained by concentration was purified using a silica gel column (hexane/ethyl acetate) to thereby obtain 5.0 g of a compound S-60 (yield: 100%).

The NMR measurement data of the compound S-60 is as follows.
$^1H$ NMR (400MHz, $CDCl_3$, $\delta$, ppm) 3.15 (s, 6H), 3.66 (d, 2H), 7.09 (Ar, 3H), 7.25 (Ar, 6H), 7.45 (Ar, 3H)

The compound S-60 (2.3 g), 3.2 g of dibenzothiophene-4-boronic acid, and 2.9 g of potassium carbonate were suspended in 23 mL of THF and 3 mL of water, and nitrogen gas was bubbled through the suspension to degas the suspension. 70 mg of dichlorobis[di-t-butyl(p-dimethylaminophenyl)phosphino]palladium (II) was added to the reaction solution, and nitrogen gas was further bubbled through the mixture for 10 minutes. The reaction solution was heated in a nitrogen atmosphere and stirred at reflux for 6 hours. The resulting mixture was cooled to room temperature, extracted with ethyl acetate, and washed with water. The aqueous layer was extracted twice with ethyl acetate. 0.5 g of activated carbon was added to the obtained organic layer, and the mixture was stirred for 30 minutes. The mixture was filtered through celite and concentrated, and the crude product obtained was purified using a silica gel column (hexane/ethyl acetate) to thereby obtain 2.9 g of a compound S-61 (yield: 87%).

The NMR measurement data of the compound S-61 is as follows.
$^1H$ NMR (400MHz, $CDCl_3$, $\delta$, ppm) 3.37 (s, 6H), 3.82 (d, 2H), 7.28 (Ar, 3H), 7.34 (Ar, 3H), 7.41 (Ar, 15H), 7.71 (Ar, 6H), 8.05 (Ar, 3H), 8.11 (Ar, 3H)

The compound S-61 (2.9 g) was dissolved in 14.5 mL of tetrahydrofuran (THF), and 40 mg of dibutyltin dilaurate was added. 850 mg of 2-isocyanatoethyl acrylate (Karenz AOI manufactured by Showa Denko Co.,Ltd.) was added to the prepared solution, and the mixture was allowed to react at room temperature. After a lapse of 24 hours, an additional 400 mg of 2-isocyanatoethyl acrylate was added, and the mixture was allowed to further react for 24 hours. Water was added to the reaction solution, and then 100 mL of ethyl acetate was added to extract the organic layer. The organic layer obtained was extracted twice with 50 mL of water, and the resulting aqueous layer was back-extracted twice with 50 mL of ethyl acetate. The organic layer obtained was dried over magnesium sulfate and concentrated at 30°C or lower. The crude product obtained by concentration was purified using a silica gel column (hexane/ethyl acetate) to thereby obtain 1.1 g of a compound M-27 (yield: 33%).

The NMR measurement data of the compound M-27 is as follows.
$^1H$ NMR (400MHz, $CDCl_3$, $\delta$, ppm) 3.22 (m, 2H), 3.37 (s, 6H), 3.99 (t, 2H), 4.30 (s, 2H), 4.70 (dd, 1H), 5.62 (d, 1H), 5.82 (dd, 1H), 6.23 (d, 1H), 7.27 (Ar, 3H), 7.38 (Ar, 18H), 7.71 (Ar, 6H), 8.05 (Ar, 3H), 8.11 (Ar, 3H)

A holographic recording medium was produced and evaluated in the same manner as in Example 1, except that compound M-27 was used as the polymerizable monomer. The results are shown in Table 1 below.

[Table 1]

| | Polymerizable monomer | Number of times of multiple recording | Total $\Delta n$ | Transmittance before recording % | Transmittance after recording % | Haze % | Haze % / total $\Delta n$ |
|---|---|---|---|---|---|---|---|
| Example 1 | M-1 | 151 | 0.0172 | 66 | 86 | 0.05 | 2.91 |
| Example 2 | M-2 | 151 | 0.0199 | 66 | 85 | 0.04 | 2.01 |
| Example 3 | M-3 | 151 | 0.0216 | 66 | 85 | 0.06 | 2.78 |
| Example 4 | M-4 | 151 | 0.0213 | 67 | 84 | 0.05 | 2.35 |
| Example 5 | M-5 | 151 | 0.0217 | 67 | 85 | 0.05 | 2.30 |
| Example 6 | M-6 | 151 | 0.0129 | 67 | 84 | 0.04 | 3.10 |
| Example 7 | M-7 | 151 | 0.0157 | 67 | 84 | 0.04 | 2.55 |
| Example 8 | M-8 | 151 | 0.0205 | 66 | 86 | 0.07 | 3.41 |
| Example 9 | M-9 | 151 | 0.0208 | 67 | 83 | 0.07 | 3.37 |
| Example 10 | M-10 | 151 | 0.0214 | 66 | 84 | 0.07 | 3.27 |
| Example 11 | M-11 | 151 | 0.0138 | 67 | 88 | 0.04 | 2.90 |
| Comparative Example 1 | M-24 | 151 | 0.0190 | 67 | 85 | 0.07 | 3.68 |

(continued)

| | Polymerizable monomer | Number of times of multiple recording | Total Δn | Transmittance before recording % | Transmittance after recording % | Haze % | Haze % / total Δn |
|---|---|---|---|---|---|---|---|
| Comparative Example 2 | M-25 | 151 | 0.0194 | 69 | 87 | 0.07 | 3.61 |
| Comparative Example 3 | M-26 | 151 | 0.0220 | 67 | 85 | 0.09 | 4.09 |
| Comparative Example 4 | M-27 | 151 | 0.0214 | 66 | 82 | 0.09 | 4.21 |

To produce each of the holographic recording mediums, the molar concentrations of the polymerizable monomer, the photopolymerization initiator, and the additives were fixed to their specific values, and only the type of polymerizable monomer was changed to produce and evaluate the holographic recording medium (Table 1).

As shown in Table 1, in Comparative Examples 1 to 4 in which the polymerizable monomer used had three high-refractive index moieties having the same structure, the ratio haze % / total Δn was 3.6 or more. However, in Examples 1 to 11 in which one of the high-refractive index moieties in the polymerizable monomer was replaced with a different structure, the ratio haze % / total Δn was reduced to 3.5 or less, and the haze of each holographic recording medium obtained relative to the total Δn was low.

In optical element applications such as light guide plates for AR glasses, the higher the total Δn of the holographic recording medium, the brighter the projected image, and the wider the viewing angle. In memory applications, increasing the total Δn can increase the recording capacity. In particular, in AR glass waveguide plate applications, when a holographic recording medium with a high haze is used, guided light is scattered, and this causes deterioration in the efficiency of light utilization and aesthetic quality. Therefore, the use of the compound of the invention that has both a high total Δn and a low haze allows an AR glass waveguide plate having high light utilization efficiency and high aesthetic quality to be produced.

As described above, the compounds of the invention used in the Examples are superior to the compounds in the Comparative Examples.

[Refractive index]

The refractive index of each of the polymerizable monomers produced in the Examples and Comparative Examples was measured using the following method. The results are shown in Table 2.

A test solution was prepared by dissolving a sample in a solution mixture of 3-phenoxybenzyl acrylate and trimethylolpropane trimethacrylate with a mass ratio of 4:1 such that a prescribed concentration was obtained. Specifically, two test solutions with sample concentrations of 10% by mass and 20% by mass were prepared.

The refractive index of each test solution was measured using a Kalnew precision refractometer (product name: KPR-2000 manufactured by Shimadzu Corporation). The temperature of the test solution was 23°C, and the measurement wavelength used was the d line (587.6 nm) of a helium lamp. A calibration curve representing the correlation between the sample concentration and the refractive index was produced based on the measurement results. Using the obtained calibration curve, the refractive index at a sample concentration of 100% by mass was determined and used as the refractive index of the sample.

[Table 2]

| | **Polymerizable Monomer** | **Refractive Index** |
|---|---|---|
| Example 1 | M-1 | 1.658 |
| Example 2 | M-2 | 1.666 |
| Example 3 | M-3 | 1.664 |
| Example 4 | M-4 | 1.696 |
| Example 5 | M-5 | 1.680 |
| Example 6 | M-6 | 1.654 |

(continued)

| | Polymerizable Monomer | Refractive Index |
|---|---|---|
| Example 7 | M-7 | 1.664 |
| Example 8 | M-8 | 1.664 |
| Example 9 | M-9 | 1.688 |
| Example 10 | M-10 | 1.672 |
| Example 11 | M-11 | 1.664 |
| Example 12 | M-12 | 1.667 |
| Example 13 | M-13 | 1.701 |
| Example 14 | M-14 | 1.670 |
| Example 15 | M-15 | 1.654 |
| Example 16 | M-16 | 1.686 |
| Example 17 | M-17 | 1.707 |
| Example 18 | M-18 | 1.683 |
| Example 19 | M-19 | 1.741 |
| Example 20 | M-20 | 1.670 |
| Example 21 | M-21 | 1.680 |
| Example 22 | M-22 | 1.713 |
| Example 23 | M-23 | 1.675 |
| Comparative Exaple 1 | M-24 | 1.679 |
| Comparative Exaple 2 | M-25 | 1.692 |
| Comparative Exaple 3 | M-26 | 1.672 |
| Comparative Exaple 4 | M-27 | 1.691 |

As can be seen from Table 2, the value of the refractive index of each of the compounds in the Examples was 1.65 or more, and each of these compounds has a refractive index high enough for a high-refractive index monomer.

As can be seen from Table 1, the total $\Delta n$ of each of the holographic recording mediums containing the compounds in the Examples is equivalent to or higher than that of each of the holographic recording mediums containing the compounds in the Comparative Examples. Therefore, the compounds in the Examples have improved compatibility with mediums while their holographic recording performance is maintained and are superior to the compounds in the Comparative Examples.

Although the present invention has been described in detail by way of the specific modes, it is apparent for those skilled in the art that various changes can be made without departing from the spirit and scope of the present invention.

The present application is based on Japanese Patent Application No. 2021-49181 filed on March 23, 2021, the entire contents of which are incorporated herein by reference.

Reference Signs List

S holographic recording medium
M1, M2, M3 mirror
L1 semiconductor laser light source for recording light
L2 laser light source for reproduction light
PD1, PD2, PD3 photodetector
PBS polarizing beam splitter

1 LED unit

## Claims

1. A compound represented by the following formula (1):

[Chem. 1]

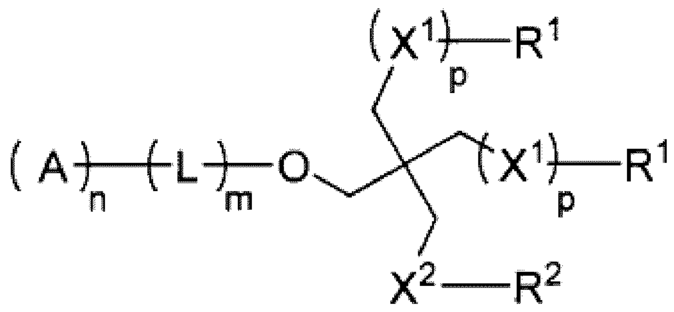

(1)

[wherein A represents a polymerizable group; L represents an optionally branched (n+1)-valent linking group; $R^1$ represents an aromatic ring group optionally having a substituent; $R^2$ represents a monovalent organic group optionally having a substituent; $X^1$ and $X^2$ each independently represent an oxygen atom, a sulfur atom, or a nitrogen atom optionally having a substituent; m represents an integer of 0 or 1; n represents an integer of 1 to 3; and p represents an integer of 0 or 1, and

wherein two $R^1$s may be bonded together at any positions to form a ring structure,
provided that $R^1 = R^2$, $X^1 = X^2$, and p = 1 do not hold simultaneously].

2. The compound according to claim 1, wherein the number of polymerizable groups in formula (1) is 1.

3. The compound according to claim 1 or 2, wherein A is an oxiranyl group, a vinyl group, an allyl group, or a (meth)acryloyl group.

4. The compound according to claim 3, wherein A is a (meth)acryloyl group.

5. The compound according to any one of claims 1 to 4, wherein $R^1$ is a fused aromatic ring group optionally having a substituent or a monocyclic aromatic group substituted with an aromatic ring group.

6. The compound according to any one of claims 1 to 5, wherein $R^2$ has a partial structure represented by the following formula (2):

[Chem. 2]

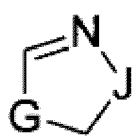

(2)

[wherein J represents a carbon atom optionally having a substituent or a nitrogen atom optionally having a substituent; and G represents a sulfur atom, an oxygen atom, or a nitrogen atom optionally having a substituent].

**7.** A compound represented by the following formula (3):

[Chem. 3]

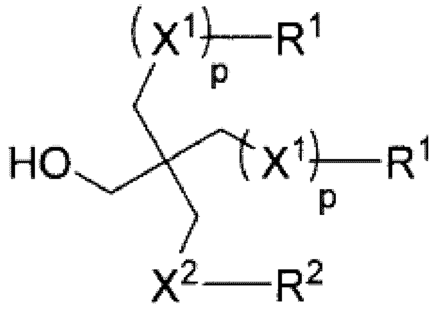

(3)

[wherein $R^1$ represents an aromatic ring group optionally having a substituent; $R^2$ represents a monovalent organic group optionally having a substituent; $X^1$ and $X^2$ each independently represent an oxygen atom, a sulfur atom, or a nitrogen atom optionally having a substituent; and p represents an integer of 0 or 1, and

wherein two $R^1$s may be bonded together at any positions to form a ring structure,
provided that $R^1 = R^2$, $X^1 = X^2$, and p = 1 do not hold simultaneously].

**8.** A method for producing the compound according to claim 7, the method comprising subjecting an aliphatic cyclic compound represented by the following formula (4) to a ring-opening reaction:

[Chem. 4]

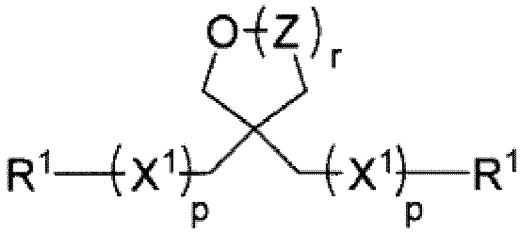

(4)

[wherein $R^1$ represents an aromatic ring group optionally having a substituent; $X^1$ represents an oxygen atom, a sulfur atom, or a nitrogen atom optionally having a substituent; p represents an integer of 0 or 1; Z represents an optionally branched aliphatic linking group optionally having a substituent; and r represents an integer of 0 or 1, and wherein two $R^1$s may be bonded together at any positions to form a ring structure].

**9.** The compound production method according to claim 8, wherein the aliphatic cyclic compound represented by the formula (4) is a compound represented by the following formula (5) or (6):

[Chem. 5]

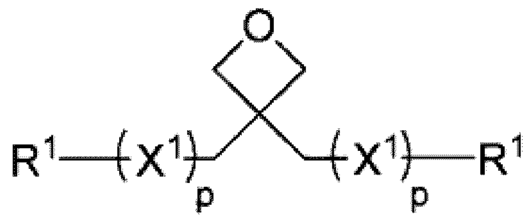

(5)

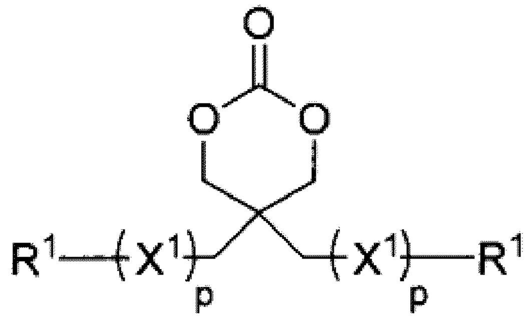

(6)

[wherein $R^1$ represents an aromatic ring group optionally having a substituent; $X^1$ represents an oxygen atom, a sulfur atom, or a nitrogen atom optionally having a substituent; and p represents an integer of 0 or 1, and wherein two $R^1$s may be bonded together at any positions to form a ring structure].

**10.** A polymerizable composition comprising: the compound according to any one of claims 1 to 6; and a polymerization initiator.

**11.** A holographic recording medium comprising the polymerizable composition according to claim 10.

**12.** A polymer obtained by polymerizing the polymerizable composition according to claim 10.

**13.** An optical material comprising the polymer according to claim 12.

**14.** An optical component comprising the polymer according to claim 12.

**15.** A large-capacity memory comprising the holographic recording medium according to claim 11.

**16.** An optical element obtained by recording a hologram in the holographic recording medium according to claim 12.

**17.** An AR glass comprising the optical element according to claim 16.

Fig.1

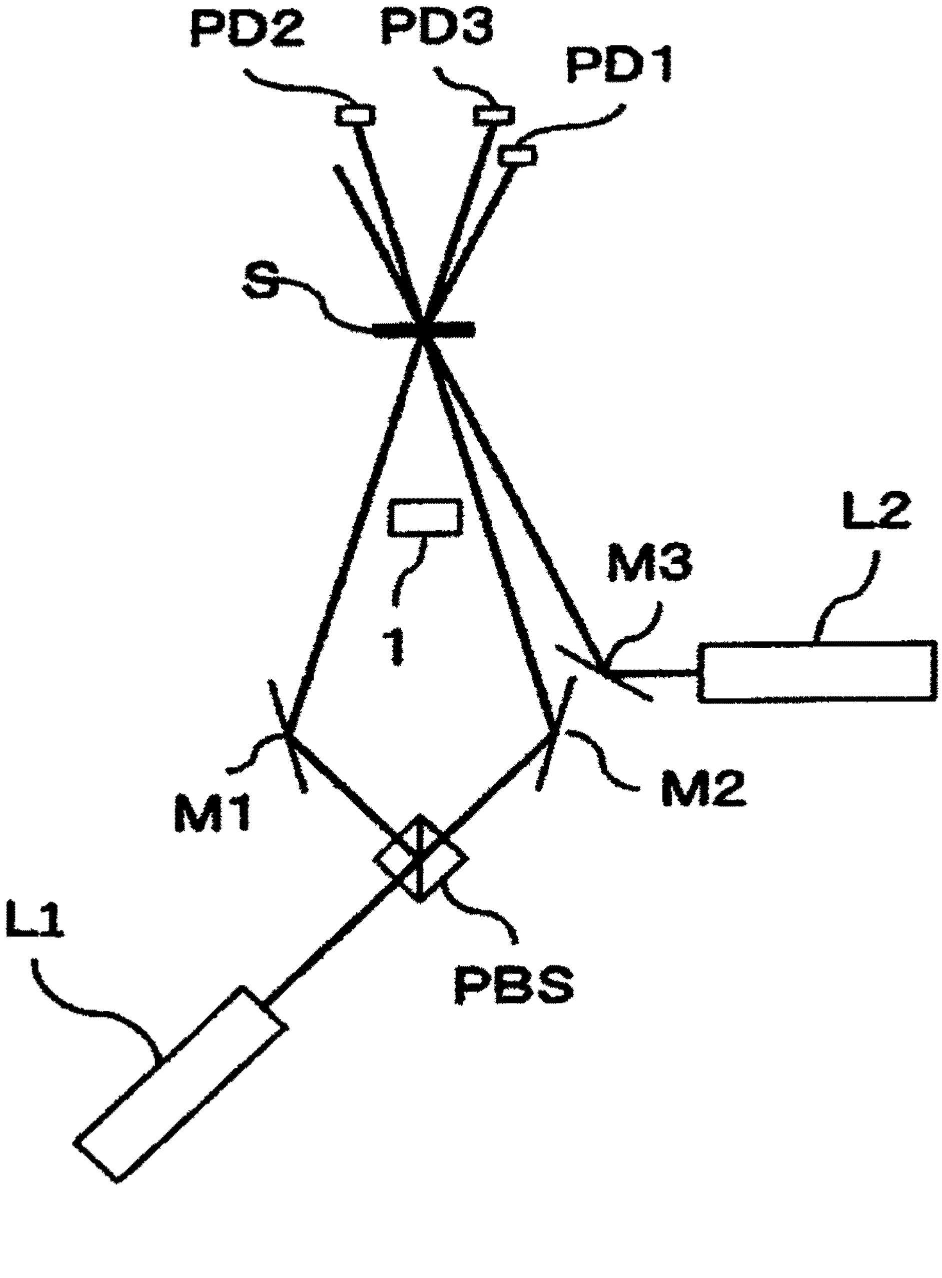

**INTERNATIONAL SEARCH REPORT**

International application No.
**PCT/JP2022/011919**

**A. CLASSIFICATION OF SUBJECT MATTER**

***C07D 417/12***(2006.01)i; ***C07D 417/14***(2006.01)i; ***C08F 20/38***(2006.01)i; ***G02B 1/04***(2006.01)i
FI: C08F20/38; C07D417/14; G02B1/04; C07D417/12

According to International Patent Classification (IPC) or to both national classification and IPC

**B. FIELDS SEARCHED**

Minimum documentation searched (classification system followed by classification symbols)

C07D417/12; C07D417/14; C08F20/38; G02B1/04

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X<br>A | KR 10-2018-0084460 A (TREEEL CO., LTD.) 25 July 2018 (2018-07-25)<br>claims | 1-3, 5, 7-10, 12-14<br>4, 6, 11, 15-17 |
| X<br>A | US 2006/0183873 A1 (XIE, Lunjia) 17 August 2006 (2006-08-17)<br>claims, paragraph [0157] | 7-9<br>1-6, 10-17 |
| A | JP 2016-222566 A (MITSUBISHI CHEMICALS CORP.) 28 December 2016 (2016-12-28)<br>claims, examples | 1-17 |
| A | JP 2017-14213 A (MITSUBISHI CHEMICALS CORP.) 19 January 2017 (2017-01-19)<br>claims, examples | 1-17 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **22 April 2022** | **10 May 2022** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**
**Information on patent family members**

International application No.
**PCT/JP2022/011919**

| Patent document cited in search report | Publication date (day/month/year) | Patent family member(s) | Publication date (day/month/year) |
|---|---|---|---|
| KR 10-2018-0084460 A | 25 July 2018 | (Family: none) | |
| US 2006/0183873 A1 | 17 August 2006 | (Family: none) | |
| JP 2016-222566 A | 28 December 2016 | (Family: none) | |
| JP 2017-14213 A | 19 January 2017 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H6220131 A
- JP 2008527413 A
- JP 2005133071 A
- WO 2021006011 A1
- WO 2021006012 A1
- JP 2017014213 A
- JP 5230189 A
- JP 2000204284 A
- JP 2000119306 A
- JP 2001220526 A
- JP 2001310937 A
- JP 3737306 B
- JP 59152396 A
- JP 61151197 A
- JP H5241338 A
- JP H269 A
- JP H255446 B
- JP 2000010277 A
- JP 2004198446 A
- JP 6069294 B
- JP 2021049181 A


### Non-patent literature cited in the description


- **H. KOGELNIK.** The Bell System Technical Journal. 1969, vol. 48, 2909-2947
- **H. KOGELNIK.** *The Bell System Technical Journal,* 1969, vol. 48, 2909-2947